(19) Europäisches Patentamt
European Patent Office
Office européen des brevets

(11) EP 1 294 765 B1

(12) # EUROPEAN PATENT SPECIFICATION

(45) Date of publication and mention of the grant of the patent:
**27.11.2013 Bulletin 2013/48**

(21) Application number: **01952183.0**

(22) Date of filing: **21.06.2001**

(51) Int Cl.:
***C07K 16/24*** (2006.01) ***C07K 14/54*** (2006.01)
***A61K 39/395*** (2006.01)

(86) International application number:
**PCT/US2001/019861**

(87) International publication number:
**WO 2002/008285 (31.01.2002 Gazette 2002/05)**

(54) **Use of an antibody specific for an IL-17 like polypeptide**

Verwendung eines Antikörpers mit Spezifizität für ein Interleukin-17-ähnliches Polypeptid.

Utilisation d'un anticorps spécifique pour polypeptides du type IL-17

(84) Designated Contracting States:
**AT BE CH CY DE DK ES FI FR GB GR IE IT LI LU MC NL PT SE TR**

(30) Priority: **22.06.2000 US 213125 P**
**02.02.2001 US 266159 P**
**16.03.2001 US 810384**

(43) Date of publication of application:
**26.03.2003 Bulletin 2003/13**

(73) Proprietor: **Amgen, Inc.**
**Thousand Oaks, CA 91320-1799 (US)**

(72) Inventors:
- **MEDLOCK, Eugene**
**Westlake Village, CA 91302 (US)**
- **YEH, Richard**
**Ithaca, NY 14850 (US)**
- **SILBIGER, Scott, M.**
**Woodland Hills, CA 91367 (US)**
- **ELLIOT, Gary, S.**
**Thousand Oaks, CA 91362 (US)**
- **NGUYEN, Hung, Q.**
**Thousand Oaks, CA 91360 (US)**
- **JING, Shuqian**
**Thousand Oaks, CA 91362 (US)**

(74) Representative: **Grünecker, Kinkeldey, Stockmair & Schwanhäusser**
**Leopoldstrasse 4**
**80802 München (DE)**

(56) References cited:
**WO-A-00/37640 WO-A-00/42187**
**WO-A-00/42188 WO-A-00/55204**
**WO-A-00/60080 WO-A-01/57202**
**WO-A-01/59120 WO-A-01/79288**
**WO-A-99/14240 WO-A-99/35263**

- **TIAN ERMING ET AL: "Evi27 encodes a novel membrane protein with homology to the IL17 receptor." ONCOGENE, vol. 19, no. 17, 20 April 2000 (2000-04-20), pages 2098-2109, XP008000240 ISSN: 0950-9232**
- **YAO Z ET AL: "MOLECULAR CHARACTERIZATION OF THE HUMAN INTERLEUKIN (IL)-17 RECEPTOR" CYTOKINE, ACADEMIC PRESS LTD, PHILADELPHIA, PA, US, vol. 9, no. 11, November 1997 (1997-11), pages 794-800, XP000867704 ISSN: 1043-4666**
- **LEE JAMES ET AL: "IL-17E, a novel proinflammatory ligand for the IL-17 receptor homolog IL-17Rh1." JOURNAL OF BIOLOGICAL CHEMISTRY, vol. 276, no. 2, 12 January 2001 (2001-01-12), pages 1660-1664, XP002196319 ISSN: 0021-9258**
- **BALLANTYNE ET AL: "Blocking IL-25 prevents airway hyperresponsiveness in allergic asthma", JOURNAL OF ALLERGY AND CLINICAL IMMUNOLOGY, MOSBY, INC, US, vol. 120, no. 6, 1 December 2007 (2007-12-01), pages 1324-1331, XP022383425, ISSN: 0091-6749, DOI: 10.1016/J.JACI.2007.07.051**
- **J. K. KOLLS: 'Interleukin-17: An Emerging Role in Lung Inflammation' AMERICAN JOURNAL OF RESPIRATORY CELL AND MOLECULAR BIOLOGY vol. 28, no. 1, 01 January 2003, pages 9 - 11, XP055018895 DOI: 10.1165/rcmb. 2002-0255PS ISSN: 1044-1549**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- KOLLER D Y ET AL: "Relationship between disease severity and inflammatory markers in cystic fibrosis.", ARCHIVES OF DISEASE IN CHILDHOOD DEC 1996 LNKD- PUBMED: 9014602, vol. 75, no. 6, December 1996 (1996-12), pages 498-501, ISSN: 1468-2044
- HÄLLGREN R ET AL: "Eosinophil activation in the lung is related to lung damage in adult respiratory distress syndrome.", THE AMERICAN REVIEW OF RESPIRATORY DISEASE MAR 1987 LNKD- PUBMED:3030169, vol. 135, no. 3, March 1987 (1987-03), pages 639-642, ISSN: 0003-0805
- FUJIMOTO K ET AL: "Eosinophilic inflammation in the airway is related to glucocorticoid reversibility in patients with pulmonary emphysema.", CHEST MAR 1999 LNKD- PUBMED:10084478, vol. 115, no. 3, March 1999 (1999-03), pages 697-702, ISSN: 0012-3692

## Description

### Field of the Invention

The present invention relates to uses of antibodies specific for IL-17 like polypeptides.

### Background of the Invention

Technical advances in identification, cloning, expression and manipulation of nucleic acid molecules and deciphering of human genome have greatly accelerated discovery of novel therapeutics. Rapid nucleic acid sequencing techniques can now generate sequence information at unprecedented rates and, coupled with computational analyses, allow the assembly of overlapping sequences into partial and entire genomes as well as identification of polypeptide-encoding regions. A comparison of a predicted amino acid sequence against a database compilation of known amino acid sequences allows one to determine the extent of homology to previously identified sequences and/or structural landmarks. The cloning and expression of a polypeptide-encoding region of a nucleic acid molecule provides a polypeptide product for structural and functional analyses. The manipulation of nucleic acid molecules and encoded polypeptides may confer advantageous properties on a product for use as a therapeutic.

In spite of significant technical advances in genome research over the past decade, the potential for development of novel therapeutics based on the human genome is still largely unrealized. Many genes encoding potentially beneficial polypeptide therapeutics or those encoding polypeptides, which may act as "targets" for therapeutic molecules, have still not been identified.

Accordingly, it is an object of the invention to identify novel polypeptides, and nucleic acid molecules encoding the same, which have diagnostic or therapeutic benefit.

### Summary of the Invention

The present invention relates to uses for antibodies against novel IL-17 like polypeptides.

The invention provides a use of an antagonistic antibody specific for a polypeptide consisting of the amino acid sequence of SEQ ID NO: 2, or a fragment thereof comprising 25, 50, 75, 100 or 150 contiguous amino acids, for preparation of a medicament for treating cystic fibrosis, acute respiratory disease syndrome or emphysema.

The invention further provides an antagonistic antibody specific for a polypeptide consisting of the amino acid sequence of SEQ ID NO: 2, or a fragment thereof comprising 25, 50, 75, 100 or 150 contiguous amino acids, for use in the treatment of cystic fibrosis, acute respiratory disease syndrome or emphysema.

The invention further provides a use of a pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for preparation of a medicament for treating cystic fibrosis, acute respiratory disease syndrome or emphysema.

The invention further provides a pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for use in the treatment of cystic fibrosis, acute respiratory disease syndrome or emphysema.

The present application discloses an isolated nucleic acid molecule comprising a nucleotide sequence selected from the group consisting of:

(a) the nucleotide sequence as set forth in SEQ ID NO:1, SEQ ID NO:3, or SEQ ID NO:9;

(b) a nucleotide sequence encoding the polypeptide as set forth in SEQ ID NO:2, SEQ ID NO:4, or SEQ ID NO:10;

(c) a nucleotide sequence which hybridizes under moderately or highly stringent conditions to the complement of (a) or (b), wherein the encoded polypeptide has an activity of the polypeptide as set forth in SEQ ID NO:2, SEQ ID NO:4, or SEQ ID NO:10; and

(d) a nucleotide sequence complementary to any of (a) through (c).

The present application also discloses an isolated nucleic acid molecule comprising a nucleotide sequence selected from the group consisting of:

(a) a nucleotide sequence encoding a polypeptide that is at least about 70, 75, 80, 85, 90, 95, 96, 97, 98 or 99 percent identical to the polypeptide as set forth in SEQ ID NO:2, SEQ ID NO:4, or SEQ ID NO:10, wherein the encoded polypeptide has an activity of the polypeptide as set forth in SEQ ID NO:2, SEQ ID NO:4, or SEQ ID NO:10;

(b) a nucleotide sequence encoding an allelic variant or splice variant of the nucleotide sequence as set forth in SEQ ID NO:1, SEQ ID NO:3, or SEQ ID NO:9, wherein the encoded polypeptide has an activity of the polypeptide as set forth in SEQ ID NO:2, SEQ ID NO:4, or SEQ ID NO:10;

(c) a nucleotide sequence of SEQ ID NO:1, SEQ ID NO:3, or SEQ ID NO:9, (a), or (b) encoding a polypeptide fragment of at least about 25 amino acid residues, wherein the polypeptide has an activity of the polypeptide as set forth in SEQ ID NO:2, SEQ ID NO:4, or SEQ ID NO:10;

(d) a nucleotide sequence of SEQ ID NO:1, SEQ ID NO:3, or SEQ ID NO:9, or (a)-(c) comprising a fragment of at least about 16 nucleotides;

(e) a nucleotide sequence which hybridizes under moderately or highly stringent conditions to the complement of any of (a)-(d), wherein the encoded polypeptide has an activity of the polypeptide as set forth in SEQ ID NO:2, SEQ ID NO:4, or SEQ ID NO:10; and

(f) a nucleotide sequence complementary to any of (a)-(d).

The present application further discloses an isolated nucleic acid molecule comprising a nucleotide sequence selected from the group consisting of:

(a) a nucleotide sequence encoding a polypeptide as set forth in SEQ ID NO:2, SEQ ID NO:4, or SEQ ID NO:10 with at least one conservative amino acid substitution, wherein the encoded polypeptide has an activity of the polypeptide as set forth in SEQ ID NO:2, SEQ ID NO:4, or SEQ ID NO:10;

(b) a nucleotide sequence encoding a polypeptide as set forth in SEQ ID NO:2, SEQ ID NO:4, or SEQ ID NO:10 with at least one amino acid insertion, wherein the encoded polypeptide has an activity of the polypeptide as set forth in SEQ ID NO:2, SEQ ID NO:4, or SEQ ID NO:10;

(c) a nucleotide sequence encoding a polypeptide as set forth in SEQ ID NO:2, SEQ ID NO:4, or SEQ ID NO:10 with at least one amino acid deletion, wherein the encoded polypeptide has an activity of the polypeptide as set forth in SEQ ID NO:2, SEQ ID NO:4, or SEQ ID NO:10;

(d) a nucleotide sequence encoding a polypeptide as set forth in SEQ ID NO:2, SEQ ID NO:4, or SEQ ID NO:10 which has a C- and/or N- terminal truncation, wherein the encoded polypeptide has an activity of the polypeptide as set forth in SEQ ID NO:2, SEQ ID NO:4, or SEQ ID NO:10;

(e) a nucleotide sequence encoding a polypeptide as set forth in SEQ ID NO:2, SEQ ID NO:4, or SEQ ID NO:10 with at least one modification selected from the group consisting of amino acid substitutions, amino acid insertions, amino acid deletions, C-terminal truncation, and N-terminal truncation, wherein the encoded polypeptide has an activity of the polypeptide as set forth in SEQ ID NO:2, SEQ ID NO:4, or SEQ ID NO:10;

(f) a nucleotide sequence of (a)-(e) comprising a fragment of at least about 16 nucleotides;

(g) a nucleotide sequence which hybridizes under moderately or highly stringent conditions to the complement of any of (a)-(f), wherein the encoded polypeptide has an activity of the polypeptide as set forth in SEQ ID NO:2, SEQ ID NO:4, or SEQ ID NO:10; and

(h) a nucleotide sequence complementary to any of (a)-(e).

The present application also discloses an isolated polypeptide comprising the amino acid sequence selected from the group consisting of:

(a) an amino acid sequence comprising the mature human IL-17 like polypeptide contained in SEQ ID NO:2, and optionally further comprising an amino terminal methionine; or an amino acid sequence comprising the mature murine IL-17 like polypeptide contained in SEQ ID NO:4 or SEQ ID NO:10;

(b) an amino acid sequence for an ortholog of SEQ ID NO:2, SEQ ID NO:4, or SEQ ID NO:10, wherein the polypeptide has an activity of the polypeptide as set forth in SEQ ID NO:2, SEQ ID NO:4, or SEQ ID NO:10;

(c) an amino acid sequence that is at least about 70, 75, 80, 85, 90, 95, 96, 97, 98, or 99 percent identical to the amino acid sequence of SEQ ID NO:2, SEQ ID NO:4, or SEQ ID NO:10, wherein the polypeptide has an activity of the polypeptide as set forth in SEQ ID NO:2, SEQ ID NO:4, or SEQ ID NO:10;

(d) a fragment of the amino acid sequence set forth in SEQ ID NO:2, SEQ ID NO:4, or SEQ ID NO:10 comprising at least about 25 amino acid residues, wherein the polypeptide has an activity of the polypeptide as set forth in SEQ ID NO:2, SEQ ID NO:4, or SEQ ID NO:10;

(e) an amino acid sequence for an allelic variant or splice variant of either the amino acid sequence as set forth in SEQ ID NO:2, SEQ ID NO:4, or SEQ ID NO:10, or at least one of (a)-(c) wherein the polypeptide has an activity of the polypeptide as set forth in SEQ ID NO:2, SEQ ID NO:4, or SEQ ID NO:10.

The present application further discloses an isolated polypeptide comprising the amino acid sequence selected from the group consisting of:

(a) the amino acid sequence as set forth in SEQ ID NO:2, SEQ ID NO:4, or SEQ ID NO:10 with at least one conservative amino acid substitution, wherein the polypeptide has an activity of the polypeptide as set forth in SEQ ID NO:2, SEQ ID NO:4, or SEQ ID NO:10;

(b) the amino acid sequence as set forth in SEQ ID NO:2, SEQ ID NO:4, or SEQ ID NO:10 with at least one amino acid insertion, wherein the polypeptide has an activity of the polypeptide as set forth in SEQ ID NO:2, SEQ ID NO: 4, or SEQ ID NO:10;

(c) the amino acid sequence as set forth in SEQ ID NO:2, SEQ ID NO:4, or SEQ ID NO:10 with at least one amino acid deletion, wherein the polypeptide has an activity of the polypeptide as set forth in SEQ ID NO:2, SEQ ID NO: 4, or SEQ ID NO:10;

(d) the amino acid sequence as set forth in SEQ ID NO:2, SEQ ID NO:4, or SEQ ID NO:10 which has a C- and/or N-terminal truncation, wherein the polypeptide has an activity of the polypeptide as set forth in SEQ ID NO:2, SEQ ID NO:4, or SEQ ID NO:10; and

(e) the amino acid sequence as set forth in SEQ ID NO:2, SEQ ID NO:4, or SEQ ID NO:10, with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2, SEQ ID NO:4, or SEQ ID NO:10.

Also disclosed are fusion polypeptides comprising the amino acid sequences of (a)-(e) above.

The present application also discloses an expression vector comprising the isolated nucleic acid molecules as set forth herein, recombinant host cells comprising recombinant nucleic acid molecules as set forth herein, and a method of producing an IL-17 like polypeptide comprising culturing the host cells and optionally isolating the polypeptide so produced.

A transgenic non-human animal comprising a nucleic acid molecule encoding an IL-17 like polypeptide is also disclosed. The IL-17 like nucleic acid molecules are introduced into the animal in a manner that allows expression and increased levels of the IL-17 like polypeptide, which may include increased circulating levels. The transgenic non-human animal is preferably a mammal.

Also disclosed are derivatives of the IL-17 like polypeptides of the present invention.

Analogs of the IL-17 like polypeptides are disclosed in the present application which result from conservative and/or non-conservative amino acids substitutions of the IL-17 like polypeptide of SEQ ID NO:2. Such analogs include an IL-17 like polypeptide wherein, for example the amino acid at position 67 of SEQ ID NO: 2 is asparagine or glutamine, the amino acid at position 69 of SEQ ID NO: 2 is lysine, gluatmine, asparagine or arginine, the amino acid at position 94 of SEQ ID NO: 2 is cysteine, serine or alanine, the amino acid at position 96 of SEQ ID NO: 2 is cysteine, serine or alanine, the amino acid at position 101 of SEQ ID NO: 2 is isoleucine, methionine, lelucine, phenylalanine, alanine, norleucine or valine, the amino acid at position 104 of SEQ ID NO: 2 is threonine or serine, the amino acid at position 129 of SEQ ID NO: 2 is cysteine, alanine or serine, the amino acid at position 140 of SEQ ID NO: 2 is cysteine, alanine or serine, the amino acid at position 152 of SEQ ID NO: 2 is cysteine, alanine or serine.

Analogs, fragments or variants of IL-17 like polypeptide that retain receptor-binding activity or cytokine biological activity are specifically contemplated. Analogs, fragments or variants of IL-17 like polypeptide that bind to receptor but fail to transduce a signal are also contemplated.

Pharmaceutical compositions comprising the disclosed nucleotides, polypeptides or selective binding agents and one or more pharmaceutically acceptable formulation agents are also disclosed. The pharmaceutical compositions are used to provide therapeutically effective amounts of the disclosed nucleotides or polypeptides. Disclosed are also methods of using the polypeptides, and nucleic acid molecules. The invention relates to selective binding agents for use in treating certain diseases, wherein the selective binding agents are antibodies.

The disclosed IL-17 like polypeptides, antibodies and derivatives thereof, other selective binding agents, small molecules and nucleic acid molecules (including antisense nucleic acids) may be used to treat, prevent, ameliorate and/or detect diseases and disorders, including those recited herein. For example, the IL-17 like polypeptides and polynucleotides may have proinflammatory activity and therefore may play a role in pathological conditions related to inflammation. IL-17 like polypeptide or polynucleotide expression may also play a role in the progression of cancer. For example, IL-17 like polypeptide and polynucleotide may play a role in lymphoma conditions and increased expression of IL-17 like polypeptide or polynucleotide may be indicative of a prelymphoma state. Decreasing IL-17 like polypeptide levels or activity may be desirable in acute or chronic inflammatory disease states, including autoimmune diseases, and in cancer disease states, including lymphoma or prelymphoma conditions. Conversely, increasing IL-17 like polypeptide activity may be desirable in other disease states, such as infection.

The present application also discloses a method of assaying test molecules to identify a test molecule which binds to an IL-17 like polypeptide. The method comprises contacting an IL-17 like polypeptide with a test molecule to determine the extent of binding of the test molecule to the polypeptide. The method further comprises determining whether such test molecules are agonists or antagonists of an IL-17 like polypeptide. The present application further discloses a method of testing the impact of molecules on the expression of an IL-17 like polypeptide or on the activity of an IL-17 like polypeptide.

One embodiment discloses methods of identifying inhibitors of an interaction of IL-17 like polypeptide with an IL-17 receptor RB-2 or RB-3 polypeptide. These methods comprise the steps of detecting binding of an IL-17 like polypeptide (such as a polypeptide comprising the mature protein sequence set out in SEQ ID NO: 2 or fragments, analogs or variants thereof that retain receptor-binding activity) to an IL-17 receptor RB-2 or RB-3 polypeptide (such as a polypeptide comprising the extracellular region of SEQ ID NO: 18 or 20, or fragments, analogs or variants thereof that retain ligand-binding activity), in the presence and absence of a test compound, and identifying the test compound as a candidate inhibitor when the binding is decreased in the presence of the compound. Suitable test compounds include nucleic acid molecules, proteins, peptides, carbohydrates, lipids, organic and inorganic compounds, libraries of which can be screened using known high throughput screening procedures. The present invention provides for antagonistic antibodies for use in treating a pathological condition mediated by IL-17 like polypeptide said condition being cystic fibrosis, acute respiratory disease syndrome or emphysema. The antagonistic antibody can be administered to specifically bind to IL-17 like polypeptide. The application also discloses a method of inhibiting undesirable interaction of IL-17 like polypeptide with IL-17 receptor RB-2 or RB-3 comprising administering a therapeutically effective amount of a molecule capable of binding the IL-17 like polypeptide or IL-17 receptor RB-2 or RB-3.

These identified candidate inhibitors include selective binding agents, fragments, analogs or variants of IL-17 like polypeptides of the present invention and fusion proteins thereof. Exemplary IL-17 like polypeptide mediated pathological conditions are described in further detail herein.

The application also discloses a method of inhibiting undesirable interaction of IL-17 like polypeptide with IL-17 receptor RB-2 or RB-3 comprising administering a therapeutically effective amount of a molecule capable of binding the IL-17 like polypeptide or IL-17 receptor RB-2 or RB-3.

Methods of regulating expression and modulating (i.e., increasing or decreasing) levels of an IL-17 like polypeptide are also disclosed. One method comprises administering to an animal a nucleic acid molecule encoding an IL-17 like polypeptide or antisense nucleic acid molecules (e.g., that specifically bind to IL-17 like polypeptide encoding DNA or RNA or regulatory sequences and inhibit expression of IL-17 like polypeptide). In another method, a nucleic acid molecule comprising elements that regulate or modulate the expression of an IL-17 like polypeptide may be administered. Examples of these methods include gene therapy, cell therapy and anti-sense therapy as further described herein. Yet other methods to decrease levels or activity of IL-17 like polypeptide involve administration of a selective binding agent (such as antibodies and derivatives thereof including chimeric, humanized or human antibodies or fragments thereof that specifically bind to the IL-17 like polypeptide or its receptor-binding sites) to antagonize the activity of IL-17 like polypeptide. Administration of an analog, fragment or variant of IL-17, including a fusion protein thereof, that antagonizes the activity of native IL-17 like polypeptide is also contemplated.

In another disclosure, the IL-17 like polypeptides may be used for identifying receptors thereof ("IL-17 like receptors"). Various forms of "expression cloning" have been extensively used to clone receptors for protein ligands. See for example, H. Simonsen and H.F. Lodish, Trends in Pharmacological Sciences, 15:437-441 (1994), and Tartaglia et al., Cell, 83:1263-1271 (1995). The isolation of the IL-17 like receptor(s) is useful for identifying or developing novel agonists and antagonists of the IL-17 like polypeptide-signaling pathway. Such agonists and antagonists include soluble IL-17 like receptor(s) (e.g. fragments lacking all or part of the transmembrane and/or cytoplasmic region(s) or fragments of the extracellular region(s) that retain ligand binding activity, analogs or variants thereof, and fusions thereof to heterologous polypeptides such as constant domains of an immunoglobulin or fragments or variants thereof that retain the ability to prolong half-life in circulation), anti-IL-17 like receptor-selective binding agents (such as antibodies and derivatives thereof including chimeric, humanized or human antibodies or fragments thereof that specifically bind to the IL-17 receptor like polypeptide or its ligand-binding sites), small molecules, and antisense oligonucleotides (e.g., that specifically bind to IL-17 like polypeptide encoding DNA or RNA or regulatory sequences and inhibit expression of IL-17 like polypeptide), any of which can be used for treating one or more of the diseases or disorders, including those recited herein. For example, IL-17 like polypeptide antagonists may be administered as an anti-inflammatory therapeutic or used to treat cancerous or lymphoma conditions.

Two receptors that bind to the disclosed IL-17 like polypeptide have been identified in Example 8 and are denoted as IL-17RB-2 and IL-17RB-3. Their nucleotide and amino acid sequences are set forth in SEQ ID NOS: 17-18 (IL-17RB-2) and SEQ ID NOs: 19-20 (IL-17RB-3), respectively. The predicted transmembrane domain spans residues 293 to 313 of SEQ ID NO: 18 and residues 351 to 371 of SEQ ID NO: 20. The predicted signal peptide spans 14 residues of SEQ ID NOS: 18 and 20. Therefore the predicted extracellular sequence spans amino acids 14 to 292 of SEQ ID NO: 18 and amino acids 14 to 350 of SEQ ID NO: 20.

In certain embodiments, an IL-17 like polypeptide agonist or antagonist may be a protein, peptide, carbohydrate, lipid, or small molecular weight molecule which interacts with IL-17 like polypeptide to regulate its activity.

## Brief Description of the Figures

Figure 1 depicts a nucleic acid sequence (SEQ ID NO:1) encoding the human IL-17 like polypeptide. Also depicted is the amino acid sequence (SEQ ID NO:2) of the human IL-17 like polypeptide. In this figure, the predicted signal peptide is underlined; it is believed that amino acids 1 through 16 comprise the leader sequence.

Figure 2A-2C depicts a nucleic acid sequence (SEQ ID NO:3) encoding the mouse IL-17 like polypeptide. Also depicted is the amino acid sequence (SEQ ID NO:4) of the mouse IL-17 like polypeptide. In this figure, the predicted signal peptide is underlined; it is believed that amino acids 1 through 18 comprise the leader sequence. Figure 2B-2C also depicts the nucleic acid sequence (SEQ ID NO:9) of a non-secreted form of mouse IL-17 like cDNA, and the corresponding amino acid sequence thereof (SEQ ID NO:10)

Figure 3A-3B depicts a pile-up of IL-17 like amino acid sequence, hIL-17L, (SEQ ID NO:2), with the amino acid sequence of a known human IL-17 family member, hIL-17, (SEQ ID NO:5).

Figure 4 depicts a pile-up of IL-17 like amino acid sequence, hIL-17L, (SEQ ID NO:2) with the amino acid sequence of a known human IL-20 family member, hIL-20, (SEQ ID NO:6) .

Figure 5 depicts a pile-up of IL-17 like amino acid sequence, hIL-17L, (SEQ ID NO:2) with the amino acid sequence of a known human IL-17 family member, hIL-17b (SEQ ID NO:7).

Figure 6A-6B depicts a pile-up of IL-17 like amino acid sequence, hIL-17L, (SEQ ID NO:2) with the amino acid sequence of a known human IL-17 Family Member, hIL-17c, (SEQ ID NO:8).

Figure 7 depicts a Northern blot detecting expression of the IL-17 like overexpressing transgene in necropsied transgenic founder mice (nos. 1, 16, 27, 29, 55, 61, 20, 52, and 66). The control mice (nos. 2, 17, 53 and 65) are non-transgenic littermates. The lane marked "bl" is a blank lane and the positive control (+) was the IL-17 like cDNA. The presence of a 0.54 kb band is indicative of transgene expression.

Figure 8 depicts a Northern blot detecting expression of the IL-17 like overexpressing transgene in hepatectomized transgenic founder mice (nos. 10,11, 30, 31, 33, 37, 46, 67, and 68). The control mice (nos. 32, 35, 36 and 45) are non-transgenic littermates. The lane marked "MI" represents the microinjection fragment which was loaded as a positive control. The presence of a 0.54 kb band is indicative of transgene expression.

Figure 9 depicts hematoxylin and eosin (A,B, G-J), B220 (C,D) and F4/80 (E,F) stained sections of lymph node (A-H) or bone marrow (I,J) from IL-17 like transgenic mice (B,D,F,H) or non-transgenic control mice (A,C,E,G). Panels A-F illustrate that the IL-17 like transgenic lymph node was markedly enlarged with its normal architecture disrupted due to a marked cellular infiltrate (asterisk in panel B) that contained large numbers of B220 positive B lymphocytes cells (panel D) and some F4/80 staining macrophages. Panel H illustrates that this cellular infiltrate also contained numerous eosinophils (arrowheads) as well as multinucleated inflammatory giant cells (arrows).

Figure 10 depicts hematoxylin and eosin (A,B; E-I) and B220 (C,D) stained sections of lymph bone marrow (A,B), spleen (C-F) and kidney (G-J) from IL-17 like transgenic mice (B,D,F,H,J) or non-transgenic control mice (A,C,E, G,I). Panel A illustrates marked eosinophilic myeloid hyperplasia. Panel D illustrates lymphoid hyperplasia with a predominance of B220 positive B cells (arrows) in the IL-17 like transgenic mouse spleen, while panel F illustrates eosinophilic myeloid hyperplasia in the IL-17 like transgenic splenic red pulp compared to the non-transgenic splenic red pulp (E). Panels H and J illustrate renal pelvic dilation (arrow in H) with a marked eosinophilic inflammatory infiltration in the renal pelvis (pyelonephritis, panel J).

Figure 11 depicts a bar chart histogram showing a significant increase in absolute numbers of CD19+ B lymphocytes in the peripheral blood of 4 out of 9 IL-17 like transgenic mice as compared to the non-trangenic littermate controls.

Figure 12 depicts a bar chart histogram showing an increase in absolute numbers of CD19+ B lymphocytes in the spleens of 5 out of 10 IL-17 like transgenic mice as compared to the non-transgenic littermate controls.

Figure 13 depicts a bar chart histogram showing a slight decrease in absolute numbers of CD19+ B lymphocytes in the bone marrow of IL-17 like transgenic mice as compared to the non-transgenic littermate controls.

Figure 14 depicts a bar chart histogram showing an increase in absolute numbers of CD4+ T lymphocytes in the peripheral blood of 4 out 9 IL-17 like transgenic mice as compared to the non-transgenic littermate controls.

Figure 15 depicts a bar chart histogram showing an increase in absolute numbers of CD4+ T lymphocytes in the spleens of IL-17 like transgenic mice as compared to the non-transgenic littermate controls.

Figure 16 depicts scatter plots representative of the changes occurring in the IL-17 like transgenic mice vs. their non-transgenic littermate controls. The two top plots labeled "A" are 2-color flow cytometric dot plots where CD45R+ and IL-17 like-Fc labeling are being depicted on their respective axes. Control plot "A" shows an absence of CD45R+/IL-17 like-Fc+ cells in the region R1 whereas in the transgenic plot " A", this population was present in region R1 and represented 8% of the total granulocyte population. In the corresponding Forward vs. Side scatter plot ("B" and "C") these cells are depicted as pink colored dots. This population was absent in the control plot "B".

Figure 17 depicts scatter plots representative of the changes occurring in the IL-17 like transgenic mice vs. their non-transgenic littermate controls. The two top plots labeled "A" are 2-color flow cytometric dot plots where CD4 and IL-17 like-Fc labeling are being depicted on their respective axis. Control plot "A" shows an absence of CD4+/IL-17 like-Fc+ cells in the region R1, whereas in the transgenic plot " A", this population was present in region R1 and represented 14% of the total granulocyte population. In the corresponding Forward vs. Side scatter plots (size vs. granularity), the IL-17 like transgenic mice (B) these cells are located just above the region where granulocytes are typically found (red colored dots). These cells are absent in the control plot "B". Furthermore, for the transgenic mice (A), there is an emergence of a population of cells that was neither CD4+ nor IL-17 like-Fc+ (region R2) but that has the scatter properties of eosinophils, localizing to the left of the granulocytes in the Forward vs. Side scatter plot "B"(green colored dots). This population was absent in the control plot "B".

Figure 18 depicts a bar chart histogram showing an increase in absolute numbers of rhIL-17 like-Fc+/CD45R+ granulocyte-like cells in the bone marrow of 5 out of 10 IL-17 like transgenic mice as compared to the non-transgenic littermate controls.

Figure 19 depicts a bar chart histogram showing an increase in absolute numbers of rhIL-17 like-Fc+/CD4+ granulocyte-like cells in the bone marrow of IL-17 like transgenic mice as compared to the non-transgenic littermate controls.

Figure 20 depicts an example of a typical Forward vs. Side scatter plot (size vs. granularity). Cells in the gate can be sorted to give a purified population.

Figure 21A-21B depicts FACS profiles of IL-17 like polypeptide overexpressing transgenic mice and non-transgenic controls of CD5, CD34 and CD4 expression on cells from specified lymphoid tissues. Percentages included refer to double positive poluations. Absolute numbers of cells for CD5+CD19+, CD34+CD19+, and CD4+Eosinophil populations are represented as percent populations (for lymphocytes) and absolute number of cells (eosinophils).

## Detailed Description of the Invention

The section headings used herein are for organizational purposes only.

## Definitions

The terms "IL-17 like gene" or "IL-17 like nucleic acid molecule" or "polynucleotide" refers to a nucleic acid molecule comprising or consisting of a nucleotide sequence as set forth in SEQ ID NO:1, SEQ ID NO:3, or SEQ ID NO: 9, a nucleotide sequence encoding the polypeptide as set forth in SEQ ID NO:2, SEQ ID NO:4, or SEQ ID NO:10, and nucleic acid molecules as defined herein.

The term "IL-17 like polypeptide" refers to a polypeptide comprising the amino acid sequence of SEQ ID NO: 2, SEQ ID NO:4, or SEQ ID NO:10, and related polypeptides. Related polypeptides include: IL-17 like polypeptide allelic variants, IL-17 like polypeptide orthologs, IL-17 like polypeptide splice variants, IL-17 like polypeptide variants and IL-17 like polypeptide derivatives. IL-17 like polypeptides may be mature polypeptides, as defined herein, and may or may not have an amino terminal methionine residue, depending on the method by which they are prepared.

The term "IL-17 like polypeptide allelic variant" refers to one of several possible naturally occurring alternate forms of a gene occupying a given locus on a chromosome of an organism or a population of organisms.

The term "IL-17 like polypeptide derivatives" refers to the polypeptide as set forth in SEQ ID NO:2, SEQ ID NO: 4, or SEQ ID NO:10, IL-17 like polypeptide allelic variants, IL-17 like polypeptide orthologs, IL-17 like polypeptide splice variants, or IL-17 like polypeptide variants, as defined herein, that have been chemically modified.

The term "IL-17 like polypeptide fragment" refers to a polypeptide that comprises a truncation at the amino terminus (with or without a leader sequence) and/or a truncation at the carboxy terminus of the polypeptide as set forth in SEQ ID NO:2, SEQ ID NO:4, or SEQ ID NO:10, IL-17 like polypeptide allelic variants, IL-17 like polypeptide orthologs, IL-17 like polypeptide splice variants and/or an IL-17 like polypeptide variant having one or more amino acid additions or substitutions or internal deletions (wherein the resulting polypeptide is at least six (6) amino acids or more in length) as compared to the IL-17 like polypeptide amino acid sequence set forth in SEQ ID NO:2, SEQ ID NO:4, or SEQ ID NO: 10. IL-17 like polypeptide fragments may result from alternative RNA splicing or from in *vivo* protease activity. In preferred embodiments, truncations comprise about 10 amino acids, or about 20 amino acids, or about 50 amino acids, or about 75 amino acids, or about 100 amino acids, or more than about 100 amino acids. The polypeptide fragments so produced will comprise about 25 contiguous amino acids, or about 50 amino acids, or about 75 amino acids, or about 100 amino acids, or about 150 amino acids, or about 200 amino acids. Such IL-17 like polypeptide fragments may optionally comprise an amino terminal methionine residue. It will be appreciated that such fragments can be used, for example, to generate antibodies to IL-17 like polypeptides.

The term "IL-17 like fusion polypeptide" refers to a fusion of one or more amino acids (such as a heterologous peptide or polypeptide) at the amino or carboxy terminus of the polypeptide as set forth in SEQ ID NO:2, SEQ ID NO: 4, or SEQ ID NO:10, IL-17 like polypeptide allelic variants, IL-17 like polypeptide orthologs, IL-17 like polypeptide splice variants, or IL-17 like polypeptide variants having one or more amino acid deletions, substitutions or internal additions as compared to the IL-17 like polypeptide amino acid sequence set forth in SEQ ID NO:2, SEQ ID NO:4, or SEQ ID NO:10.

The term "IL-17 like polypeptide ortholog" refers to a polypeptide from another species that corresponds to an IL-17 like polypeptide amino acid sequence as set forth in SEQ ID NO:2, SEQ ID NO:4, or SEQ ID NO:10. For example, mouse and human IL-17 like polypeptides are considered orthologs of each other.

The term "IL-17 like polypeptide splice variant" refers to a nucleic acid molecule, usually RNA, which is generated by alternative processing of intron sequences in an RNA transcript of IL-17 like polypeptide amino acid sequence as set forth in SEQ ID NO:2, SEQ ID NO:4, or SEQ ID NO:10.

The term "IL-17 like polypeptide variants" refers to IL-17 like polypeptides comprising amino acid sequences having one or more amino acid sequence substitutions, deletions (such as internal deletions and/or IL-17 like polypeptide fragments), and/or additions (such as internal additions and/or IL-17 like fusion polypeptides) as compared to the IL-17 like polypeptide amino acid sequence set forth in SEQ ID NO:2, SEQ ID NO:4, or SEQ ID NO:10 (with or without a leader sequence). Variants may be naturally occurring (*e.g.*, IL-17 like polypeptide allelic variants, IL-17 like polypeptide orthologs and IL-17 like polypeptide splice variants) or may be artificially constructed. Such IL-17 like polypeptide variants may be prepared from the corresponding nucleic acid molecules having a DNA sequence that varies accordingly from the DNA sequence as set forth in SEQ ID NO:1, SEQ ID NO:3, or SEQ ID NO:9. In preferred embodiments, the variants have from 1 to 3, or from 1 to 5, or from 1 to 10, or from 1 to 15, or from 1 to 20, or from 1 to 25, or from 1 to 50, or from1 to 75, or from 1 to 100, or more than 100 amino acid substitutions, insertions, additions and/or deletions, wherein the substitutions may be conservative, or non-conservative, or any combination thereof.

The term "antigen" refers to a molecule or a portion of a molecule capable of being bound by a selective binding agent, such as an antibody, and additionally capable of being used in an animal to produce antibodies capable of binding to an epitope of each antigen. An antigen may have one or more epitopes.

The term "biologically active IL-17 like polypeptides" refers to IL-17 like polypeptides having at least one activity characteristic of the polypeptide comprising the amino acid sequence of SEQ ID NO:2, SEQ ID NO:4, or SEQ ID NO:10.

The terms "effective amount" and "therapeutically effective amount" each refer to the amount of a IL-17 like polypeptide or IL-17 like nucleic acid molecule used to support an observable level of one or more biological activities of the IL-17 like polypeptides as set forth herein.

The term "expression vector" refers to a vector which is suitable for use in a host cell and contains nucleic acid sequences which direct and/or control the expression of heterologous nucleic acid sequences. Expression includes, but is not limited to, processes such as transcription, translation, and RNA splicing, if introns are present.

The term "host cell" is used to refer to a cell which has been transformed, or is capable of being transformed with a nucleic acid sequence and then of expressing a selected gene of interest. The term includes the progeny of the parent cell, whether or not the progeny is identical in morphology or in genetic make-up to the original parent, so long as the selected gene is present.

The term "identity" as known in the art refers to a relationship between the sequences of two or more polypeptide molecules or two or more nucleic acid molecules, as determined by comparing the sequences. In the art, "identity" also means the degree of sequence relatedness between nucleic acid molecules or polypeptides, as the case may be, as determined by the match between strings of two or more nucleotide or two or more amino acid sequences. "Identity" measures the percent of identical matches between the smaller of two or more sequences with gap alignments (if any) addressed by a particular mathematical model or computer program (i.e., "algorithms").

The term "similarity" is a related concept but, in contrast to "identity", refers to a measure of similarity which includes both identical matches and conservative substitution matches. If two polypeptide sequences have, for example, 10/20 identical amino acids, and the remainder are all non-conservative substitutions, then the percent identity and similarity would both be 50%. If, in the same example, there are five more positions where there are conservative substitutions, then the percent identity remains 50%, but the percent similarity would be 75% (15/20). Therefore, in cases where there are conservative substitutions, the degree of percent similarity between two polypeptides will be higher than the percent identity between those two polypeptides.

The term "isolated nucleic acid molecule" refers to a nucleic acid molecule of the invention that (1) has been separated from at least about 50 percent of proteins, lipids, carbohydrates or other materials with which it is naturally found when total DNA is isolated from the source cells, (2) is not linked to all or a portion of a polynucleotide to which the "isolated nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule(s) or other contaminants that are found in its natural environment that would interfere with its use in polypeptide production or its therapeutic, diagnostic, prophylactic or research use.

The term "isolated polypeptide" refers to a polypeptide of the present invention that (1) has been separated from at least about 50 percent of polynucleotides, lipids, carbohydrates or other materials with which it is naturally found when isolated from the source cell, (2) is not linked (by covalent or noncovalent interaction) to all or a portion of a polypeptide to which the "isolated polypeptide" is linked in nature, (3) is operably linked (by covalent or noncovalent interaction) to a polypeptide with which it is not linked in nature, or (4) does not occur in nature. Preferably, the isolated polypeptide is substantially free from any other contaminating polypeptides or other contaminants that are found in its natural environment that would interfere with its therapeutic, diagnostic, prophylactic or research use.

The term "mature IL-17 like polypeptide" refers to an IL-17 like polypeptide lacking a leader sequence. A mature IL-17 like polypeptide may also include other modifications such as proteolytic processing of the amino terminus (with or without a leader sequence) and/or the carboxy terminus, cleavage of a smaller polypeptide from a larger precursor, N-linked and/or O-linked glycosylation, and the like. An exemplary mature human IL-17 like polypeptide can be found within the amino acid sequence of SEQ ID NO:2. An exemplary mature mouse IL-17 like polypeptide can be found within the amino acid sequence of SEQ ID NO:4 and SEQ ID NO:10. The terms "nucleic acid sequence" or "nucleic acid molecule" refer to a DNA or RNA sequence. The terms encompass molecules formed from any of the known base analogs of DNA and RNA such as, but not limited to 4-acetylcytosine, 8-hydroxy-N6-methyladenosine, aziridinyl-cytosine, pseudoisocytosine, 5-(carboxyhydroxylmethyl) uracil, 5-fluorouracil, 5-bromouracil, 5-carboxymethylaminomethyl-2-thiouracil, 5-carboxy-methylaminomethyluracil, dihydrouracil, inosine, N6-iso-pentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5'-methoxycarbonyl-methyluracil, 5-methoxyuracil, 2-methylthio-N6-iso-

pentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "naturally occurring" or "native" when used in connection with biological materials such as nucleic acid molecules, polypeptides, host cells, and the like, refer to materials which are found in nature and are not manipulated by man. Similarly, "non-naturally occurring" or "non-native" as used herein refers to a material that is not found in nature or that has been structurally modified or synthesized by man.

The term "operably linked" is used herein to refer to a method of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence, and the promoter sequence can still be considered "operably linked" to the coding sequence.

The terms "pharmaceutically acceptable carrier" or "physiologically acceptable carrier" as used herein refer to one or more formulation materials suitable for accomplishing or enhancing the delivery of the IL-17 like polypeptide, IL-17 like nucleic acid molecule or IL-17 like selective binding agent as a pharmaceutical composition.

The term "selective binding agent" refers to a molecule or molecules having specificity for an IL-17 like polypeptide. As used herein the terms, "specific" and "specificity" refer to the ability of the selective binding agents to bind to human IL-17 like polypeptides and not to bind to human non-IL-17 like polypeptides. It will be appreciated, however, that the selective binding agents may also bind orthologs of the polypeptide as set forth in SEQ ID NO:2, SEQ ID NO: 4, or SEQ ID NO:10, that is, interspecies versions thereof, such as mouse and rat polypeptides.

The term "transduction" is used to refer to the transfer of genes from one bacterium to another, usually by a phage. "Transduction" also refers to the acquisition and transfer of eukaryotic cellular sequences by retroviruses.

The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. See, for example, Graham et al., Virology, 52:456 (1973); Sambrook et al., Molecular Cloning, a Laboratory Manual, Cold Spring Harbor Laboratories, New York, (1989); Davis et al., Basic Methods in Molecular Biology, Elsevier, (1986); and Chu et al., Gene, 13:197 (1981). Such techniques can be used to introduce one or more exogenous DNA moieties into suitable host cells.

The term "transformation" as used herein refers to a change in a cells genetic characteristics, and a cell has been transformed when it has been modified to contain new DNA. For example, a cell is transformed where it is genetically modified from its native state. Following transfection or transduction, the transforming DNA may recombine with that of the cell by physically integrating into a chromosome of the cell, it may be maintained transiently as an episomal element without being replicated, or I may replicate independently as a plasmid. A cell is considered to have been stably transformed when the DNA is replicated with the division of the cell.

The term "vector" is used to refer to any molecule (e.g., nucleic acid, plasmid or virus) used to transfer coding information to a host cell.

## Relatedness of Nucleic Acid Molecules and/or Polypeptides

It is understood that related nucleic acid molecules include allelic or splice variants of the nucleic acid molecule of SEQ ID NO:1, SEQ ID NO:3, or SEQ ID NO:9, and include sequences which are complementary to any of the above nucleotide sequences. Related nucleic acid molecules also include a nucleotide sequence encoding a polypeptide comprising or consisting essentially of a substitution, modification, addition and/or deletion of one or more amino acid residues compared to the polypeptide in SEQ ID NO:2, SEQ ID NO:4, or SEQ ID NO:10.

Fragments include molecules which encode a polypeptide of at least about 25 amino acid residues, or about 50, or about 75, or about 100,or greater than about 100, amino acid residues of the polypeptide of SEQ ID NO:2, SEQ ID NO:4, or SEQ ID NO:10.

In addition, related IL-17 like nucleic acid molecules include those molecules which comprise nucleotide sequences which hybridize under moderately or highly stringent conditions as defined herein with the fully complementary sequence of the nucleic acid molecule of SEQ ID NO:1, SEQ ID NO:3, or SEQ ID NO:9, or of a molecule encoding a polypeptide, which polypeptide comprises the amino acid sequence as shown in SEQ ID NO:2, SEQ ID NO:4, or SEQ ID NO:10, or of a nucleic acid fragment as defined herein, or of a nucleic acid fragment encoding a polypeptide as defined herein. Hybridization probes may be prepared using the IL-17 like sequences provided herein to screen cDNA, genomic or synthetic DNA libraries for related sequences. Regions of the DNA and/or amino acid sequence of IL-17 like polypeptide that exhibit significant identity to known sequences are readily determined using sequence alignment algorithms as described herein, and those regions may be used to design probes for screening.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength and the concentration of denaturing agents such as formamide. Examples of "highly stringent conditions" for hybridization and washing are 0.015M sodium chloride, 0.0015M sodium citrate at 65-68°C or 0.015M sodium chloride, 0.0015M sodium citrate, and 50% formamide at 42°C. See Sambrook, Fritsch & Maniatis, Molecular Cloning: A Laboratory Manual, 2nd Ed., Cold Spring Harbor Laboratory, Cold Spring Harbor, N.Y. (1989) and Anderson et al., Nucleic Acid Hybridization: a practical approach, Ch. 4, IRL Press Limited, Oxford, England (1999).

More stringent conditions (such as higher temperature, lower ionic strength, higher formamide, or other denaturing agent) may also be used; however, the rate of hybridization will be affected. Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate ($NaDodSO_4$ or SDS), ficoll, Denhardt's solution, sonicated salmon sperm DNA (or another non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4; however, at typical ionic strength conditions, the rate of hybridization is nearly independent of pH. See Anderson et al., *supra.*

Factors affecting the stability of a DNA duplex include base composition, length, and degree of base pair mismatch. Hybridization conditions can be adjusted by one skilled in the art in order to accommodate these variables and allow DNAs of different sequence relatedness to form hybrids. The melting temperature of a perfectly matched DNA duplex can be estimated by the following equation:

$$T_m(^\circ C) = 81.5 + 16.6(\log[Na+]) + 0.41(\%G+C) - 600/N - 0.72(\%formamide)$$

where N is the length of the duplex formed, [Na+] is the molar concentration of the sodium ion in the hybridization or washing solution, %G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015M sodium chloride, 0.0015M sodium citrate at 50-65°C or 0.015M sodium chloride, 0.0015M sodium citrate, and 20% formamide at 37-50°C. By way of example, a "moderately stringent" condition of 50°C in 0.015 M sodium ion will allow about a 21% mismatch.

It will be appreciated by those skilled in the art that there is no absolute distinction between "highly" and "moderately" stringent conditions. For example, at 0.015M sodium ion (no formamide), the melting temperature of perfectly matched long DNA is about 71°C. With a wash at 65°C (at the same ionic strength), this would allow for approximately a 6% mismatch. To capture more distantly related sequences, one skilled in the art can simply lower the temperature or raise the ionic strength.

A good estimate of the melting temperature in 1M NaCl* for oligonucleotide probes up to about 20nt is given by:

$$Tm = 2^\circ C \text{ per A-T base pair} + 4^\circ C \text{ per G-C base pair}$$

*The sodium ion concentration in 6X salt sodium citrate (SSC) is 1M. See Suggs et al., Developmental Biology Using Purified Genes, p. 683, Brown and Fox (eds.) (1981).

High stringency washing conditions for oligonucleotides are usually at a temperature of 0-5°C below the Tm of the oligonucleotide in 6X SSC, 0.1% SDS.

In another embodiment, related nucleic acid molecules comprise or consist of a nucleotide sequence that is about 70 percent (70%) identical to the nucleotide sequence as shown in SEQ ID NO:1, SEQ ID NO:3, or SEQ ID NO: 9, or comprise or consist essentially of a nucleotide sequence encoding a polypeptide that is about 70 percent (70%) identical to the polypeptide as set forth in SEQ ID NO:2, SEQ ID NO:4, or SEQ ID NO:10. In preferred embodiments, the nucleotide sequences are about 75 percent, or about 80 percent, or about 85 percent, or about 90 percent, or about 95, 96, 97, 98, or 99 percent identical to the nucleotide sequence as shown in SEQ ID NO:1, SEQ ID NO:3, or SEQ ID NO:9, or the nucleotide sequences encode a polypeptide that is about 75 percent, or about 80 percent, or about 85 percent, or about 90 percent, or about 95, 96, 97, 98, or 99 percent identical to the polypeptide sequence as set forth in SEQ ID NO:2, SEQ ID NO:4, or SEQ ID NO:10.

Differences in the nucleic acid sequence may result in conservative and/or non-conservative modifications of the amino acid sequence relative to the amino acid sequence of SEQ ID NO:2, SEQ ID NO:4, or SEQ ID NO:10.

Conservative modifications to the amino acid sequence of SEQ ID NO:2, SEQ ID NO:4, or SEQ ID NO:10 (and corresponding modifications to the encoding nucleotides) will produce IL-17 like polypeptides having functional and chemical characteristics similar to those of a naturally occurring IL-17 like polypeptide. In contrast, substantial modifications in the functional and/or chemical characteristics of IL-17 like polypeptides may be accomplished by selecting substitutions in the amino acid sequence of SEQ ID NO:2, SEQ ID NO:4, or SEQ ID NO:10 that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis."

Conservative amino acid substitutions also encompass non-naturally occurring amino acid residues which are typically incorporated by chemical peptide synthesis rather than by synthesis in biological systems. These include peptidomimetics and other reversed or inverted forms of amino acid moieties.

Naturally occurring residues may be divided into classes based on common side chain properties:

1) hydrophobic: norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

For example, non-conservative substitutions may involve the exchange of a member of one of these classes for a member from another class. Such substituted residues may be introduced into regions of the human IL-17 like polypeptide that are homologous with non-human IL-17 like polypeptide orthologs, or into the non-homologous regions of the molecule.

In making such changes, the hydropathic index of amino acids may be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids may be substituted for other amino acids having a similar hydropathic index or score and still retain a similar biological activity. In making changes based upon the hydropathic index, the substitution of amino acids whose hydropathic indices are within ±2 is preferred, those which are within ±1 are particularly preferred, and those within ±0.5 are even more particularly preferred.

It is also understood in the art that the substitution of like amino acids can be made effectively on the basis of hydrophilicity, particularly where the biologically functional equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, i.e., with a biological property of the protein.

The following hydrophilicity values have been assigned to these amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, the substitution of amino acids whose hydrophilicity values are within ±2 is preferred, those which are within ±1 are particularly preferred, and those within ±0.5 are even more particularly preferred. One may also identify epitopes from primary amino acid sequences on the basis of hydrophilicity. These regions are also referred to as "epitopic core regions."

Desired amino acid substitutions (whether conservative or non-conservative) can be determined by those skilled in the art at the time such substitutions are desired. For example, amino acid substitutions can be used to identify important residues of the IL-17 like polypeptide, or to increase or decrease the affinity of the IL-17 like polypeptides described herein.

Exemplary amino acid substitutions are set forth in Table I.

Table I

| Amino Acid Substitutions | | |
|---|---|---|
| Original Residues | Exemplary Substitutions | Preferred Substitutions |
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of the polypeptide as set forth in SEQ ID NO:2, SEQ ID NO:4, or SEQ ID NO:10 using well-known techniques. For identifying suitable areas of the molecule that may be changed without destroying activity, one skilled in the art may target areas not believed to be important for activity. For example, when similar polypeptides with similar activities from the same species or from other species are known, one skilled in the art may compare the amino acid sequence of an IL-17 like polypeptide to such similar polypeptides. With such a comparison, one can identify residues and portions of the molecules that are conserved among similar polypeptides. It will be appreciated that changes in areas of an IL-17 like polypeptide that are not conserved relative to such similar polypeptides would be less likely to adversely affect the biological activity and/or structure of the IL-17 like polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in an IL-17 like polypeptide that correspond to amino acid residues which are important for activity or structure in similar polypeptides. One skilled in the art may opt for chemically similar amino acid substitutions for such predicted important amino acid residues of IL-17 like polypeptides.

One skilled in the art can also analyze the three-dimensional structure and amino acid sequence in relation to that structure in similar polypeptides. In view of such information, one skilled in the art may predict the alignment of amino acid residues of an IL-17 like polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each desired amino acid residue. The variants can then be screened using activity assays know to those skilled in the art. Such variants could be used to gather information about suitable variants. For example, if one discovered that a change to a particular amino acid residue resulted in destroyed, undesirably reduced, or unsuitable activity, variants with such a change would be avoided. In other words, based on information gathered from such routine experiments, one skilled in the art can readily determine the amino acids where further substitutions should be avoided either alone or in combination with other mutations.

A number of scientific publications have been devoted to the prediction of secondary structure. See Moult J., Curr. Op. in Biotech., 7(4):422-427 (1996), Chou et al., Biochemistry, 13 (2) :222-245 (1974) ; Chou et al., Biochemistry, 113 (2) :211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural database (PDB) has provided enhanced predictability of secondary structure, including the potential number of folds within a polypeptide's or protein's structure. See Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will become dramatically more accurate.

Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (See Holm, supra (1999), and Brenner, supra (1997)).

IL-17 like polypeptide analogs of the invention can be determined by comparing the amino acid sequence of IL-17 like polypeptide with related family members. Exemplary IL-17 like polypeptide related family member are human IL-17 (SEQ ID NO: 5), human IL-20 (SEQ ID NO: 6). Human IL-17B (SEQ ID NO: 7) and human IL-17C (SEQ ID NO: 8). This comparison can be accomplished by using a Pileup alignment (Wisconsin GCG Program Package) or an equivalent (overlapping) comparison with multiple family members within conserved and non-conserved regions.

As shown in Figure 5, the predicted amino acid sequence of human IL-17 like polypeptide (which represent amino acid 37 to 160 of SEQ ID NO: 2) is aligned with a known human IL-17B (SEQ ID NO: 7). Other IL-17 like polypeptide analogs can be determined using these or other methods known to those of skill in the art. These overlapping sequences provide guidance for conservative and non-conservative amino acids substitutions resulting in additional IL-17 like analogs. It will be appreciated that these amino acid substitutions can consist of naturally occurring or non-naturally occurring amino acids. For example, as depicted in Figure 5, alignment of the of related family members indicates potential IL-17 like analogs may have the Asn residue at position 67 of SEQ ID NO: 2 (position 101 on Fig. 5) substituted with a Gln residue, the Arg residue at position 69 of SEQ ID NO: 2 (position 103 on Fig. 5) substituted with a Lys, Gln or Asn residue, and/or the Cys residue at position 94 of SEQ ID NO: 2 (position 128 on Fig. 5) substituted with a Ser or Ala residue. In addition, potential IL-17 like analogs may have the Cys residue at position 96 of SEQ ID NO: 2 (position 130 on Fig. 5) substituted with a Ala or Ser residue, the Val residue at position 101 of SEQ ID NO: 2 (position 132 on Fig. 5) substituted with a Ile, Leu, Met, Phe, Ala, or norleucine residue, the Thr residue at position 104 of SEQ ID NO: 2 (position 138 on Fig. 5) substituted with a Ser residue, the Cys residue at position 129 of SEQ ID NO: 2 (position 163 on Fig. 5) substituted with a Ser or Ala residue, and/or the Cys residue at position 140 of SEQ ID NO: 2 (position 174 on Fig. 5) substituted with a Ser or Ala residue.

Preferred IL-17 like polypeptide variants include glycosylation variants wherein the number and/or type of glycosylation site has been altered compared to the amino acid sequence set forth in SEQ ID NO:2, SEQ ID NO:4, or SEQ ID NO:10. In one embodiment, IL-17 like polypeptide variants comprise a greater or a lesser number of N-linked glycosylation sites than the amino acid sequence set forth in SEQ ID NO:2, SEQ ID NO:4, or SEQ ID NO:10. An N-linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred IL-17 like variants include cysteine variants wherein one or more cysteine residues are deleted from or substituted for another amino acid (e.g., serine) as compared to the amino acid sequence set forth in SEQ ID NO:2, SEQ ID NO:4, or SEQ ID NO:10. Cysteine variants are useful when IL-17 like polypeptides must be refolded into a biologically active conformation such as after the isolation of insoluble inclusion bodies. Cysteine variants generally have fewer cysteine residues than the native protein, and typically have an even number to minimize interactions resulting from unpaired cysteines.

In addition, the polypeptide comprising the amino acid sequence of SEQ ID NO:2, SEQ ID NO:4, or SEQ ID NO:10, or an IL-17 like polypeptide variant may be fused to a homologous polypeptide to form a homodimer or to a heterologous polypeptide to form a heterodimer. Heterologous peptides and polypeptides include, but are not limited to: an epitope to allow for the detection and/or isolation of an IL-17 like fusion polypeptide; a transmembrane receptor protein or a portion thereof, such as an extracellular domain, or a transmembrane and intracellular domain; a ligand or a portion thereof which binds to a transmembrane receptor protein; an enzyme or portion thereof which is catalytically active; a polypeptide or peptide which promotes oligomerization, such as a leucine zipper domain; a polypeptide or peptide which increases stability, such as an immunoglobulin constant region; and a polypeptide which has a therapeutic activity different from the polypeptide comprising the amino acid sequence as set forth in SEQ ID NO:2, SEQ ID NO: 4, or SEQ ID NO:10, or an IL-17 like polypeptide variant.

Fusions can be made either at the amino terminus or at the carboxy terminus of the polypeptide comprising the amino acid sequence set forth in SEQ ID NO:2, SEQ ID NO:4, or SEQ ID NO:10, or an IL-17 like polypeptide variant. Fusions may be direct with no linker or adapter molecule, or indirect using a linker or adapter molecule. A linker or adapter molecule may be one or more amino acid residues, typically from about 20 to about 50 amino acid residues. A linker or adapter molecule may also be designed with a cleavage site for a DNA restriction endonuclease or for a protease to allow for the separation of the fused moieties. It will be appreciated that once constructed, the fusion polypeptides can be derivatized according to the methods described herein.

In a further disclosure, the polypeptide comprising the amino acid sequence of SEQ ID NO:2 or an IL-17 like polypeptide variant is fused to one or more domains of an Fc region of human IgG. Antibodies comprise two functionally independent parts, a variable domain known as "Fab", which binds antigens, and a constant domain known as "Fc", which is involved in effector functions such as complement activation and attack by phagocytic cells. An Fc has a long serum half-life, whereas an Fab is short-lived. Capon et al., Nature, 337:525-31 (1989). When constructed together with a therapeutic protein, an Fc domain can provide longer half-life or incorporate such functions as Fc receptor binding, protein A binding, complement fixation and perhaps even placental transfer. *Id.*
Table II summarizes the use of certain Fc fusions known in the art.

Table II

| Fc Fusion with Therapeutic Proteins | | | |
|---|---|---|---|
| Form of Fc | Fusion partner | Therapeutic implications | Reference |
| IgG1 | N-terminus of CD30-L | Hodgkin's disease; anaplastic lymphoma; T-cell leukemia | U.S. Patent No. 5,480,981 |
| Murine Fcγ2a | IL-10 | anti-inflammatory; transplant rejection | Zheng et al. (1995), J. Immunol., 154: 5590-5600 |
| IgG1 | TNF receptor | septic shock | Fisher et al. (1996), N. Engl. J. Med., 334: 1697-1702; Van Zee et al., (1996), J. Immunol., 156: 2221-2230 |
| IgG, IgA, IgM, or IgE (excluding the first domain) | TNF receptor | inflammation, autoimmune disorders | U.S. Pat. No. 5,808,029, issued September 15, 1998 |
| IgG1 | CD4 receptor | AIDS | Capon et al. (1989), Nature 337: 525-531 |
| IgG1, IgG3 | N-terminus of IL-2 | anti-cancer, antiviral | Harvill et al. (1995), Immunotech., 1: 95-105 |
| IgG1 | C-terminus of OPG | osteoarthriti s; bone density | WO 97/23614, published July 3, 1997 |
| IgG1 | N-terminus of leptin | anti-obesity | PCT/US 97/23183, filed December 11, 1997 |
| Human Ig Cγ1 | CTLA-4 | autoimmune disorders | Linsley (1991), J. Exp. Med., 174:561-569 |

In one example, all or a portion of the human IgG hinge, $CH_2$ and $CH_3$ regions may be fused at either the N-terminus or C-terminus of the IL-17 like polypeptides using methods known to the skilled artisan. The resulting IL-17 like fusion polypeptide may be purified by use of a Protein A affinity column. Peptides and proteins fused to an Fc region have been found to exhibit a substantially greater half-life in vivo than the unfused counterpart. Also, a fusion to an Fc region allows for dimerization/multimerization of the fusion polypeptide. The Fc region may be a naturally occurring Fc region, or may be altered to improve certain qualities, such as therapeutic qualities, circulation time, reduce aggregation, etc.

Identity and similarity of related nucleic acid molecules and polypeptides can be readily calculated by known methods. Such methods include, but are not limited to, those described in Computational Molecular Biology, Lesk, A.M., ed., Oxford University Press, New York (1988); Biocomputing: Informatics and Genome Projects, Smith, D.W., ed., Academic Press, New York (1993); Computer Analysis of Sequence Data, Part 1, Griffin, A.M., and Griffin, H.G., eds., Humana Press, New Jersey (1994); Sequence Analysis in Molecular Biology, von Heinje, G., Academic Press (1987); Sequence Analysis Primer, Gribskov, M. and Devereux, J., eds., M. Stockton Press, New York (1991); and Carillo et al., SIAM J. Applied Math., 48:1073 (1988).

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., Nucl. Acid. Res., 12:387 (1984); Genetics Computer Group, University of Wisconsin, Madison, WI, BLASTP, BLASTN, and FASTA (Altschul et al., J. Mol. Biol., 215: 403-410 (1990)). The BLASTX program is publicly available from the National Center for Biotechnology Information (NCBI) and other sources (BLAST Manual, Altschul et al. NCB/NLM/NIH Bethesda, MD 20894; Altschul et al., *supra* (1990)). The well-known Smith Waterman algorithm may also be used to determine identity.

Certain alignment schemes for aligning two amino acid sequences may result in the matching of only a short region of the two sequences, and this small aligned region may have very high sequence identity even though there is no significant relationship between the two full-length sequences. Accordingly, in a preferred embodiment the selected alignment method (GAP program) will result in an alignment that spans at least 50 contiguous amino acids of the target polypeptide.

For example, using the computer algorithm GAP (Genetics Computer Group, University of Wisconsin, Madison, WI), two polypeptides for which the percent sequence identity is to be determined are aligned for optimal matching of their respective amino acids (the "matched span", as determined by the algorithm). A gap opening penalty (which is calculated as 3X the average diagonal; the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. A standard comparison matrix (see Dayhoff et al., Atlas of Protein Sequence and Structure, 5(3)(1978) for the PAM 250 comparison matrix; Henikoff et al., Proc. Natl. Acad. Sci USA, 89:10915-10919 (1992) for the BLOSUM 62 comparison matrix) is also used by the algorithm.

Preferred parameters for a polypeptide sequence comparison include the following:

Algorithm: Needleman et al., J. Mol. Biol., 48 :443-453 (1970);
Comparison matrix: BLOSUM 62 from Henikoff et *al., supra* (1992);
Gap Penalty: 12
Gap Length Penalty: 4
Threshold of Similarity: 0

The GAP program is useful with the above parameters. The aforementioned parameters are the default parameters for polypeptide comparisons (along with no penalty for end gaps) using the GAP algorithm.

Preferred parameters for nucleic acid molecule sequence comparisons include the following:

Algorithm: Needleman *et al., supra* (1970);
Comparison matrix: matches = +10, mismatch = 0
Gap Penalty: 50
Gap Length Penalty: 3

The GAP program is also useful with the above parameters. The aforementioned parameters are the default parameters for nucleic acid molecule comparisons.

Other exemplary algorithms, gap opening penalties, gap extension penalties, comparison matrices, thresholds of similarity, etc. may be used, including those set forth in the Program Manual, Wisconsin Package, Version 9, September, 1997. The particular choices to be made will be apparent to those of skill in the art and will depend on the specific comparison to be made, such as DNA-to-DNA, protein-to-protein, protein-to-DNA; and additionally, whether the comparison is between given pairs of sequences (in which case GAP or BestFit are generally preferred) or between one sequence and a large database of sequences (in which case FASTA or BLASTA are preferred).

**Synthesis**

It will be appreciated by those skilled in the art the nucleic acid and polypeptide molecules described herein may be produced by recombinant and other means.

**Nucleic Acid Molecules**

The nucleic acid molecules encode a polypeptide comprising the amino acid sequence of an IL-17 like polypeptide and can readily be obtained in a variety of ways including, without limitation, chemical synthesis, cDNA or genomic library screening, expression library screening and/or PCR amplification of cDNA.

Recombinant DNA methods used herein are generally those set forth in Sambrook et al., Molecular Cloning: A Laboratory Manual, Cold Spring Harbor Laboratory Press, Cold Spring Harbor, NY (1989), and/or Ausubel et al., eds., Current Protocols in Molecular Biology, Green Publishers Inc. and Wiley and Sons, NY (1994). The present application discloses nucleic acid molecules as described herein and methods for obtaining such molecules.

Where a gene encoding the amino acid sequence of an IL-17 like polypeptide has been identified from one species, all or a portion of that gene may be used as a probe to identify orthologs or related genes from the same species. The probes or primers may be used to screen cDNA libraries from various tissue sources believed to express the IL-17 like polypeptide. In addition, part or all of a nucleic acid molecule having the sequence as set forth in SEQ ID NO:1, SEQ ID NO:3, or SEQ ID NO:9 may be used to screen a genomic library to identify and isolate a gene encoding the amino acid sequence of an IL-17 like polypeptide. Typically, conditions of moderate or high stringency will be employed for screening to minimize the number of false positives obtained from the screening.

Nucleic acid molecules encoding the amino acid sequence of IL-17 like polypeptides may also be identified by expression cloning which employs the detection of positive clones based upon a property of the expressed protein. Typically, nucleic acid libraries are screened by the binding of an antibody or other binding partner (e.g., receptor or ligand) to cloned proteins which are expressed and displayed on a host cell surface. The antibody or binding partner is modified with a detectable label to identify those cells expressing the desired clone.

Recombinant expression techniques conducted in accordance with the descriptions set forth below may be followed to produce these polynucleotides and to express the encoded polypeptides. For example, by inserting a nucleic acid sequence which encodes the amino acid sequence of an IL-17 like polypeptide into an appropriate vector, one skilled in the art can readily produce large quantities of the desired nucleotide sequence. The sequences can then be used to generate detection probes or amplification primers. Alternatively, a polynucleotide encoding the amino acid sequence of an IL-17 like polypeptide can be inserted into an expression vector. By introducing the expression vector into an appropriate host, the encoded IL-17 like polypeptide may be produced in large amounts.

Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding the amino acid sequence of an IL-17 like polypeptide, are then added to the cDNA along with a polymerase such as *Taq* polymerase, and the polymerase amplifies the cDNA region between the two primers.

Another means of preparing a nucleic acid molecule encoding the amino acid sequence of an IL-17 like polypeptide is chemical synthesis using methods well known to the skilled artisan, such as those described by Engels et al., Angew. Chem. Intl. Ed., 28:716-734 (1989). These methods include, *inter alia,* the phosphotriester, phosphoramidite and H-phosphonate methods for nucleic acid synthesis. A preferred method for such chemical synthesis is polymer-supported synthesis using standard phosphoramidite chemistry. Typically, the DNA encoding the amino acid sequence of an IL-17 like polypeptide will be several hundred nucleotides in length. Nucleic acids larger than about 100 nucleotides can be synthesized as several fragments using these methods. The fragments can then be ligated together to form the full-length nucleotide sequence of an IL-17 like polypeptide. Usually, the DNA fragment encoding the amino terminus of the polypeptide will have an ATG, which encodes a methionine residue. This methionine may or may not be present on the mature form of the IL-17 like polypeptide, depending on whether the polypeptide produced in the host cell is designed to be secreted from that cell. Other methods known to the skilled artisan may be used as well.

In certain embodiments, nucleic acid variants contain codons which have been altered for the optimal expression of an IL-17 like polypeptide in a given host cell. Particular codon alterations will depend upon the IL-17 like polypeptide (s) and host cell(s) selected for expression. Such "codon optimization" can be carried out by a variety of methods, for example, by selecting codons which are preferred for use in highly expressed genes in a given host cell. Computer algorithms which incorporate codon frequency tables such as "Ecohigh.cod" for codon preference of highly expressed bacterial genes may be used and are provided by the University of Wisconsin Package Version 9.0, Genetics Computer Group, Madison, WI. Other useful codon frequency tables include "Celegans_high.cod", "Celegans_low.cod", "Drosophila_high.cod", "Human_high.cod", "Maize_high.cod", and "Yeast_high.cod".

**Vectors and Host Cells**

A nucleic acid molecule encoding the amino acid sequence of an IL-17 like polypeptide may be inserted into an appropriate expression vector using standard ligation techniques. The vector is typically selected to be functional in the particular host cell employed (i.e., the vector is compatible with the host cell machinery such that amplification of the gene and/or expression of the gene can occur). A nucleic acid molecule encoding the amino acid sequence of an IL-17 like polypeptide may be amplified/expressed in prokaryotic, yeast, insect (baculovirus systems), and/or eukaryotic host cells. Selection of the host cell will depend in part on whether an IL-17 like polypeptide is to be post-translationally modified (e.g., glycosylated and/or phosphorylated). If so, yeast, insect, or mammalian host cells are preferable. For a review of expression vectors, see Meth. Enz., vol.185, D.V. Goeddel, ed., Academic Press Inc., San Diego, CA (1990).

Typically, expression vectors used in any of the host cells will contain sequences for plasmid maintenance and for cloning and expression of exogenous nucleotide sequences. Such sequences, collectively referred to as "flanking sequences" in certain embodiments, will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, i.e., an oligonucleotide molecule located at the 5' or 3' end of the IL-17 like polypeptide coding sequence; the oligonucleotide sequence encodes polyHis (such as hexaHis), or another "tag" such as FLAG, HA (hemaglutinin influenza virus) or *myc* for which commercially available antibodies exist. This tag is typically fused to the polypeptide upon expression of the polypeptide, and can serve as a means for affinity purification of the IL-17 like polypeptide from the host cell. Affinity purification can be accomplished, for example, by column chromatography using antibodies against the tag as an affinity matrix. Optionally, the tag can subsequently be removed from the purified IL-17 like polypeptide by various means such as using certain peptidases for cleavage.

Flanking sequences may be homologous (*i.e.*, from the same species and/or strain as the host cell), heterologous (*i.e.*, from a species other than the host cell species or strain), hybrid (*i.e.*, a combination of flanking sequences from more than one source) or synthetic, or the flanking sequences may be native sequences which normally function to regulate IL-17 like polypeptide expression. As such, the source of a flanking sequence may be any prokaryotic or eukaryotic organism, any vertebrate or invertebrate organism, or any plant, provided that the flanking sequence is functional in, and can be activated by, the host cell machinery.

The flanking sequences useful in the vectors of this invention may be obtained by any of several methods well known in the art. Typically, flanking sequences useful herein other than the IL-17 like gene flanking sequences will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Where all or only a portion of the flanking sequence is known, it may be obtained using PCR and/or by screening a genomic library with suitable oligonucleotide and/or flanking sequence fragments from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen® column chromatography (Chatsworth, CA), or other methods known to the skilled artisan. The selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. Amplification of the vector to a certain copy number can, in some cases, be important for the optimal expression of an IL-17 like polypeptide. If the vector of choice does not contain an origin of replication site, one may be chemically synthesized based on a known sequence, and ligated into the vector. For example, the origin of replication from the plasmid pBR322 (Product No. 303-3s, New England Biolabs, Beverly, MA) is suitable for most gram-negative bacteria, and various origins (*e.g.,* SV40, polyoma, adenovirus, vesicular stomatitus virus (VSV) or papillomaviruses such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding region and serves to terminate transcription.. Usually, a transcription termination sequence in prokaryotic cells is a G-C rich fragment followed by a poly T sequence. While the sequence is easily cloned from a library or even purchased commercially as part of a vector, it can also be readily synthesized using methods for nucleic acid synthesis such as those described herein.

A selectable marker gene element encodes a protein necessary for the survival and growth of a host cell grown in a selective culture medium. Typical selection marker genes encode proteins that (a) confer resistance to antibiotics or other toxins, e.g., ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene which will be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure which only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the DNA that encodes an IL-17 like polypeptide. As a result, increased quantities of IL-17 like polypeptide are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of an IL-17 like polypeptide to be expressed. The Shine-Dalgarno sequence is varied but is typically a polypurine (i.e., having a high A-G content). Many Shine-Dalgarno sequences have been identified, each of which can be readily synthesized using methods set forth herein and used in a prokaryotic vector.

A leader, or signal, sequence may be used to direct an IL-17 like polypeptide out of the host cell. Typically, a nucleotide sequence encoding the signal sequence is positioned in the coding region of an IL-17 like nucleic acid molecule, or directly at the 5' end of an IL-17 like polypeptide coding region. Many signal sequences have been identified, and any of those that are functional in the selected host cell may be used in conjunction with an IL-17 like nucleic acid molecule. Therefore, a signal sequence may be homologous (naturally occurring) or heterologous to an IL-17 like gene or cDNA. Additionally, a signal sequence may be chemically synthesized using methods described herein. In most cases, the secretion of an IL-17 like polypeptide from the host cell via the presence of a signal peptide will result in the removal of the signal peptide from the secreted IL-17 like polypeptide. The signal sequence may be a component of the vector, or it may be a part of an IL-17 like nucleic acid molecule that is inserted.into the vector.

Included within the scope of this disclosure is the use of either a nucleotide sequence encoding a native IL-17 like polypeptide signal sequence joined to an IL-17 like polypeptide coding region or a nucleotide sequence encoding a heterologous signal sequence joined to an IL-17 like polypeptide coding region. The heterologous signal sequence selected should be one that is recognized and processed, i.e., cleaved by a signal peptidase, by the host cell. For prokaryotic host cells that do not recognize and process the native IL-17 like polypeptide signal sequence, the signal sequence is substituted by a prokaryotic signal sequence selected, for example, from the group of the alkaline phosphatase, penicillinase, or heat-stable enterotoxin II leaders. For yeast secretion, the native IL-17 like polypeptide signal sequence may be substituted by the yeast invertase, alpha factor, or acid phosphatase leaders. In mammalian cell expression the native signal sequence is satisfactory, although other mammalian signal sequences may be suitable.

In some cases, such as where glycosylation is desired in a eukaryotic host cell expression system, one may manipulate the various presequences to improve glycosylation or yield. For example, one may alter the peptidase cleavage site of a particular signal peptide, or add presequences, which also may affect glycosylation. The final protein product may have, in the -1 position (relative to the first amino acid of the mature protein), one or more additional amino acids incident to expression which may not have been totally removed. For example, the final protein product may have one or two amino acid residues found in the peptidase cleavage site, attached to the N-terminus. Alternatively, use of some enzyme cleavage sites may result in a slightly truncated form of the desired IL-17 like polypeptide, if the enzyme cuts at such area within the mature polypeptide.

In many cases, transcription of a nucleic acid molecule is increased by the presence of one or more introns in the vector; this is particularly true where a polypeptide is produced in eukaryotic host cells, especially mammalian host cells. The introns used may be naturally occurring within the IL-17 like gene, especially where the gene used is a full length genomic sequence or a fragment thereof. Where the intron is not naturally occurring within the gene (as for most cDNAs), the intron(s) may be obtained from another source. The position of the intron with respect to flanking sequences and the IL-17 like gene is generally important, as the intron must be transcribed to be effective. Thus, when an IL-17 like cDNA molecule is being transcribed, the preferred position for the intron is 3' to the transcription start site, and 5' to the polyA transcription termination sequence. Preferably, the intron or introns will be located on one side or the other (i.e., 5' or 3') of the cDNA such that it does not interrupt the coding sequence. Any intron from any source, including viral, prokaryotic and eukaryotic (plant or animal) organisms, may be used to practice this invention, provided that it is compatible with the host cell(s) into which it is inserted. Also included herein are synthetic introns. Optionally, more than one intron may be used in the vector.

The expression and cloning vectors disclosed herein will each typically contain a promoter that is recognized by the host organism and operably linked to the molecule encoding an IL-17 like polypeptide. Promoters are untranscribed sequences located upstream (5') to the start codon of a structural gene (generally within about 100 to 1000 bp) that control the transcription of the structural gene. Promoters are conventionally grouped into one of two classes, inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A suitable promoter is operably linked to the DNA encoding an IL-17 like polypeptide by removing the promoter from the source DNA by restriction enzyme digestion and inserting the desired promoter sequence into the vector. The native IL-17 like gene promoter sequence may be used to direct amplification and/or expression of an IL-17 like nucleic acid molecule. A heterologous promoter is preferred, however, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; alkaline phosphatase, a tryptophan (trp) promoter system; and hybrid promoters such as the tac promoter. Other known bacterial promoters are also suitable. Their sequences have been published, thereby enabling one skilled in the art to ligate them to the desired DNA sequence(s), using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowl pox virus, adenovirus (such as Adenovirus 2), bovine papilloma virus, avian sarcoma virus, cytomegalovirus (CMV), a retrovirus, hepatitis-B virus and most preferably Simian Virus 40 (SV40). Other suitable mammalian promoters include heterologous mammalian promoters, *e.g.,* heat-shock promoters and the actin promoter.

Additional promoters which may be of interest in controlling IL-17 like gene transcription include, but are not limited to: the SV40 early promoter region (Bernoist and Chambon, Nature, 290:304-310, (1981)), the CMV promoter, the promoter contained in the 3' long terminal repeat of Rous sarcoma virus (Yamamoto et al., Cell, 22:787-797, (1980)); the herpes thymidine kinase promoter (Wagner et al., Proc. Natl. Acad. Sci. USA, 78:144-145, (1981)), the regulatory sequences of the metallothionine gene (Brinster et al., Nature, 296:39-42, (1982)), prokaryotic expression vectors such as the beta-lactamase promoter (Villa-Kamaroff, et al., Proc. Natl. Acad. Sci. USA, 75:3727-3731, (1978)), or the tac promoter (DeBoer, et al., Proc. Natl. Acad. Sci. USA, 80:21-25, (1983)). Also of interest are the following animal transcriptional control regions, which exhibit tissue specificity and have been utilized in transgenic animals: the elastase I gene control region which is active in pancreatic acinar cells [Swift et al., Cell, 38:639-646, (1984); Ornitz et al., Cold Spring Harbor Symp. Quant. Biol., 50:399-409, (1986); MacDonald, Hepatology, 7:425-515 (1987)]; the insulin gene control region which is active in pancreatic beta cells (Hanahan, Nature, 315:115-122, (1985)); the immunoglobulin gene control region which is active in lymphoid cells (Grosschedl et al., Cell, 38:647-658 (1984)); Adames et al., Nature, 318: 533-538, (1985)); (Alexander et al., Mol. Cell. Biol., 7:1436-1444, (1987)); the mouse mammary tumor virus control region which is active in testicular, breast, lymphoid and mast cells (Leder et al., Cell, 45:485-495, (1986)); the albumin gene control region which is active in liver (Pinkert et al., Genes and Devel., 1:268-276, (1987)); the alphafetoprotein gene control region which is active in liver (Krumlauf et al., Mol. Cell. Biol., 5:1639-1648, (1985)); Hammer et al., Science, 235:53-58, (1987)); the alpha 1-antitrypsin gene control region which is active in the liver (Kelsey et al., Genes and Devel., 1:161-171, (1987)); the beta-globin gene control region which is active in myeloid cells [Mogram et al., Nature, 315:338-340, (1985); Kollias et al., Cell, 46:89-94, (1986)]; the myelin basic protein gene control region which is active in oligodendrocyte cells in the brain (Readhead et al., Cell, 48:703-712, (1987)); the myosin light chain-2 gene control region which is active in skeletal muscle (Sani, Nature, 314:283-286, (1985)); and the gonadotropic releasing hormone gene control region which is active in the hypothalamus (Mason et al., Science, 234:1372-1378, (1986)).

An enhancer sequence may be inserted into the vector to increase the transcription of a DNA encoding an IL-17 like polypeptide as disclosed herein by higher eukaryotes. Enhancers are cis-acting elements of DNA, usually about 10-300 bp in length, that act on the promoter to increase transcription. Enhancers are relatively orientation and position independent. They have been found 5' and 3' to the transcription unit. Several enhancer sequences available from mammalian genes are known (e.g., globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to an IL-17 like nucleic acid molecule, it is typically located at a site 5' from the promoter.

Expression vectors disclosed herein may be constructed from a starting vector such as a commercially available vector. Such vectors may or may not contain all of the desired flanking sequences. Where one or more of the desired flanking sequences are not already present in the vector, they may be individually obtained and ligated into the vector. Methods used for obtaining each of the flanking sequences are well known to one skilled in the art.

Preferred vectors for practicing this disclosure are those which are compatible with bacterial, insect, and mammalian host cells. Such vectors include, inter alia, pCRII, pCR3, and pcDNA3.1 (Invitrogen Company, Carlsbad, CA), pBSII (Stratagene Company, La Jolla, CA), pET15□ (Novagen, Madison, WI), pGEX (Pharmacia Biotech, Piscataway, NJ), pEGFP-N2 (Clontech, Palo Alto, CA), pETL (BlueBacII; Invitrogen), pDSR-alpha (PCT Publication No. WO 90/14363) and pFastBacDual (Gibco/BRL, Grand Island, NY).

Additional suitable vectors include, but are not limited to, cosmids, plasmids or modified viruses, but it will be appreciated that the vector system must be compatible with the selected host cell. Such vectors include, but are not limited to, plasmids such as Bluescript® plasmid derivatives (a high copy number ColE1-based phagemid, Stratagene Cloning Systems Inc., La Jolla CA), PCR cloning plasmids designed for cloning Taq-amplified PCR products (e.g., TOPO™ TA Cloning® Kit, PCR2.1 plasmid derivatives, Invitrogen, Carlsbad, CA), and mammalian, yeast, or virus vectors such as a baculovirus expression system (pBacPAK plasmid derivatives, Clontech, Palo Alto, CA).

After the vector has been constructed and a nucleic acid molecule encoding an IL-17 like polypeptide has been inserted into the proper site of the vector, the completed vector may be inserted into a suitable host cell for amplification and/or polypeptide expression. The transformation of an expression vector for an IL-17 like polypeptide into a selected host cell may be accomplished by well-known methods such as transfection, infection, calcium chloride, electroporation, microinjection, lipofection or the DEAE-dextran method or other known techniques. The method selected will in part be a function of the type of host cell to be used. These methods and other suitable methods are well known to the skilled artisan and are set forth, for example, in Sambrook et al., supra.

Host cells may be prokaryotic host cells (such as *E. coli*) or eukaryotic host cells (such as yeast, an insect or vertebrate cells). The host cell, when cultured under appropriate conditions, synthesizes an IL-17 like polypeptide which can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity (such as glycosylation or phosphorylation), and ease of folding into a biologically active molecule.

A number of suitable host cells are known in the art and many are available from the American Type Culture Collection (ATCC), 10801 University Boulevard, Manassas, VA 20110-2209. Examples include, but are not limited to, mammalian cells, such as Chinese hamster ovary cells (CHO) (ATCC No. CCL61); CHO DHFR-cells (Urlaub et al., Proc. Natl. Acad. Sci. USA, 97:4216-4220 (1980)); human embryonic kidney (HEK) 293 or 293T cells (ATCC No. CRL1573); or 3T3 cells (ATCC No. CCL92). The selection of suitable mammalian host cells and methods for transformation, culture, amplification, screening, product production and purification are known in the art. Other suitable mammalian cell lines are the monkey COS-1 (ATCC No. CRL1650) and COS-7 (ATCC No. CRL1651) cell lines, and the CV-1 cell line (ATCC No. CCL70). Further exemplary mammalian host cells include primate cell lines and rodent cell lines, including transformed cell lines. Normal diploid cells, cell strains derived from in vitro culture of primary tissue, as well as primary explants, are also suitable. Candidate cells may be genotypically deficient in the selection gene, or may contain a dominant acting selection gene. Other suitable mammalian cell lines include, but are not limited to, mouse neuroblastoma N2A cells, HeLa, mouse L-929 cells, 3T3 lines derived from Swiss, Balb-c or NIH mice, BHK or HaK hamster cell lines, which are available from the ATCC. Each of these cell lines is known by and available to those skilled in the art of protein expression.

Similarly useful as host cells suitable for the present invention are bacterial cells. For example, the various strains of E. *coli (e.g.,* HB101, (ATCC No. 33694) DH5$\alpha$, DH10 and MC1061 (ATCC No. 53338)) are well known as host cells in the field of biotechnology. Various strains of B. *subtilis, Pseudomonas spp.,* other *Bacillus spp., Streptomyces spp.*, and the like may also be employed in this method.

Many strains of yeast cells known to those skilled in the art are also available as host cells for expression of the polypeptides of the present invention. Preferred yeast cells include, for example, *Saccharomyces cerivisae* and *Pichia pastoris.*

Additionally, where desired, insect cell systems may be utilized in the methods of the present invention. Such systems are described for example in Kitts et al., Biotechniques, 14:810-817 (1993); Lucklow, Curr. Opin. Biotechnol., 4:564-572 (1993); and Lucklow et al., J. Virol., 67:4566-4579 (1993). Preferred insect cells are Sf-9 and Hi5 (Invitrogen, Carlsbad, CA).

One may also use transgenic animals to express glycosylated IL-17 like polypeptides. For example, one may use a transgenic milk-producing animal (a cow or goat, for example) and obtain the present glycosylated polypeptide in the animal milk. One may also use plants to produce IL-17 like polypeptides; however, in general, the glycosylation occurring in plants is different from that produced in mammalian cells, and may result in a glycosylated product which is not suitable for human therapeutic use.

Polypeptide Production

Host cells comprising an IL-17 like polypeptide expression vector may be cultured using standard media well known to the skilled artisan. The media will usually contain all nutrients necessary for the growth and survival of the cells. Suitable media for culturing *E. coli* cells include, for example, Luria Broth (LB) and/or Terrific Broth (TB). Suitable media for culturing eukaryotic cells include Roswell Park Memorial Institute medium 1640 (RPMI 1640), Minimal Essential Medium (MEM) and/or Dulbecco's Modified Eagle Medium (DMEM), all of which may be supplemented with serum and/or growth factors as indicated by the particular cell line being cultured. A suitable medium for insect cultures is Grace's medium supplemented with yeastolate, lactalbumin hydrolysate and/or fetal calf serum, as necessary.

Typically, an antibiotic or other compound useful for selective growth of transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline and neomycin.

The amount of an IL-17 like polypeptide produced by a host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays such as DNA binding gel shift assays.

If an IL-17 like polypeptide has been designed to be secreted from the host cells, the majority of polypeptide may be found in the cell culture medium. If however, the IL-17 like polypeptide is not secreted from the host cells, it will be present in the cytoplasm and/or the nucleus (for eukaryotic host cells) or in the cytosol (for bacterial host cells).

For an IL-17 like polypeptide situated in the host cell cytoplasm and/or the nucleus (for eukaryotic host cells) or in the cytosol (for bacterial host cells), intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation.

If an IL-17 like polypeptide has formed inclusion bodies in the cytosol, the inclusion bodies can often bind to the inner and/or outer cellular membranes and thus will be found primarily in the pellet material after centrifugation. The pellet material can then be treated at pH extremes or with a chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The IL-17 like polypeptide in its now soluble form can then be analyzed using gel electrophoresis, immunoprecipitation or the like. If it is desired to isolate the IL-17 like polypeptide, isolation may be accomplished using standard methods such as those described herein and in Marston et al., Meth. Enz., 182:264-275 (1990).

In some cases, an IL-17 like polypeptide may not be biologically active upon isolation. Various methods for "refolding" or converting the polypeptide to its tertiary structure and generating disulfide linkages can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol(DTT)/ dithiane DTT, and 2-2mercaptoethanol(bME)/dithio-b(ME). A cosolvent may be used to increase the efficiency of the refolding, and the more common reagents used for this purpose include glycerol, polyethylene glycol of various molecular weights, arginine and the like.

If inclusion bodies are not formed to a significant degree upon expression of an IL-17 like polypeptide, then the polypeptide will be found primarily in the supernatant after centrifugation of the cell homogenate. The polypeptide may be further isolated from the supernatant using methods such as those described herein.

The purification of an IL-17 like polypeptide from solution can be accomplished using a variety of techniques. If the polypeptide has been synthesized such that it contains a tag such as Hexahistidine (IL-17 like polypeptide/hexaHis) or other small peptide such as FLAG (Eastman Kodak Co., New Haven, CT) or *myc* (Invitrogen, Carlsbad, CA) at either its carboxyl or amino terminus, it may be purified in a one-step process by passing the solution through an affinity column where the column matrix has a high affinity for the tag.

For example, polyhistidine binds with great affinity and specificity to nickel; thus affinity column of nickel (such as the Qiagen® nickel columns) can be used for purification of IL-17 like polypeptide/polyHis. See for example, Ausubel et al., eds., Current Protocols in Molecular Biology, Section 10.11.8, John Wiley & Sons, New York (1993).

Additionally, the IL-17-like polypeptide may be purified through use of a monoclonal antibody which is capable of specifically recognizing and binding to the IL-17-like polypeptide.

Suitable procedures for purification thus include, without limitation, affinity chromatography, immunoaffinity chromatography, ion exchange chromatography, molecular sieve chromatography, High Performance Liquid Chromatography (HPLC), electrophoresis (including native gel electrophoresis) followed by gel elution, and preparative isoelectric focusing ("Isoprime" machine/technique, Hoefer Scientific, San Francisco, CA). In some cases, two or more purification techniques may be combined to achieve increased purity.

IL-17 like polypeptides may also be prepared by chemical synthesis methods (such as solid phase peptide synthesis) using techniques known in the art, such as those set forth by Merrifield et al., J. Am. Chem. Soc., 85:2149 (1963), Houghten et al., Proc. Natl. Acad. Sci. USA, 82:5132 (1985*), and* Stewart and Young, "Solid Phase Peptide Synthesis", Pierce Chemical Co., Rockford, IL (1984). Such polypeptides may be synthesized with or without a methionine on the amino terminus. Chemically synthesized IL-17 like polypeptides may be oxidized using methods set forth in these references to form disulfide bridges. Chemically synthesized IL-17 like polypeptides are expected to have comparable biological activity to the corresponding IL-17 like polypeptides produced recombinantly or purified from natural sources, and thus may be used interchangeably with a recombinant or natural IL-17 like polypeptide.

Another means of obtaining an IL-17 like polypeptide is via purification from biological samples such as source tissues and/or fluids in which the IL-17 like polypeptide is naturally found. Such purification can be conducted using methods for protein purification as described herein. The presence of the IL-17 like polypeptide during purification may be monitored, for example, using an antibody prepared against recombinantly produced IL-17 like polypeptide or peptide fragments thereof.

A number of additional methods for producing nucleic acids and polypeptides are known in the art, and the methods can be used to produce polypeptides having specificity for IL-17 like. See for example, Roberts et al., Proc. Natl. Acad. Sci. USA, 94:12297-12303 (1997), which describes the production of fusion proteins between an mRNA and its encoded peptide. See also Roberts, R., Curr. Opin. Chem. Biol., 3:268-273 (1999). Additionally, U.S. Patent No. 5,824,469 describes methods of obtaining oligonucleotides capable of carrying out a specific biological function. The procedure involves generating a heterogeneous pool of oligonucleotides, each having a 5' randomized sequence, a central preselected sequence, and a 3' randomized sequence. The resulting heterogeneous pool is introduced into a population of cells that do not exhibit the desired biological function. Subpopulations of the cells are then screened for those which exhibit a predetermined biological function. From that subpopulation, oligonucleotides capable of carrying out the desired biological function are isolated.

U.S. Patent Nos. 5,763,192, 5,814,476, 5,723,323 and 5,817,483 describe processes for producing peptides or polypeptides. This is done by producing stochastic genes or fragments thereof, and then introducing these genes into host cells which produce one or more proteins encoded by the stochastic genes. The host cells are then screened to identify those clones producing peptides or polypeptides having the desired activity.

Another method for producing peptides or polypeptides is described in PCT/US98/20094 (WO99/15650) filed by Athersys, Inc. Known as "Random Activation of Gene Expression for Gene Discovery" (RAGE-GD), the process involves the activation of endogenous gene expression or over-expression of a gene by in situ recombination methods. For example, expression of an endogenous gene is activated or increased by integrating a regulatory sequence into the target cell which is capable of activating expression of the gene by non-homologous or illegitimate recombination. The target DNA is first subjected to radiation, and a genetic promoter inserted. The promoter eventually locates a break at the front of a gene, initiating transcription of the gene. This results in expression of the desired peptide or polypeptide.

It will be appreciated that these methods can also be used to create comprehensive IL-17 like protein expression libraries, which can subsequently be used for high throughput phenotypic screening in a variety of assays, such as biochemical assays, cellular assays, and whole organism assays (e.g., plant, mouse, etc.).

## Chemical Derivatives

Chemically modified derivatives of the IL-17 like polypeptides may be prepared by one skilled in the art, given the disclosures set forth hereinbelow. IL-17 like polypeptide derivatives are modified in a manner that is different either in the type or location of the molecules naturally attached to the polypeptide. Derivatives may include molecules formed by the deletion of one or more naturally-attached chemical groups. The polypeptide comprising the amino acid sequence of SEQ ID NO:2, SEQ ID NO:4, or SEQ ID NO:10, or an IL-17 like polypeptide variant, may be modified by the covalent attachment of one or more polymers.

For example, the polymer selected is typically water soluble so that the protein to which it is attached does not precipitate in an aqueous environment, such as a physiological environment. Included within the scope of suitable polymers is a mixture of polymers. Preferably, for therapeutic use of the end-product preparation, the polymer will be pharmaceutically acceptable.

The polymers each may be of any molecular weight and may be branched or unbranched. The polymers each typically have an average molecular weight of between about 2kDa to about 100kDa (the term "about" indicating that in preparations of a water soluble polymer, some molecules will weigh more, some less, than the stated molecular weight). The average molecular weight of each polymer is preferably between about 5kDa, about 50kDa, more preferably between about 12kDa to about 40kDa and most preferably between about 20kDa to about 35kDa.

Suitable water soluble polymers or mixtures thereof include, but are not limited to, N-linked or O-linked carbohydrates; sugars; phosphates; polyethylene glycol (PEG) (including the forms of PEG that have been used to derivatize proteins, including mono-($C_1$-$C_{10}$) alkoxy- or aryloxy-polyethylene glycol); monomethoxy-polyethylene glycol; dextran (such as low molecular weight dextran of, for example, about 6 kDa);, cellulose; or other carbohydrate-based polymers, poly-(N-vinyl pyrrolidone) polyethylene glycol, propylene glycol homopolymers, a polypropylene oxide/ethylene oxide co-polymer, polyoxyethylated polyols (e.g., glycerol) and polyvinyl alcohol. Also encompassed by the present invention are bifunctional crosslinking molecules which may be used to prepare covalently attached multimers of the polypeptide comprising the amino acid sequence of SEQ ID NO:2 or an IL-17 like polypeptide variant.

In general, chemical derivatization may be performed under any suitable condition used to react a protein with an activated polymer molecule. Methods for preparing chemical derivatives of polypeptides will generally comprise the steps of (a) reacting the polypeptide with the activated polymer molecule (such as a reactive ester or aldehyde derivative of the polymer molecule) under conditions whereby the polypeptide comprising the amino acid sequence of SEQ ID NO: 2, SEQ ID NO:4, or SEQ ID NO:10, or an IL-17 like polypeptide variant becomes attached to one or more polymer molecules, and (b) obtaining the reaction product(s). The optimal reaction conditions will be determined based on known parameters and the desired result. For example, the larger the ratio of polymer molecules:protein, the greater the percentage of attached polymer molecule. In one embodiment, the IL-17 like polypeptide derivative may have a single polymer molecule moiety at the amino terminus (see for example, U.S. Patent No. 5,234,784).

The pegylation of the polypeptide may be specifically carried out by any of the pegylation reactions known in the art, as described for example in the following references: Francis et al., Focus on Growth Factors, 3:4-10 (1992); EP 0154316; EP 0401384 and U.S. Patent No. 4,179,337. For example, pegylation may be carried out via an acylation reaction or an alkylation reaction with a reactive polyethylene glycol molecule (or an analogous reactive water-soluble polymer) as described herein. For the acylation reactions, the polymer(s) selected should have a single reactive ester group. For reductive alkylation, the polymer(s) selected should have a single reactive aldehyde group. A reactive aldehyde is, for example, polyethylene glycol propionaldehyde, which is water stable, or mono $C_1$-$C_{10}$ alkoxy or aryloxy derivatives thereof (see U.S. Patent No. 5,252,714).

In another embodiment, IL-17 like polypeptides may be chemically coupled to biotin, and the biotin/IL-17 like polypeptide molecules which are conjugated are then allowed to bind to avidin, resulting in tetravalent avidin/biotin/IL-17 like polypeptide molecules. IL-17 like polypeptides may also be covalently coupled to dinitrophenol (DNP) or trinitrophenol (TNP) and the resulting conjugates precipitated with anti-DNP or anti-TNP-IgM to form decameric conjugates with a valency of 10.

Generally, conditions which may be alleviated or modulated by the administration of the present IL-17 like polypeptide derivatives include those described herein for IL-17 like polypeptides. However, the IL-17 like polypeptide derivatives disclosed herein may have additional activities, enhanced or reduced biological activity, or other characteristics, such as increased or decreased half-life, as compared to the non-derivatized molecules.

## Genetically Engineered Non-Human Animals

Additionally included within the scope of the present dislosure are non-human animals such as mice, rats or other rodents, rabbits, goats or sheep, or other farm animals, in which the gene (or genes) encoding the native IL-17 like polypeptide has (have) been disrupted ("knocked out") such that the level of expression of this gene or genes is significantly decreased or completely abolished. Such animals may be prepared using techniques and methods such as those described in U.S. Patent No. 5,557,032.

The present disclosure further includes non-human animals such as mice, rats or other rodents, rabbits, goats, sheep, or other farm animals, in which either the native form of the IL-17 like gene(s) for that animal or a heterologous IL-17 like gene(s) is (are) over-expressed by the animal, thereby creating a "transgenic" animal. Such transgenic animals may be prepared using well-known methods such as those described in U.S. Patent No. 5,489,743 and PCT Application No. WO 94/28122.

The present disclosure further includes non-human animals in which the promoter for one or more of the IL-17 like polypeptides of the present disclosure is either activated or inactivated (e.g., by using homologous recombination methods) to alter the level of expression of one or more of the native IL-17 like polypeptides.

These non-human animals may be used for drug candidate screening. In such screening, the impact of a drug candidate on the animal may be measured; for example, drug candidates may decrease or increase the expression of the IL-17 like gene. In certain embodiments, the amount of IL-17 like polypeptide that is produced may be measured after the exposure of the animal to the drug candidate. Additionally, in certain embodiments, one may detect the actual impact of the drug candidate on the animal. For example, the overexpression of a particular gene may result in, or be associated with, a disease or pathological condition. In such cases, one may test a drug candidate's ability to decrease expression of the gene or its ability to prevent or inhibit a pathological condition. In other examples, the production of a particular metabolic product such as a fragment of a polypeptide, may result in, or be associated with, a disease or pathological condition. In such cases, one may test a drug candidate's ability to decrease the production of such a metabolic product or its ability to prevent or inhibit a pathological condition.

## Microarray

It will be appreciated that DNA microarray technology can be utilized in accordance with the present disclosure. DNA microarrays are miniature, high density arrays of nucleic acids positioned on a solid support, such as glass. Each cell or element within the array has numerous copies of a single species of DNA which acts as a target for hybridization for its cognate mRNA. In expression profiling using DNA microarray technology, mRNA is first extracted from a cell or tissue sample and then converted enzymatically to fluorescently labeled cDNA. This material is hybridized to the microarray and unbound cDNA is removed by washing. The expression of discrete genes represented on the array is then visualized by quantitating the amount of labeled cDNA which is specifically bound to each target DNA. In this way, the expression of thousands of genes can be quantitated in a high throughput, parallel manner from a single sample of biological material.

This high throughput expression profiling has a broad range of applications with respect to the IL-17-like molecules of the invention, including but not limited to: the identification and validation of IL-17-like disease-related genes as targets for therapeutics; molecular toxicology of IL-17-like molecules and inhibitors thereof; stratification of populations and generation of surrogate markers for clinical trials; and the enhancement of an IL-17-like related small molecule drug discovery by aiding in the identification of selective compounds in high throughput screens (HTS).

## Selective Binding Agents

As used herein, the term "selective binding agent" refers to a molecule which has specificity for one or more IL-17 like polypeptides. Suitable selective binding agents include, but are not limited to, antibodies and derivatives thereof, polypeptides, and small molecules. Suitable selective binding agents may be prepared using methods known in the art. An exemplary IL-17 like polypeptide selective binding agent of the present invention is capable of binding a certain portion of the IL-17 like polypeptide thereby inhibiting the binding of the polypeptide to the IL-17 like polypeptide receptor(s).

Selective binding agents such as antibodies and antibody fragments that bind IL-17 like polypeptides are within the scope of the present disclosure. The antibodies may be polyclonal including monospecific polyclonal, monoclonal (MAbs), recombinant, chimeric, humanized such as CDR-grafted, human, single chain, and/or bispecific, as well as fragments, variants or derivatives thereof. Antibody fragments include those portions of the antibody which bind to an epitope on the IL-17 like polypeptide. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Polyclonal antibodies directed toward an IL-17 like polypeptide generally are produced in animals (e.g., rabbits or mice) by means of multiple subcutaneous or intraperitoneal injections of IL-17 like polypeptide and an adjuvant. It may be useful to conjugate an IL-17 like polypeptide to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-IL-17 like polypeptide antibody titer.

Monoclonal antibodies directed toward an IL-17 like polypeptide are produced using any method which provides for the production of antibody molecules by continuous cell lines in culture. Examples of suitable methods for preparing monoclonal antibodies include the hybridoma methods of Kohler et al., Nature, 256:495-497 (1975) and the human B-cell hybridoma method, Kozbor, J. Immunol., 133:3001 (1984) and Brodeur et al., Monoclonal Antibody Production Techniques and Applications, pp. 51-63 (Marcel Dekker, Inc., New York, 1987). Also provided by the invention are hybridoma cell lines which produce monoclonal antibodies reactive with IL-17 like polypeptides.

Monoclonal antibodies disclosed herein may be modified for use as therapeutics. One embodiment is a "chimeric" antibody in which a portion of the heavy and/or light chain is identical with or homologous to a corresponding sequence in antibodies derived from a particular species or belonging to a particular antibody class or subclass, while the remainder of the chain(s) is/are identical with or homologous to a corresponding sequence in antibodies derived from another species or belonging to another antibody class or subclass. Also included are fragments of such antibodies, so long as they exhibit the desired biological activity. See, U.S. Patent No. 4,816,567 and Morrison et al., Proc. Natl. Acad. Sci. USA, 81:6851-6855 (1985).

In another embodiment, a monoclonal antibody of the invention is a "humanized" antibody. Methods for humanizing non-human antibodies are well known in the art (see U.S. Patent Nos. 5,585,089, and 5,693,762). Generally, a humanized antibody has one or more amino acid residues introduced into it from a source which is non-human. Humanization can be performed, for example, using methods described in the art (Jones et al., Nature 321:522-525 (1986); Riechmann et al., Nature, 332:323-327 (1988); Verhoeyen et al., Science, 239:1534-1536 (1988)), by substituting at least a portion of a rodent complementarity-determining region (CDR) for the corresponding regions of a human antibody.

Also encompassed by the present disclosure are human antibodies which bind IL-17 like polypeptides. Using transgenic animals (*e.g.*, mice) that are capable of producing a repertoire of human antibodies in the absence of endogenous immunoglobulin production, such antibodies are produced by immunization with an IL-17 like antigen *(i.e.,* having at least 6 contiguous amino acids), optionally conjugated to a carrier. See, for example, Jakobovits et al., Proc. Natl. Acad. Sci. USA, 90:2551-2555 (1993) ; Jakobovits et al., Nature, 362:255-258 (1993) and Bruggermann et al., Year in Immunol., 7:33 (1993). In one method, such transgenic animals are produced by incapacitating the endogenous loci encoding the heavy and light immunoglobulin chains therein, and inserting loci encoding human heavy and light chain proteins into the genome thereof. Partially modified animals, that is those having less than the full complement of modifications, are then cross-bred to obtain an animal having all of the desired immune system modifications. When administered an immunogen, these transgenic animals produce antibodies with human (rather than *e.g.*, murine) amino acid sequences, including variable regions which are immunospecific for these antigens. See PCT application nos. PCT/US96/05928 and PCT/US93/06926. Additional methods are described in U.S. Patent No. 5,545,807, PCT application nos. PCT/US91/245, PCT/GB89/01207, and in EP 546073B1 and EP 546073A1. Human antibodies may also be produced by the expression of recombinant DNA in host cells or by expression in hybridoma cells as described herein.

In an alternative embodiment, human antibodies can be produced from phage-display libraries (Hoogenboom et al., J. Mol. Biol., 227:381 (1991) and Marks et al., J. Mol. Biol., 222:581 (1991)). These processes mimic immune selection through the display of antibody repertoires on the surface of filamentous bacteriophage, and subsequent selection of phage by their binding to an antigen of choice. One such technique is described in PCT Application No. PCT/US98/17364, which describes the isolation of high affinity and functional agonistic antibodies for MPL-and msk-receptors using such an approach.

Chimeric, CDR grafted, and humanized antibodies are typically produced by recombinant methods. Nucleic acids encoding the antibodies are introduced into host cells and expressed using materials and procedures described herein. In a preferred embodiment, the antibodies are produced in mammalian host cells, such as CHO cells. Monoclonal (e.g., human) antibodies may be produced by the expression of recombinant DNA in host cells or by expression in hybridoma,cells as described herein.

The anti-IL-17 like antibodies of the disclosure may be employed in any known assay method, such as competitive binding assays, direct and indirect sandwich assays, and immunoprecipitation assays (Sola, Monoclonal Antibodies: A Manual of Techniques, pp. 147-158 (CRC Press, Inc., 1987)) for the detection and quantitation of IL-17 like polypeptides. The antibodies will bind IL-17 like polypeptides with an affinity which is appropriate for the assay method being employed.

For diagnostic applications, in certain embodiments, anti-IL-17 like antibodies may be labeled with a detectable moiety. The detectable moiety can be any one which is capable of producing, either directly or indirectly, a detectable signal. For example, the detectable moiety may be a radioisotope, such as $^{3}H$, $^{14}C$, $^{32}P$, $^{35}S$, or $^{125}I$; a fluorescent or chemiluminescent compound, such as fluorescein isothiocyanate, rhodamine, or luciferin; or an enzyme, such as alkaline phosphatase, β-galactosidase, or horseradish peroxidase (Bayer et al., Meth. Enz., 184:138-163 (1990)).

Competitive binding assays rely on the ability of a labeled standard (*e.g.*, an IL-17 like polypeptide, or an immunologically reactive portion thereof) to compete with the test sample analyte (an IL-17 like polypeptide) for binding with a limited amount of anti-IL-17 like antibody. The amount of an IL-17 like polypeptide in the test sample is inversely proportional to the amount of standard that becomes bound to the antibodies. To facilitate determining the amount of standard that becomes bound, the antibodies typically are insolubilized before or after the competition, so that the standard and analyte that are bound to the antibodies may conveniently be separated from the standard and analyte which remain unbound.

Sandwich assays typically involve the use of two antibodies, each capable of binding to a different immunogenic portion, or epitope, of the protein to be detected and/or quantitated. In a sandwich assay, the test sample analyte is typically bound by a first antibody which is immobilized on a solid support, and thereafter a second antibody binds to the analyte, thus forming an insoluble three-part complex. See, *e.g.*, U.S. Patent No. 4,376,110. The second antibody may itself be labeled with a detectable moiety (direct sandwich assays) or may be measured using an anti-immunoglobulin antibody that is labeled with a detectable moiety (indirect sandwich assays). For example, one type of sandwich assay is an enzyme-linked immunosorbent assay (ELISA), in which case the detectable moiety is an enzyme.

The selective binding agents, including anti-IL-17 like antibodies, are also useful for in *vivo* imaging. An antibody labeled with a detectable moiety may be administered to an animal, preferably into the bloodstream, and the presence and location of the labeled antibody in the host is assayed. The antibody may be labeled with any moiety that is detectable in an animal, whether by nuclear magnetic resonance, radiology, or other detection means known in the art.

Selective binding agents disclosed herein, including antibodies, may be used as therapeutics. These therapeutic agents are generally agonists or antagonists in that they either enhance or reduce, respectively, at least one of the biological activities of an IL-17 like polypeptide, including IL-17 like polypeptide proinflammatory activity. In one embodiment, antagonist antibodies used according to the invention are antibodies or binding fragments thereof which are capable of specifically binding to an IL-17 like polypeptide and which are capable of inhibiting or eliminating the functional activity of an IL-17 like polypeptide in vivo or in vitro. In preferred embodiments, the selective binding agent, *e.g.,* an antagonist antibody, will inhibit the functional activity of an IL-17 like polypeptide by at least about 50%, and preferably by at least about 80%. In another disclosure, the selective binding agent may be an antibody that is capable of interacting with an IL-17 like binding partner (a ligand or receptor) thereby inhibiting or eliminating IL-17 like activity in vitro or *in vivo.* Selective binding agents, including agonist and antagonist anti-IL-17 like antibodies, are identified by screening assays which are well known in the art.

Also disclosed is a kit comprising IL-17 like selective binding agents (such as antibodies) and other reagents useful for detecting IL-17 like polypeptide levels in biological samples. Such reagents may include a detectable label, blocking serum, positive and negative control samples, and detection reagents.

The IL-17 like polypeptides as disclosed herein can be used to clone IL-17 like receptors, using an expression cloning strategy. Radiolabeled ($^{125}$-Iodine) IL-17 like polypeptide or affinity/activity-tagged IL-17 like polypeptide (such as an Fc fusion or an alkaline phosphatase fusion) can be used in binding assays to identify a cell type or cell line or tissue that expresses IL-17 like receptor(s). RNA isolated from such cells or tissues can be converted to cDNA, cloned into a mammalian expression vector, and transfected into mammalian cells (such as COS or 293 cells) to create an expression library. A radiolabeled or tagged IL-17 like polypeptide can then be used as an affinity ligand to identify and isolate from this library the subset of cells which express the IL-17 like receptor(s) on their surface. DNA can then be isolated from these cells and transfected into mammalian cells to create a secondary expression library in which the fraction of cells expressing IL-17 like receptor(s) is many-fold higher than in the original library. This enrichment process can be repeated iteratively until a single recombinant clone containing an IL-17 like receptor is isolated. Isolation of the IL-17 like receptor(s) is useful for identifying or developing novel agonists and antagonists of the IL-17 like polypeptide signaling pathway. Such agonists and antagonists include soluble IL-17 like receptor(s), anti-IL-17 like receptor antibodies, small molecules, proteins, peptides, carbohydrates, lipids, or antisense oligonucleotides, and they may be used for treating, preventing, or diagnosing one or more disease or disorder, including those described herein.

## Assaying for Other Modulators of IL-17-Like Polypeptide Activity

In some situations, it may be desirable to identify molecules that are modulators, i.e., agonists or antagonists, of the activity of IL-17 like polypeptide. Natural or synthetic molecules that modulate IL-17 like polypeptide may be identified using one or more screening assays, such as those described herein. Such molecules may be administered either in an ex vivo manner, or in an in vivo manner by injection, or by oral delivery, implantation device or the like.

"Test molecule(s)" refers to the molecule(s) that is/are under evaluation for the ability to modulate (i.e., increase or decrease) the activity of an IL-17 like polypeptide. Most commonly, a test molecule will interact directly with an IL-17 like polypeptide. However, it is also contemplated that a test molecule may also modulate IL-17 like polypeptide activity indirectly, such as by affecting IL-17 like gene expression, or by binding to an IL-17 like binding partner (e.g., receptor or ligand). In one embodiment, a test molecule will bind to an IL-17 like polypeptide with an affinity constant of at least about $10^{-6}$ M, preferably about $10^{-8}$ M, more preferably about $10^{-9}$ M, and even more preferably about $10^{-10}$ M.

Methods for identifying compounds which interact with IL-17 like polypeptides are encompassed by the present invention. In certain embodiments, an IL-17 like polypeptide is incubated with a test molecule under conditions which permit the interaction of the test molecule with an IL-17 like polypeptide, and the extent of the interaction can be measured. The test molecule(s) can be screened in a substantially purified form or in a crude mixture.

In certain embodiments, an IL-17 like polypeptide agonist or antagonist may be a protein, peptide, carbohydrate, lipid or small molecular weight molecule which interacts with IL-17 like polypeptide to regulate its activity. Molecules which regulate IL-17 like polypeptide expression include nucleic acids which are complementary to nucleic acids encoding an IL-17 like polypeptide, or are complementary to nucleic acid sequences which direct or control the expression of IL-17 like polypeptide, and which act as anti-sense regulators of expression.

Once a set of test molecules has been identified as interacting with an IL-17 like polypeptide, the molecules may be further evaluated for their ability to increase or decrease IL-17 like polypeptide activity. The measurement of the interaction of test molecules with IL-17 like polypeptides may be carried out in several formats, including cell-based binding assays, membrane binding assays, solution-phase assays and immunoassays. In general, test molecules are incubated with an IL-17 like polypeptide for a specified period of time, and IL-17 like polypeptide activity is determined by one or more assays for measuring biological activity.

The interaction of test molecules with IL-17 like polypeptides may also be assayed directly using polyclonal or monoclonal antibodies in an immunoassay. Alternatively, modified forms of IL-17 like polypeptides containing epitope tags as described herein may be used in immunoassays.

In the event that IL-17 like polypeptides display biological activity through an interaction with a binding partner (e.g., a receptor or a ligand), a variety of in vitro assays may be used to measure the binding of an IL-17 like polypeptide to the corresponding binding partner (such as a selective binding agent, receptor or ligand). These assays may be used to screen test molecules for their ability to increase or decrease the rate and/or the extent of binding of an IL-17 like polypeptide to its binding partner. In one assay, an IL-17 like polypeptide is immobilized in the wells of a microtiter plate. Radiolabeled IL-17 like binding partner (for example, iodinated IL-17 like binding partner) and the test molecule(s) can then be added either one at a time (in either order) or simultaneously to the wells. After incubation, the wells can be washed and counted (using a scintillation counter) for radioactivity to determine the extent to which the binding partner bound to IL-17 like polypeptide. Typically, the molecules will be tested over a range of concentrations, and a series of control wells lacking one or more elements of the test assays can be used for accuracy in the evaluation of the results. An alternative to this method involves reversing the "positions" of the proteins, i.e., immobilizing IL-17 like binding partner to the microtiter plate wells, incubating with the test molecule and radiolabeled IL-17 like polypeptide, and determining the extent of IL-17 like polypeptide binding. See, for example, chapter 18, Current Protocols in Molecular Biology, Ausubel et al., eds., John Wiley & Sons, New York, NY (1995).

As an alternative to radiolabeling, an IL-17 like polypeptide or its binding partner may be conjugated to biotin and the presence of biotinylated protein can then be detected using streptavidin linked to an enzyme, such as horseradish peroxidase (HRP) or alkaline phosphatase (AP), that can be detected colorometrically or by fluorescent tagging of streptavidin. An antibody directed to an IL-17 like polypeptide or to an IL-17 like binding partner and conjugated to biotin may also be used and can be detected after incubation with enzyme-linked streptavidin linked to AP or HRP.

An IL-17 like polypeptide or an IL-17 like binding partner can also be immobilized by attachment to agarose beads, acrylic beads or other types of such inert solid phase substrates. The substrate-protein complex can be placed in a solution containing the complementary protein and the test compound. After incubation the beads can be precipitated by centrifugation, and the amount of binding between an IL-17 like polypeptide and its binding partner can be assessed using the methods described herein. Alternatively, the substrate-protein complex can be immobilized in a column, and the test molecule and complementary protein are passed through the column. The formation of a complex between an IL-17 like polypeptide and its binding partner can then be assessed using any of the techniques set forth herein, i.e., radiolabeling, antibody binding or the like.

Another *in vitro* assay that is useful for identifying a test molecule which increases or decreases the formation of a complex between an IL-17 like polypeptide and an IL-17 like binding partner is a surface plasmon resonance detector system such as the BIAcore assay system (Pharmacia, Piscataway, NJ). The BIAcore system may be carried out using the manufacturer's protocol. This assay essentially involves the covalent binding of either IL-17 like polypeptide or an IL-17 like binding partner to a dextran-coated sensor chip which is located in a detector. The test compound and the other complementary protein can then be injected, either simultaneously or sequentially, into the chamber containing the sensor chip. The amount of complementary protein that binds can be assessed based on the change in molecular mass which is physically associated with the dextran-coated side of the sensor chip; the change in molecular mass can be measured by the detector system.

In some cases, it may be desirable to evaluate two or more test compounds together for their ability to increase or decrease the formation of a complex between an IL-17 like polypeptide and an IL-17 like binding partner. In these cases, the assays set forth herein can be readily modified by adding such additional test compound(s) either simultaneous with, or subsequent to, the first test compound. The remainder of the steps in the assay are as set forth herein.

In vitro assays such as those described herein may be used advantageously to screen large numbers of compounds for effects on complex formation by an IL-17 like polypeptide and an IL-17 like binding partner. The assays may be automated to screen compounds generated in phage display, synthetic peptide and chemical synthesis libraries.

Compounds which increase or decrease the formation of a complex between an IL-17 like polypeptide and an IL-17 like binding partner may also be screened in cell culture using cells and cell lines expressing either IL-17 like polypeptide or IL-17 like binding partner. Cells and cell lines may be obtained from any mammal, but preferably will be from human or other primate, canine or rodent sources. The binding of an IL-17 like polypeptide to cells expressing IL-17 like binding partner at the surface is evaluated in the presence or absence of test molecules, and the extent of binding may be determined by, for example, flow cytometry using a biotinylated antibody to an IL-17 like binding partner. Cell culture assays can be used advantageously to further evaluate compounds that score positive in protein binding assays described herein.

Cell cultures can also be used to screen the impact of a drug candidate. For example, drug candidates may decrease or increase the expression of the IL-17 like gene. In certain embodiments, the amount of IL-17 like polypeptide that is produced may be measured after exposure of the cell culture to the drug candidate. In certain embodiments, one may detect the actual impact of the drug candidate on the cell culture. For example, the overexpression of a particular gene may have a particular impact on the cell culture. In such cases, one may test a drug candidate's ability to increase or decrease the expression of the gene or its ability to prevent or inhibit a particular impact on the cell culture. In other examples, the production of a particular metabolic product such as a fragment of a polypeptide may result in, or be associated with, a disease or pathological condition. In such cases, one may test a drug candidate's ability to decrease the production of such a metabolic product in a cell culture.

## P-38 Inhibitors

Where intervention between extracellular stimulus and the secretion of IL-1 and/or TNFα from a cell is desired, this can be achieved by blocking signal transduction through the inhibition of a kinase which lies on the signal pathway. This can be achieved for example through the inhibition of "P-38" (also called "RK" or "SAPK-2", Lee et al., Nature, 372: 739 (1994)), a known serine/threonine (ser/thr) kinase. See Han et al., Biochimica Biophysica Acta, 1265:224-227 (1995). A linear relationship has been shown for effectiveness in a competitive binding assay to P-38, and the same inhibitor diminishing levels of IL-1 secretion from monocytes following LPS stimulation. Following LPS stimulation of monocytes, the levels of messenger RNA for TNFα have been shown to increase 100 fold, but the protein levels of TNFα increased 10,000 fold. Thus, a considerable amplification of the TNF signaling occurs at the translational level. Inhibition of P-38 appears to diminish translational efficiency, and further evidence that TNFα is under translational control is found in the deletion experiments of Beutler et *al.* and Lee, wherein segments of 3' untranslated mRNA (3' UTR) are removed resulting in high translational efficiency for TNFα. Notably, P-38 inhibitors did not have an effect on the level of TNFα (i.e., translational efficiency) when the appropriate segments of TNFα mRNA were deleted.

It has been found that elevated levels of TNFα and/or IL-1 may contribute to the onset, etiology, or exacerbate a number of disease states, including, but not limited to: rheumatoid arthritis; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; antiviral therapy including those viruses sensitive to TNFα inhibition - HIV-1, HIV-2, HIV-3, cytomegalovirus (CMV), influenza, adenovirus, and the herpes viruses including HSV-1, HSV-2, and herpes zoster; muscle degeneration; cachexia; Reiter's syndrome; type II diabetes; bone resorption diseases; graft vs. host reaction; ischemia reperfusion injury; atherosclerosis; brain trauma; Alzheimer's disease; multiple sclerosis; cerebral malaria; sepsis; septic shock; toxic shock syndrome; fever and myalgias due to infection.

Substituted imidazole, pyrrole, pyridine, pyrimidine and the like compounds have been described for use in the treatment of cytokine mediated diseases by inhibition of proinflammatory cytokines, such as IL-1, IL-6, IL-8 and TNF. Substituted imidazoles for use in the treatment of cytokine mediated diseases have been described in U.S. Patent No. 5,593,992; WO 93/14081; WO 97/18626; WO 96/21452; WO 96/21654; WO 96/40143; WO 97/05878; WO 97/05878.

Substituted imidazoles for use in the treatment of inflammation has been described in US Pat. 3,929,807. Substituted pyrrole compounds for use in the treatment of cytokine mediated diseases have been described in WO 97/05877; WO 97/05878; WO 97/16426; WO 97/16441; and WO 97/16442. Substituted aryl and heteroaryl fused pyrrole compounds for use in the treatment of cytokine mediated diseases have been described in WO 98/22457. Substituted pyridine, pyrimidine, pyrimidinone, and pyridazine compounds for use in the treatment of cytokine mediated diseases have been described in WO 98/24780; WO 98/24782; WO 99/24404; and WO 99/32448.

## Internalizing Proteins

The tat protein sequence (from HIV) can be used to internalize proteins into a cell. See e.g., Falwell et al., Proc. Natl. Acad. Sci. USA, 91:664-668 (1994). For example, an 11 amino acid sequence (YGRKKRRQRRR; SEQ ID NO: 13) of the HIV tat protein (termed the "protein transduction domain", or TAT PDT) has been described as mediating delivery across the cytoplasmic membrane and the nuclear membrane of a cell. See Schwarze et al., Science, 285: 1569-1572 (1999); and Nagahara et al., Nature Medicine, 4:1449-1452 (1998). In these procedures, FITC-constructs (FITC-GGGGYGRKKRRQRRR; SEQ ID NO: 14) are prepared which bind to cells as observed by fluorescence-activated cell sorting (FACS) analysis, and these constructs penetrate tissues after i.p. adminstration. Next, tat-bgal fusion proteins are constructed. Cells treated with this construct demonstrate b-gal activity. Following injection, a number of tissues, including liver, kidney, lung, heart and brain tissue, have been found to demonstrate expression using these procedures. It is believed that these constructions underwent some degree of unfolding in order to enter the cell; as such, refolding may be required after entering the cell.

It will thus be appreciated that the tat protein sequence may be used to internalize a desired protein or polypeptide into a cell. For example, using the tat protein sequence, an IL-17 like antagonist (such as an anti-IL-17 like selective binding agent, small molecule, soluble receptor, or antisense oligonucleotide) can be administered intracellularly to inhibit the activity of an IL-17 like molecule. As used herein, the term "IL-17 like molecule" refers to both IL-17 like nucleic acid molecules and IL-17 like polypeptides as defined herein. Where desired, the IL-17 like protein itself may also be internally administered to a cell using these procedures. See also, Strauss, E., "Introducing Proteins Into the Body's Cells", Science, 285:1466-1467 (1999).

**Therapeutic Uses**

Expression of the human IL-17 like polypeptide has been found in the following types of cells: testis, prostate, mammary gland, lymph node, and femur. Expression of the mouse IL-17 like polypeptide has been found in the following types of cells: T cells, and embryo cells.

A non-exclusive list of acute and chronic diseases which can be treated, diagnosed, ameliorated, or prevented with the IL-17 like nucleic acids, polypeptides, and agonists and antagonists of the present disclosure include:

- The diagnosis and/or treatment of diseases involving immune system dysfunction. Examples of such diseases include, but are not limited to, rheumatoid arthritis, psioriatic arthritis, inflammatory arthritis, osteoarthritis, inflammatory joint disease, autoimmune disease including autoimmune vasculitis, multiple sclerosis, lupus, diabetes (e.g., insulin diabetes), inflammatory bowel disease, transplant rejection, graft vs. host disease, and inflammatory conditions resulting from strain, sprain, cartilage damage, trauma, orthopedic surgery, infection or other disease processes. Other diseases influenced by the dysfunction of the immune system are encompassed within the scope of the disclosure, including but not limited to, allergies. The IL-17 like nucleic acids, polypeptides, and agonists and antagonists of the invention can also be used to inhibit T cell proliferation, to inhibit T cell activation, and/or to inhibit B cell proliferation and/or immunoglobulin secretion.

- The diagnosis and/or treatment of diseases involving infection. Examples of such diseases include, but are not limited to, leprosy; viral infections such as hepatitis or HIV, bacterial infection such as *clostridium* associated illnesses, including *clostridium*-associated diarrhea, pulmonary tuberculosis, acute febrile illness from bacteria such as or virus, fever, acute phase response of the liver, septicemia, septic shock. Other diseases involving infection are encompassed within the scope of the disclosure.

- The diagnosis and/or treatment of diseases involving weight disorders. Examples of such diseases include, but are not limited to obesity, anorexia, cachexia, including AIDS-induced cachexia, myopathies (e.g., muscle protein metabolism, such as in sepsis), and hypoglycemia. Other diseases involving weight disorders are encompassed within the scope of the disclosure.

- The diagnosis and/or treatment of diseases involving neuronal dysfunction. Examples of such diseases include, but are not limited to Alzheimer's, Parkinson's disease, neurotoxicity (*e.g.*, as induced by HIV), ALS, brain injury, stress, depression, nociception and other pain (including cancer-related pain), hyperalgesia, epilepsy, learning impairment and memory disorders, sleep disturbance, and peripheral and central neuropathies. Other neurological disorders are encompassed within the scope of The disclosure.

- The diagnosis and/or treatment of diseases involving the lung. Examples of such diseases include, but are not limited to, acute or chronic lung injury including interstitial lung disease, acute respiratory disease syndrome, pulmonary hypertension, emphysema, cystic fibrosis, pulmonary fibrosis, and asthma. Other diseases of the lung are encompassed within the scope of the disclosure.

- The diagnosis and/or treatment of diseases involving the skin. Examples of such diseases include, but are not limited to, psoriasis, eczema, and wound healing. Other diseases of the skin are encompassed within the scope of the disclosure.

- The diagnosis and/or treatment of diseases involving the kidney. Examples of such diseases include, but are not limited to, acute and chronic glomerulonephritis. Other diseases of the kidney are encompassed within the scope of the disclosure.

- The diagnosis and/or treatment of diseases involving the bone. Examples of such diseases include, but are not limited to, osteoporosis, osteopetrosis, osteogenesis imperfecta, Paget's disease, periodontal disease, temporal mandibular joint disease, and hypercalcemia. Other diseases of the bone are encompassed within the scope of the disclosure.

- The diagnosis and/or treatment of diseases involving the vascular system. Examples of such diseases include, but are not limited to hemorrhage or stroke, hemorrhagic shock, ischemia, including cardiac ischemia and cerebral ischemia *(e.g.,* brain injury as a result of trauma, epilepsy, hemorrhage or stroke, each of which may lead to neurodegeneration), atherosclerosis, congestive heart failure; restenosis, reperfusion injury, and angiogenesis. Other diseases of the vascular system are encompassed within the scope of the disclosure.

- The diagnosis and/or treatment of tumor cells. Examples of such diseases include, but are not limited to, lymphomas, bone sarcoma, chronic and acute myelogenous leukemia (AML and CML) including myelomonocytic leukemis (M4 AML), and other leukemias, multiple myeloma, lung, breast cancer, tumor metastasis, and side effects from radiation therapy. Other diseases involving tumor cells are encompassed within the scope of the disclosure.

- The diagnosis and/or treatment of reproductive disorders. Examples of such diseases include, but are not limited to, infertility, miscarriage, pre-term labor and delivery, and endometriosis. Other diseases involving the reproductive system are encompassed within the scope of the disclosure.

- The diagnosis and/or treatment of eye disorders. Examples of such diseases include, but are not limited to, inflammatory eye disease, as may be associated with, for example, corneal transplant; retinal degeneration, blindness, macular degeneration, glaucoma, uveitis, and retinal neuropathy. Other diseases of the eye are encompassed within the scope of the disclosure.

- The diagnosis and/or treatment of diseases involving inflammation. Examples of such diseases include but are not limited to those described herein.

The invention provides a use of an antagonistic antibody specific for a polypeptide consisting of the amino acid sequence of SEQ ID NO: 2, or a fragment thereof comprising 25, 50, 75, 100 or 150 contiguous amino acids, for preparation of a medicament for treating cystic fibrosis, acute respiratory disease syndrome or emphysema.

The invention further provides an antagonistic antibody specific for a polypeptide consisting of the amino acid sequence of SEQ ID NO: 2, or a fragment thereof comprising 25, 50, 75, 100 or 150 contiguous amino acids, for use in the treatment of cystic fibrosis, acute respiratory disease syndrome or emphysema.

The invention further provides a use of a pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for preparation of a medicament for treating cystic fibrosis, acute respiratory disease syndrome or emphysema.

The invention further provides a pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for use in the treatment of cystic fibrosis, acute respiratory disease syndrome or emphysema.

It has also been found that the present IL-17 like nucleic acids, polypeptides, and agonists disclosed herein can increase bone marrow and spleen cellularity, eosinophils, colony forming cells (CFCs), and lymphocyte production. The present IL-17 like nucleic acids and polypeptides thus modulate hematopoietic cell growth, including the stimulation of proliferation and/or differentiation of at least 1 early or multipotent progenitor committed to at least 1 granulocyte and/or megakaryocyte lineage. Conversely, IL-17 like antagonists are capable of decreasing levels and/or production of these cells.

In addition, the IL-17 like nucleic acids, polypeptides, and agonists disclosed herein have proinflammatory activity. The IL-17 like polypeptides induce production of proinflammatory cytokines such as TNF-$\alpha$, IL-1$\alpha$, IL-1$\beta$ and IL-6.

Additionally, the IL-17 like nucleic acids, polypeptides, and agonists and antagonists disclosed herein can be used to stimulate hematopoiesis and production of neutrophils, granulocytes, or platelets, and are thus useful for patients undergoing chemotherapy. The IL-17 like nucleic acids, polypeptides, and agonists and antagonists disclosed herein may also be used to treat viral or bacterial infections, immune related diseases, anemia, leukemia, thrombocytopenia, uremia, Von Willebrand's disease, postoperative cardiovascular dysfunction, treatment of AIDS (acquired immune deficiency syndrome)-related bone marrow failure, and inflammatory diseases of the gastrointestinal system, joints, and lungs.

Other diseases which are treatable using agents within the scope of the present disclosure include acute pancreatitis, chronic fatigue syndrome, fibromyalgia, and Kawasaki's disease (MLNS).

Other diseases associated with undesirable levels of one or more of IL-1, IL-lra, the ligand of the present IL-17 like polypeptide, and/or the present IL-17 like polypeptide itself are encompassed within the scope of the present disclosure. Undesirable levels include excessive and/or sub-normal levels of IL-1, IL-lra, the receptor(s) of the present IL-17 like polypeptide, and/or the IL-17 like polypeptides described herein.

IL-1 inhibitors include any protein capable of specifically preventing activation of cellular receptors to IL-1, which may result from any number of mechanisms. Such mechanisms include downregulating IL-1 production, binding free IL-1, interfering with IL-1 binding to its receptor, interfering with formation of the IL-1 receptor complex (i.e., association of IL-1 receptor with IL-1 receptor accessory protein), or interfering with modulation of IL-1 signaling after binding to its receptor. Classes of interleukin-1 inhibitors include:

- Interleukin-1 receptor antagonists such as IL-1ra, as described herein;
- Anti-IL-1 receptor monoclonal antibodies (e.g., EP 623674);
- IL-1 binding proteins such as soluble IL-1 receptors *(e.g.,* U.S. Pat. No. 5,492,888, U.S. Pat. No. 5,488,032, and U.S. Pat. No. 5,464,937, U.S. Pat. No. 5,319,071, and U.S. Pat. No. 5,180,812;
- Anti-IL-1 monoclonal antibodies (*e.g.*, WO 9501997, WO 9402627, WO 9006371, U.S. Pat. No. 4,935,343, EP 364778, EP 267611 and EP 220063;
- IL-1 receptor accessory proteins and antibodies thereto (*e.g.*, WO 96/23067);
- Inhibitors of interleukin-1β converting enzyme (ICE) or caspase I, which can be used to inhibit IL-1 beta production and secretion;
- Interleukin-1β protease inhibitors;
- Other compounds and proteins which block *in vivo* synthesis or extracellular release of IL-1.

Exemplary IL-1 inhibitors are disclosed in the following references:

US Pat. Nos. 5747444; 5359032; 5608035; 5843905; 5359032; 5866576; 5869660; 5869315; 5872095; 5955480;

International (WO) patent applications 98/21957, 96/09323, 91/17184, 96/40907, 98/32733, 98/42325, 98/44940, 98/47892, 98/56377, 99/03837, 99/06426, 99/06042, 91/17249, 98/32733, 98/17661, 97/08174, 95/34326, 99/36426, and 99/36415;

European (EP) patent applications 534978 and 894795; and French patent application FR 2762514;

Interleukin-1 receptor antagonist (IL-1ra) is a human protein that acts as a natural inhibitor of interleukin-1. Preferred receptor antagonists (including IL-1ra and variants and derivatives thereof), as well as methods of making and using thereof, are described in U.S. Patent No. 5,075,222; WO 91/08285; WO 91/17184; AU 9173636; WO 92/16221; WO93/21946; WO 94/06457; WO 94/21275; FR 2706772; WO 94/21235; DE 4219626, WO 94/20517; WO 96/22793; WO 97/28828; and WO 99/36541. The proteins include glycosylated as well as non-glycosylated IL-1 receptor antagonists.

Specifically, three exemplary forms of IL-1ra and variants thereof are disclosed and described in the 5,075,222 patent. The first of these, called "IL-1i" in the 5,075,222 patent, is characterized as a 22-23 kD molecule on SDS-PAGE with an approximate isoelectric point of 4.8, eluting from a MonoQ FPLC column at around 52 mM NaCl in Tris buffer, pH 7.6. The second, IL-1raβ, is characterized as a 22-23 kD protein, eluting from a MonoQ column at 48 mM NaCl. Both IL-1raα and IL-1raβ are glycosylated. The third, IL-1rax, is characterized as a 20 kD protein, eluting from a MonoQ column at 48 mM NaCl, and is non-glycosylated. U.S. patent No. 5,075,222 also discloses methods for isolating the genes responsible for coding the inhibitors, cloning the gene in suitable vectors and cell types, and expressing the gene to produce the inhibitors.

Those skilled in the art will recognize that many combinations of deletions, insertions, and substitutions (individually or collectively "variant(s)" herein) can be made within the amino acid sequences of IL-lra, provided that the resulting molecule is biologically active (e.g., possesses the ability to affect one or more of the diseases and disorders such as those recited herein.)

As contemplated by the present invention, an agonist or antagonist of the IL-17 like polypeptide (including, but not limited to, anti-IL-17 like selective binding agents [such as antibodies], IL-17 like polypeptide receptors [such as soluble IL-17-like receptors], small molecules, and antisense oligonucleotides may be administered as an adjunct to other therapy and also with other pharmaceutical compositions suitable for the indication being treated. An agonist or antagonist of the IL-17 like polypeptide, and/or an IL-17 like receptor itself, and any of one or more additional therapies or pharmaceutical formulations may be administered separately, sequentially, or simultaneously.

In a specific embodiment, the present disclosure is directed to the use of an agonist or antagonist of the IL-17 like polypeptide, and/or an IL-17 like receptor in combination (pre-treatment, post-treatment, or concurrent treatment) with any of one or more TNF inhibitors for the treatment or prevention of the diseases and disorders recited herein.

Such TNF inhibitors include compounds and proteins which block *in vivo* synthesis or extracellular release of TNF. In a specific embodiment, the present disclosure is directed to the use of an agonist or antagonist of the IL-17 like polypeptide, and/or an IL-17 like receptor in combination (pre-treatment, post-treatment, or concurrent treatment) with any of one or more of the following TNF inhibitors: TNF binding proteins (soluble TNF receptor type-I and soluble TNF receptor type-II ("sTNFRs"), as defined herein), anti-TNF antibodies, granulocyte colony stimulating factor; thalidomide; BN 50730; tenidap; E 5531; tiapafant PCA 4248; nimesulide; panavir; rolipram; RP 73401; peptide T; MDL 201, 449A; (1R,3S)-Cis-1-[9-(2,6-diaminopurinyl)]-3-hydroxy-4-cyclopentene hydrochloride; (1R,3R)-trans-1-(9-(2,6-diamino)purine]-3-acetoxycyclopentane; (1R,3R)-trans-1-[9-adenyl)-3-azidocyclopentane hydrochloride and (1R,3R)-trans-1-(6-hydroxy-purin-9-yl)-3-azidocyclo-pentane. TNF binding proteins are disclosed in the art (EP 308 378, EP 422 339, GB 2 218 101, EP 393 438, WO 90/13575, EP 398 327, EP 412 486, WO 91/03553, EP 418 014, JP 127,800/1991, EP 433 900, U.S. Patent No. 5,136,021, GB 2 246 569, EP 464 533, WO 92/01002, WO 92/13095, WO 92/16221, EP 512 528, EP 526 905, WO 93/07863, EP 568 928, WO 93/21946, WO 93/19777, EP 417 563, WO94/06476, and PCT International Application No. PCT/US97/12244).

For example, EP 393 438 and EP 422 339 teach the amino acid and nucleic acid sequences of a soluble TNF receptor type I (also known as "sTNFR-I" or "30kDa TNF inhibitor") and a soluble TNF receptor type II (also known as "sTNFR-II" or "40kDa TNF inhibitor"), collectively termed "sTNFRs", as well as modified forms thereof *(e.g.,* fragments, functional derivatives and variants). EP 393 438 and EP 422 339 also disclose methods for isolating the genes responsible for coding the inhibitors, cloning the gene in suitable vectors and cell types and expressing the gene to produce the inhibitors. Additionally, polyvalent forms *(i.e.,* molecules comprising more than one active moiety) of sTNFR-I and sTNFR-II have also been disclosed. In one embodiment, the polyvalent form may be constructed by chemically coupling at least one TNF inhibitor and another moiety with any clinically acceptable linker, for example polyethylene glycol (WO 92/16221 and WO 95/34326), by a peptide linker (Neve et al. (1996), Cytokine, 8(5) :365-370, by chemically coupling to biotin and then binding to avidin (WO 91/03553) and, finally, by combining chimeric antibody molecules (U.S. Patent 5,116,964, WO 89/09622, WO 91/16437 and EP 315062.

Anti-TNF antibodies include MAK 195F Fab antibody (Holler et al. (1993), 1st International Symposium on Cytokines in Bone Marrow Transplantation, 147); CDP 571 anti-TNF monoclonal antibody (Rankin et al. (1995), British Journal of Rheumatology, 34:334-342); BAY X 1351 murine anti-tumor necrosis factor monoclonal antibody (Kieft et al. (1995), 7th European Congress of Clinical Microbiology and Infectious Diseases, page 9); CenTNF cA2 anti-TNF monoclonal antibody (Elliott et al. (1994), Lancet, 344:1125-1127 and Elliott et al. (1994), Lancet, 344:1105-1110).

In a specific embodiment, the present disclosure is directed to the use of agonist or antagonist of the IL-17 like polypeptide, and/or an IL-17 like receptor in combination (pretreatment, post-treatment, or concurrent treatment) with secreted or soluble human fas antigen or recombinant versions thereof (WO 96/20206 and Mountz et al., J. Immunology, 155:4829-4837; and EP 510 691. WO 96/20206 discloses secreted human fas antigen (native and recombinant, including an Ig fusion protein), methods for isolating the genes responsible for coding the soluble recombinant human fas antigen, methods for cloning the gene in suitable vectors and cell types, and methods for expressing the gene to produce the inhibitors. EP 510 691 teaches DNAs coding for human fas antigen, including soluble fas antigen, vectors expressing for said DNAs and transformants transfected with the vector. When administered parenterally, doses of a secreted or soluble fas antigen fusion protein each are generally from about 1 micrograms/kg to about 100 micrograms/kg.

Current treatment of the diseases and disorders recited herein, including acute and chronic inflammation such as rheumatic diseases, commonly includes the use of first line drugs for control of pain and inflammation; these drugs are classified as non-steroidal, anti-inflammatory drugs (NSAIDs). Secondary treatments include corticosteroids, slow acting antirheumatic drugs (SAARDs), or disease modifying (DM) drugs. Information regarding the following compounds can be found in The Merck Manual of Diagnosis and Therapy, Sixteenth Edition, Merck, Sharp & Dohme Research Laboratories, Merck & Co., Rahway, NJ (1992) and in Pharmaprojects, PJB Publications Ltd.

In a specific embodiment, the present disclosure is directed to the use of an agonist or antagonist of the IL-17 like polypeptide, and/or an IL-17 like receptor and any of one or more NSAIDs for the treatment of the diseases and disorders recited herein, including acute and chronic inflammation such as rheumatic diseases; and graft versus host disease. NSAIDs owe their anti-inflammatory action, at least in part, to the inhibition of prostaglandin synthesis (Goodman and Gilman in "The Pharmacological Basis of Therapeutics," MacMillan 7th Edition (1985)). NSAIDs can be characterized into at least nine groups: (1) salicylic acid derivatives; (2) propionic acid derivatives; (3) acetic acid derivatives; (4) fenamic acid derivatives; (5) carboxylic acid derivatives; (6) butyric acid derivatives; (7) oxicams; (8) pyrazoles and (9) pyrazolones.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more salicylic acid derivatives, prodrug esters or pharmaceutically acceptable salts thereof. Such salicylic acid derivatives, prodrug esters and pharmaceutically acceptable salts thereof comprise: acetaminosalol, aloxiprin, aspirin, benorylate, bromosaligenin, calcium acetylsalicylate, choline magnesium trisalicylate, magnesium salicylate, choline salicylate, diflusinal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide O-acetic acid, salsalate, sodium salicylate and sulfasalazine. Structurally related salicylic acid derivatives having similar analgesic and anti-inflammatory properties are also intended to be encompassed by this group.

In an additional specific embodiment, the present disclosure is directed to the use of an agonist or antagonist of the IL-17 like polypeptide, and/or an IL-17 like receptor in combination (pretreatment, post-treatment, or concurrent treatment) with any of one or more propionic acid derivatives, prodrug esters or pharmaceutically acceptable salts thereof. The propionic acid derivatives, prodrug esters, and pharmaceutically acceptable salts thereof comprise: alminoprofen, benoxaprofen, bucloxic acid, carprofen, dexindoprofen, fenoprofen, flunoxaprofen, fluprofen, flurbiprofen, furcloprofen, ibuprofen, ibuprofen aluminum, ibuproxam, indoprofen, isoprofen, ketoprofen, loxoprofen, miroprofen, naproxen, naproxen sodium, oxaprozin, piketoprofen, pimeprofen, pirprofen, pranoprofen, protizinic acid, pyridoxiprofen, suprofen, tiaprofenic acid and tioxaprofen. Structurally related propionic acid derivatives having similar analgesic and anti-inflammatory properties are also intended to be encompassed by this group.

In yet another specific embodiment, the present disclosure is directed to the use of an agonist or antagonist of the IL-17 like polypeptide, and/or an IL-17 like receptor in combination (pretreatment, post-treatment, or concurrent treatment) with any of one or more acetic acid derivatives, prodrug esters or pharmaceutically acceptable salts thereof. The acetic acid derivatives, prodrug esters, and pharmaceutically acceptable salts thereof comprise: acemetacin, alclofenac, amfenac, bufexamac, cinmetacin, clopirac, delmetacin, diclofenac potassium, diclofenac sodium, etodolac, felbinac, fenclofenac, fenclorac, fenclozic acid, fentiazac, furofenac, glucametacin, ibufenac, indomethacin, isofezolac, isoxepac, lonazolac, metiazinic acid, oxametacin, oxpinac, pimetacin, proglumetacin, sulindac, talmetacin, tiaramide, tiopinac, tolmetin, tolmetin sodium, zidometacin and zomepirac. Structurally related acetic acid derivatives having similar analgesic and anti-inflammatory properties are also intended to be encompassed by this group.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more fenamic acid derivatives, prodrug esters or pharmaceutically acceptable salts thereof. The fenamic acid derivatives, prodrug esters and pharmaceutically acceptable salts thereof comprise: enfenamic acid, etofenamate, flufenamic acid, isonixin, meclofenamic acid, meclofenamate sodium, medofenamic acid, mefenamic acid, niflumic acid, talniflumate, terofenamate, tolfenamic acid and ufenamate. Structurally related fenamic acid derivatives having similar analgesic and anti-inflammatory properties are also intended to be encompassed by this group.

In an additional specific embodiment, the present disclosure is directed to the use of an agonist or antagonist of the IL-17 like polypeptide, and/or an IL-17 like receptor in combination (pretreatment, post-treatment, or concurrent treatment) with any of one or more carboxylic acid derivatives, prodrug esters or pharmaceutically acceptable salts thereof. The carboxylic acid derivatives, prodrug esters, and pharmaceutically acceptable salts thereof which can be used comprise: clidanac, diflunisal, flufenisal, inoridine, ketorolac and tinoridine. Structurally related carboxylic acid derivatives having similar analgesic and anti-inflammatory properties are also intended to be encompassed by this group.

In yet another specific embodiment, the present disclosure is directed to the use of an agonist or antagonist of the IL-17 like polypeptide, and/or an IL-17 like receptor in combination (pretreatment, post-treatment, or concurrent treatment) with any of one or more butyric acid derivatives, prodrug esters or pharmaceutically acceptable salts thereof. The butyric acid derivatives, prodrug esters, and pharmaceutically acceptable salts thereof comprise: bumadizon, butibufen, fenbufen and xenbucin. Structurally related butyric acid derivatives having similar analgesic and anti-inflammatory properties are also intended to be encompassed by this group.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more oxicams, prodrug esters, or pharmaceutically acceptable salts thereof. The oxicams, prodrug esters, and pharmaceutically acceptable salts thereof comprise: droxicam, enolicam, isoxicam, piroxicam, sudoxicam, tenoxicam and 4-hydroxyl-1,2-benzothiazine-1,1-dioxide-4-(N-phenyl)-carboxamide. Structurally related oxicams having similar analgesic and anti-inflammatory properties are also intended to be encompassed by this group.

In still another specific embodiment, the present disclosure is directed to the use of an agonist or antagonist of the IL-17 like polypeptide, and/or an IL-17 like receptor in combination (pretreatment, post-treatment, or concurrent treatment) with any of one or more pyrazoles, prodrug esters, or pharmaceutically acceptable salts thereof. The pyrazoles, prodrug esters, and pharmaceutically acceptable salts thereof which may be used comprise: difenamizole and epirizole. Structurally related pyrazoles having similar analgesic and anti-inflammatory properties are also intended to be encompassed by this group.

In an additional specific embodiment, the present disclosure is directed to the use of an agonist or antagonist of the IL-17 like polypeptide, and/or an IL-17 like receptor in combination (pretreatment, post-treatment or, concurrent treatment) with any of one or more pyrazolones, prodrug esters, or pharmaceutically acceptable salts thereof. The pyrazolones, prodrug esters and pharmaceutically acceptable salts thereof which may be used comprise: apazone, azapropazone, benzpiperylon, feprazone, mofebutazone, morazone, oxyphenbutazone, phenylbutazone, pipebuzone, propylphenazone, ramifenazone, suxibuzone and thiazolinobutazone. Structurally related pyrazalones having similar analgesic and anti-inflammatory properties are also intended to be encompassed by this group.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more of the following NSAIDs: ε-acetamidocaproic acid, S-adenosylmethionine, 3-amino-4-hydroxybutyric acid, amixetrine, anitrazafen, antrafenine, bendazac, bendazac lysinate, benzydamine, beprozin, broperamole, bucolome, bufezolac, ciproquazone, cloximate, dazidamine, deboxamet, detomidine, difenpiramide, difenpyramide, difisalamine, ditazol, emorfazone, fanetizole mesylate, fenflumizole, floctafenine, flumizole, flunixin, fluproquazone, fopirtoline, fosfosal, guaimesal, guaiazolene, isonixirn, lefetamine HCl, leflunomide, lofemizole, lotifazole, lysin clonixinate, meseclazone, nabumetone, nictindole, nimesulide, orgotein, orpanoxin, oxaceprol, oxapadol, paranyline, perisoxal, perisoxal citrate, pifoxime, piproxen, pirazolac, pirfenidone, proquazone, proxazole, thielavin B, tiflamizole, timegadine, tolectin, tolpadol, tryptamid and those designated by company code number such as 480156S, AA861, AD1590, AFP802, AFP860, AI77B, AP504, AU8001, BPPC, BW540C, CHINOIN 127, CN100, EB382, EL508, F1044, FK-506, GV3658, ITF182, KCNTEI6090, KME4, LA2851, MR714, MR897, MY309, ONO3144, PR823, PV102, PV108, R830, RS2131, SCR152, SH440, SIR133, SPAS510, SQ27239, ST281, SY6001, TA60, TAI-901 (4-benzoyl-1-indancarboxylic acid), TVX2706, U60257, UR2301 and WY41770. Structurally related NSAIDs having similar analgesic and anti-inflammatory properties to the NSAIDs are also intended to be encompassed by this group.

In still another specific embodiment, the present disclosure is directed to the use of an agonist or antagonist of the IL-17 like polypeptide, and/or an IL-17 like receptor in combination (pretreatment, post-treatment or concurrent treatment) with any of one or more corticosteroids, prodrug esters or pharmaceutically acceptable salts thereof for the treatment of the diseases and disorders recited herein, including acute and chronic inflammation such as rheumatic diseases, graft versus host disease and multiple sclerosis. Corticosteroids, prodrug esters and pharmaceutically acceptable salts thereof include hydrocortisone and compounds which are derived from hydrocortisone, such as 21-acetoxypregnenolone, alclomerasone, algestone, amcinonide, beclomethasone, betamethasone, betamethasone valerate, budesonide, chloroprednisone, clobetasol, clobetasol propionate, clobetasone, clobetasone butyrate, clocortolone, cloprednol, corticosterone, cortisone, cortivazol, deflazacon, desonide, desoximerasone, dexamethasone, diflorasone, diflucortolone, difluprednate, enoxolone, fluazacort, flucloronide, flumethasone, flumethasone pivalate, flucinolone acetonide, flunisolide, fluocinonide, fluorocinolone acetonide, fluocortin butyl, fluocortolone, fluocortolone hexanoate, diflucortolone valerate, fluorometholone, fluperolone acetate, fluprednidene acetate, fluprednisolone, flurandenolide, formocortal, halcinonide, halometasone, halopredone acetate, hydrocortamate, hydrocortisone, hydrocortisone acetate, hydrocortisone butyrate, hydrocortisone phosphate, hydrocortisone 21-sodium succinate, hydrocortisone tebutate, mazipredone, medrysone, meprednisone, methylprednisolone, mometasone furoate, paramethasone, prednicarbate, prednisolone, prednisolone 21-diedryaminoacetate, prednisolone sodium phosphate, prednisolone sodium succinate, prednisolone sodium 21-m-sulfobenzoate, prednisolone sodium 21-stearoglycolate, prednisolone tebutate, prednisolone 21-trimethylacetate, prednisone, prednival, prednylidene, prednylidene 21-diethylaminoacetate, tixocortol, triamcinolone, triamcinolone acetonide, triamcinolone benetonide and triamcinolone hexacetonide. Structurally related corticosteroids having similar analgesic and anti-inflammatory properties are also intended to be encompassed by this group.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more slow-acting antirheumatic drugs (SAARDs) or disease modifying antirheumatic drugs (DMARDS), prodrug esters, or pharmaceutically acceptable salts thereof for the treatment of the diseases and disorders recited herein, including acute and chronic inflammation such as rheumatic diseases, graft versus host disease and multiple sclerosis. SAARDs or DMARDS, prodrug esters and pharmaceutically acceptable salts thereof comprise: allocupreide sodium, auranofin, aurothioglucose, aurothioglycanide, azathioprine, brequinar sodium, bucillamine, calcium 3-aurothio-2-propanol-1-sulfonate, chlorambucil, chloroquine, clobuzarit, cuproxoline, cyclophosphamide, cyclosporin, dapsone, 15-deoxyspergualin, diacerein, glucosamine, gold salts *(e.g.,* cycloquine gold salt, gold sodium thiomalate, gold sodium thiosulfate), hydroxychloroquine, hydroxychloroquine sulfate, hydroxyurea, kebuzone, levamisole, lobenzarit, melittin, 6-mercaptopurine, methotrexate, mizoribine, mycophenolate mofetil, myoral, nitrogen mustard, D-penicillamine, pyridinol imidazoles such as SKNF86002 and SB203580, rapamycin, thiols, thymopoietin and vincristine. Structurally related SAARDs or DMARDs having similar analgesic and anti-inflammatory properties are also intended to be encompassed by this group.

In another specific embodiment, the present disclosure is directed to the use of an agonist or antagonist of the IL-17 like polypeptide, and/or an IL-17 like receptor in combination (pretreatment, post-treatment, or concurrent treatment) with any of one or more COX2 inhibitors, prodrug esters or pharmaceutically acceptable salts thereof for the treatment of the diseases and disorders recited herein, including acute and chronic inflammation. Examples of COX2 inhibitors, prodrug esters or pharmaceutically acceptable salts thereof include, for example, celecoxib. Structurally related COX2 inhibitors having similar analgesic and anti-inflammatory properties are also intended to be encompassed by this group.

In still another specific embodiment, the present disclosure is directed to the use of an agonist or antagonist of the IL-17 like polypeptide, and/or an IL-17 like receptor in combination (pretreatment, post-treatment, or concurrent treatment) with any of one or more antimicrobials, prodrug esters or pharmaceutically acceptable salts thereof for the treatment of the diseases and disorders recited herein, including acute and chronic inflammation. Antimicrobials include, for example, the broad classes of penicillins, cephalosporins and other beta-lactams, aminoglycosides, azoles, quinolones, macrolides, rifamycins, tetracyclines, sulfonamides, lincosamides and polymyxins. The penicillins include, but are not limited to penicillin G, penicillin V, methicillin, nafcillin, oxacillin, cloxacillin, dicloxacillin, floxacillin, ampicillin, ampicillin/sulbactam, amoxicillin, amoxicillin/clavulanate, hetacillin, cyclacillin, bacampicillin, carbenicillin, carbenicillin indanyl, ticarcillin, ticarcillin/clavulanate, azlocillin, mezlocillin, peperacillin, and mecillinam. The cephalosporins and other beta-lactams include, but are not limited to cephalothin, cephapirin, cephalexin, cephradine, cefazolin, cefadroxil, cefaclor, cefamandole, cefotetan, cefoxitin, ceruroxime, cefonicid, ceforadine, cefixime, cefotaxime, moxalactam, ceftizoxime, cetriaxone, cephoperazone, ceftazidime, imipenem and aztreonam. The aminoglycosides include, but are not limited to streptomycin, gentamicin, tobramycin, amikacin, netilmicin, kanamycin and neomycin. The azoles include, but are not limited to fluconazole. The quinolones include, but are not limited to nalidixic acid, norfloxacin, enoxacin, ciprofloxacin, ofloxacin, sparfloxacin and temafloxacin. The macrolides include, but are not limited to erythomycin, spiramycin and azithromycin. The rifamycins include, but are not limited to rifampin. The tetracyclines include, but are not limited to spicycline, chlortetracycline, clomocycline, demeclocycline, deoxycycline, guamecycline, lymecycline, meclocycline, methacycline, minocycline, oxytetracycline, penimepicycline, pipacycline, rolitetracycline, sancycline, senociclin and tetracycline. The sulfonamides include, but are not limited to sulfanilamide, sulfamethoxazole, sulfacetamide, sulfadiazine, sulfisoxazole and cotrimoxazole (trimethoprim/sulfamethoxazole). The lincosamides include, but are not limited to clindamycin and lincomycin. The polymyxins (polypeptides) include, but are not limited to polymyxin B and colistin.

## IL-17 like Compositions and Administration

Therapeutic compositions are within the scope of the present disclosure. Such IL-17 like pharmaceutical compositions may comprise a therapeutically effective amount of an IL-17 like polypeptide or an IL-17 like nucleic acid molecule in admixture with a pharmaceutically or physiologically acceptable formulation agent selected for suitability with the mode of administration. Pharmaceutical compositions may comprise a therapeutically effective amount of one or more IL-17 like selective binding agents in admixture with a pharmaceutically or physiologically acceptable formulation agent selected for suitability with the mode of administration.

Acceptable formulation materials preferably are nontoxic to recipients at the dosages and concentrations employed.

The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine); antimicrobials; antioxidants (such as ascorbic acid, sodium sulfite or sodium hydrogensulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. *(*Remington's Pharmaceutical Sciences, 18th Edition, A.R. Gennaro, ed., Mack Publishing Company (1990).

The optimal pharmaceutical composition will be determined by one skilled in the art depending upon, for example, the intended route of administration, delivery format and desired dosage. See, for example, *Remington's Pharmaceutical Sciences, supra.* Such compositions may influence the physical state, stability, rate of *in vivo* release and rate of *in vivo* clearance of the IL-17 like molecule.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute therefor. In one embodiment of the present invention, IL-17 like polypeptide compositions may be prepared for storage by mixing the selected composition having the desired degree of purity with optional formulation agents *(Remington's Pharmaceutical Sciences, supra)* in the form of a lyophilized cake or an aqueous solution. Further, the IL-17 like polypeptide product may be formulated as a lyophilizate using appropriate excipients such as sucrose.

The IL-17 like pharmaceutical compositions can be selected for parenteral delivery. Alternatively, the compositions may be selected for inhalation or for delivery through the digestive tract, such as orally. The preparation of such pharmaceutically acceptable compositions is within the skill of the art.

The formulation components are present in concentrations that are acceptable to the site of administration. For example, buffers are used to maintain the composition at physiological pH or at a slightly lower pH, typically within a pH range of from about 5 to about 8.

When parenteral administration is contemplated, the therapeutic compositions for use in this invention may be in the form of a pyrogen-free, parenterally acceptable aqueous solution comprising the desired IL-17 like molecule in a pharmaceutically acceptable vehicle. A particularly suitable vehicle for parenteral injection is sterile distilled water in which an IL-17 like molecule is formulated as a sterile, isotonic solution, properly preserved. Yet another preparation can involve the formulation of the desired molecule with an agent, such as injectable microspheres, bio-erodible particles, polymeric compounds (such as polylactic acid or polyglycolic acid), beads or liposomes, that provides for the controlled or sustained release of the product which may then be delivered via a depot injection. Hyaluronic acid may also be used, and this may have the effect of promoting sustained duration in the circulation. Other suitable means for the introduction of the desired molecule include implantable drug delivery devices.

In one embodiment, a pharmaceutical composition may be formulated for inhalation. For example, an IL-17 like molecule may be formulated as a dry powder for inhalation. IL-17 like polypeptide or IL-17 like nucleic acid molecule inhalation solutions may also be formulated with a propellant for aerosol delivery. In yet another embodiment, solutions may be nebulized. Pulmonary administration is further described in PCT application no. PCT/US94/001875, which describes pulmonary delivery of chemically modified proteins.

It is also contemplated that certain formulations may be administered orally. In one embodiment of the present invention, IL-17 like molecules which are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the IL-17 like molecule. Diluents, flavorings, low melting point waxes, vegetable oils, lubricants, suspending agents, tablet disintegrating agents, and binders may also be employed.

Another pharmaceutical composition may involve an effective quantity of IL-17 like molecules in a mixture with non-toxic excipients which are suitable for the manufacture of tablets. By dissolving the tablets in sterile water, or another appropriate vehicle, solutions can be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional IL-17 like pharmaceutical compositions will be evident to those skilled in the art, including formulations involving IL-17 like polypeptides in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, PCT Application No. PCT/US93/00829 which describes the controlled release of porous polymeric microparticles for the delivery of pharmaceutical compositions. Additional examples of sustained-release preparations include semipermeable polymer matrices in the form of shaped articles, *e.g.* films, or microcapsules. Sustained release matrices may include polyesters, hydrogels, polylactides (U.S. patent no. 3,773,919 and EP 058,481), copolymers of L-glutamic acid and gamma ethyl-L-glutamate (Sidman et al., Biopolymers, 22:547-556 (1983)), poly (2-hydroxyethyl-methacrylate) (Langer et al., J. Biomed. Mater. Res., 15:167-277 (1981) and Langer, Chem. Tech., 12:98-105 (1982)), ethylene vinyl acetate (Langer *et al., supra)* or poly-D(-)-3-hydroxybutyric acid (EP 133,988). Sustained release compositions may also include liposomes, which can be prepared by any of several methods known in the art. *See e.g.*, Eppstein et al., Proc. Natl. Acad. Sci . USA, 82:3688-3692 (1985); EP 036,676; EP 088,046 and EP 143,949.

The IL-17 like pharmaceutical composition to be used for in vivo administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in a solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or as a dehydrated or lyophilized powder. Such formulations may be stored either in a ready-to-use form or in a form *(e.g.,* lyophilized) requiring reconstitution prior to administration.

In a specific embodiment, the present disclosure is directed to kits for producing a single-dose administration unit. The kits may each contain both a first container having a dried protein and a second container having an aqueous formulation. Also included within the scope of this invention are kits containing single and multi-chambered pre-filled syringes (*e.g.*, liquid syringes and lyosyringes).

The effective amount of an IL-17 like pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the IL-17 like molecule is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.1 μg/kg to up to about 100 mg/kg or more, depending on the factors mentioned above. In other embodiments, the dosage may range from 0.1 μg/kg up to about 100 mg/kg; or 1 μg/kg up to about 100 mg/kg; or 5 μg/kg up to about 100 mg/kg.

The frequency of dosing will depend upon the pharmacokinetic parameters of the IL-17 like molecule in the formulation used. Typically, a clinician will administer the composition until a dosage is reached that achieves the desired effect. The composition may therefore be administered as a single dose, or as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. Appropriate dosages may be ascertained through use of appropriate dose-response data.

The route of administration of the pharmaceutical composition is in accord with known methods, *e.g.,* orally, through injection by intravenous, intraperitoneal, intracerebral (intra-parenchymal), intracerebroventricular, intramuscular, intra-ocular, intraarterial, intraportal, or intralesional routes; by sustained release systems or by implantation devices. Where desired, the compositions may be administered by bolus injection or continuously by infusion, or by implantation device.

Alternatively or additionally, the composition may be administered locally via implantation of a membrane, sponge or another appropriate material onto which the desired molecule has been absorbed or encapsulated. Where an implantation device is used, the device may be implanted into any suitable tissue or organ, and delivery of the desired molecule may be via diffusion, timed-release bolus, or continuous administration.

In some cases, it may be desirable to use IL-17 like pharmaceutical compositions in an ex *vivo* manner. In such instances, cells, tissues or organs that have been removed from the patient are exposed to IL-17 like pharmaceutical compositions after which the cells, tissues and/or organs are subsequently implanted back into the patient.

In other cases, an IL-17 like polypeptide can be delivered by implanting certain cells that have been genetically engineered, using methods such as those described herein, to express and secrete the polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semi-permeable polymeric enclosures or membranes that allow the release of the protein product(s) but prevent the destruction of the cells by the patient's immune system or by other detrimental factors from the surrounding tissues.

Additional embodiments of the present disclosure relate to cells and methods (*e.g.*, homologous recombination and/or other recombinant production methods) for both the *in vitro* production of therapeutic polypeptides and for the production and delivery of therapeutic polypeptides by gene therapy or cell therapy. Homologous and other recombination methods may be used to modify a cell that contains a normally transcriptionally-silent IL-17 like gene, or an underexpressed gene, and thereby produce a cell which expresses therapeutically efficacious amounts of IL-17 like polypeptides.

Homologous recombination is a technique originally developed for targeting genes to induce or correct mutations in transcriptionally active genes (Kucherlapati, Prog. in Nucl. Acid Res. & Mol. Biol., 36:301 (1989)). The basic technique was developed as a method for introducing specific mutations into specific regions of the mammalian genome (Thomas et al., Cell, 44:419-428 (1986); Thomas and Capecchi, Cell, 51:503-512 (1987); Doetschman et al., Proc. Natl. Acad. Sci., 85:8583-8587 (1988)) or to correct specific mutations within defective genes (Doetschman et al., Nature, 330: 576-578 (1987)). Exemplary homologous recombination techniques are described in U.S. Patent No. 5,272,071 (EP 9193051, EP Publication No. 505500 and PCT/US90/07642, International Publication No. WO 91/09955).

Through homologous recombination, the DNA sequence to be inserted into the genome can be directed to a specific region of the gene of interest by attaching it to targeting DNA. The targeting DNA is a nucleotide sequence that is complementary (homologous) to a region of the genomic DNA. Small pieces of targeting DNA that are complementary to a specific region of the genome are put in contact with the parental strand during the DNA replication process. It is a general property of DNA that has been inserted into a cell to hybridize and therefore, recombine with other pieces of endogenous DNA through shared homologous regions. If this complementary strand is attached to an oligonucleotide that contains a mutation or a different sequence or an additional nucleotide, it too is incorporated into the newly synthesized strand as a result of the recombination. As a result of the proofreading function, it is possible for the new sequence of DNA to serve as the template. Thus, the transferred DNA is incorporated into the genome.

Attached to these pieces of targeting DNA are regions of DNA which may interact with or control the expression of an IL-17 like polypeptide, e.g., flanking sequences. For example, a promoter/enhancer element, a suppressor or an exogenous transcription modulatory element is inserted in the genome of the intended host cell in proximity and orientation sufficient to influence the transcription of DNA encoding the desired IL-17 like polypeptide. The control element controls a portion of the DNA present in the host cell genome. Thus, the expression of the desired IL-17 like polypeptide may be achieved not by transfection of DNA that encodes the IL-17 like gene itself, but rather by the use of targeting DNA (containing regions of homology with the endogenous gene of interest), coupled with DNA regulatory segments that provide the endogenous gene sequence with recognizable signals for transcription of an IL-17 like gene.

In an exemplary method, the expression of a desired targeted gene in a cell (i.e., a desired endogenous cellular gene) is altered via homologous recombination into the cellular genome at a preselected site by the introduction of DNA which includes at least a regulatory sequence, an exon and a splice donor site. These components are introduced into the chromosomal (genomic) DNA in such a manner that this, in effect, results in the production of a new transcription unit (in which the regulatory sequence, the exon and the splice donor site present in the DNA construct are operatively linked to the endogenous gene). As a result of the introduction of these components into the chromosomal DNA, the expression of the desired endogenous gene is altered.

Altered gene expression, as described herein, encompasses activating (or causing to be expressed) a gene which is normally silent (unexpressed) in the cell as obtained, as well as increasing the expression of a gene which is not expressed at physiologically significant levels in the cell as obtained. The embodiments further encompass changing the pattern of regulation or induction such that it is different from the pattern of regulation or induction that occurs in the cell as obtained, and reducing (including eliminating) the expression of a gene which is expressed in the cell as obtained.

One method by which homologous recombination can be used to increase, or cause, IL-17 like polypeptide production from a cell's endogenous IL-17 like gene involves first using homologous recombination to place a recombination sequence from a site-specific recombination system (*e.g.,* Cre/loxP, FLP/FRT) (see, Sauer, Current Opinion In Biotechnology, 5:521-527 (1994) and Sauer, Methods In Enzymology, 225:890-900 (1993)) upstream (that is, 5' to) of the cell's endogenous genomic IL-17 like polypeptide coding region. A plasmid containing a recombination site homologous to the site that was placed just upstream of the genomic IL-17 like polypeptide coding region is introduced into the modified cell line along with the appropriate recombinase enzyme. This recombinase enzyme causes the plasmid to integrate, via the plasmid's recombination site, into the recombination site located just upstream of the genomic IL-17 like polypeptide coding region in the cell line (Baubonis and Sauer, Nucleic Acids Res., 21:2025-2029, 1993 and O'Gorman et al., Science, 251:1351-1355 (1991)). Any flanking sequences known to increase transcription *(e.g.,* enhancer/promoter, intron or translational enhancer), if properly positioned in this plasmid, would integrate in such a manner as to create a new or modified transcriptional unit resulting in *de novo* or increased IL-17 like polypeptide production from the cell's endogenous IL-17 like gene.

A further method to use the cell line in which the site-specific recombination sequence has been placed just upstream of the cell's endogenous genomic IL-17 like polypeptide coding region is to use homologous recombination to introduce a second recombination site elsewhere in the cell line's genome. The appropriate recombinase enzyme is then introduced into the two-recombination-site cell line, causing a recombination event (deletion, inversion or translocation) (Sauer, Current Opinion In Biotechnology, supra (1994) and Sauer, Methods In Enzymology, supra, (1993)) that would create a new or modified transcriptional unit resulting in *de novo* or increased IL-17 like polypeptide production from the cell's endogenous IL-17 like gene.

An additional approach for increasing, or causing, the expression of IL-17 like polypeptide from a cell's endogenous IL-17 like gene involves increasing, or causing, the expression of a gene or genes *(e.g.,* transcription factors) and/or decreasing the expression of a gene or genes *(e.g.,* transcriptional repressors) in a manner which results in *de novo* or increased IL-17 like polypeptide production from the cell's endogenous IL-17 like gene. This method includes the introduction of a non-naturally occurring polypeptide *(e.g.,* a polypeptide comprising a site-specific DNA binding domain fused to a transcriptional factor domain) into the cell such that *de novo* or increased IL-17 like polypeptide production from the cell's endogenous IL-17 like gene results.

The present disclosure further relates to DNA constructs useful in the method of altering expression of a target gene. In certain embodiments, the exemplary DNA constructs comprise: (a) one or more targeting sequences; (b) a regulatory sequence; (c) an exon and (d) an unpaired splice-donor site. The targeting sequence in the DNA construct directs the integration of elements (a)-(d) into a target gene in a cell such that the elements (b)-(d) are operatively linked to sequences of the endogenous target gene. In another embodiment, the DNA constructs comprise: (a) one or more targeting sequences, (b) a regulatory sequence, (c) an exon, (d) a splice-donor site, (e) an intron and (f) a splice-acceptor site, wherein the targeting sequence directs the integration of elements (a)-(f) such that the elements of (b)-(f) are operatively linked to the endogenous gene. The targeting sequence is homologous to the preselected site in the cellular chromosomal DNA with which homologous recombination is to occur. In the construct, the exon is generally 3' of the regulatory sequence and the splice-donor site is 3' of the exon.

If the sequence of a particular gene is known, such as the nucleic acid sequence of IL-17 like polypeptide presented herein, a piece of DNA that is complementary to a selected region of the gene can be synthesized or otherwise obtained, such as by appropriate restriction of the native DNA at specific recognition sites bounding the region of interest. This piece serves as a targeting sequence(s) upon insertion into the cell and will hybridize to its homologous region within the genome. If this hybridization occurs during DNA replication, this piece of DNA, and any additional sequence attached thereto, will act as an Okazaki fragment and will be incorporated into the newly synthesized daughter strand of DNA. The present disclosure, therefore, includes nucleotides encoding a IL-17 like polypeptide, which nucleotides may be used as targeting sequences.

IL-17 like polypeptide cell therapy, *e.g.,* the implantation of cells producing IL-17 like polypeptides, is also contemplated. This embodiment involves implanting cells capable of synthesizing and secreting a biologically active form of IL-17 like polypeptide. Such IL-17 like polypeptide-producing cells can be cells that are natural producers of IL-17 like polypeptides or may be recombinant cells whose ability to produce IL-17 like polypeptides has been augmented by transformation with a gene encoding the desired IL-17 like polypeptide or with a gene augmenting the expression of IL-17 like polypeptide. Such a modification may be accomplished by means of a vector suitable for delivering the gene as well as promoting its expression and secretion. In order to minimize a potential immunological reaction in patients being administered an IL-17 like polypeptide, as may occur with the administration of a polypeptide of a foreign species, it is preferred that the natural cells producing IL-17 like polypeptide be of human origin and produce human IL-17 like polypeptide. Likewise, it is preferred that the recombinant cells producing IL-17 like polypeptide be transformed with an expression vector containing a gene encoding a human IL-17 like polypeptide.

Implanted cells may be encapsulated to avoid the infiltration of surrounding tissue. Human or non-human animal cells may be implanted in patients in biocompatible, semipermeable polymeric enclosures or in membranes that allow the release of IL-17 like polypeptide but prevent the destruction of the cells by the patient's immune system or by other detrimental factors from the surrounding tissue. Alternatively, the patient's own cells, transformed to produce IL-17 like polypeptides *ex vivo,* may be implanted directly into the patient without such encapsulation.

Techniques for the encapsulation of living cells are known in the art, and the preparation of the encapsulated cells and their implantation in patients may be routinely accomplished. For example, Baetge et al. (WO 95/05452 and PCT/US94/09299) describe membrane capsules containing genetically engineered cells for the effective delivery of biologically active molecules. The capsules are biocompatible and are easily retrievable. The capsules encapsulate cells transfected with recombinant DNA molecules comprising DNA sequences coding for biologically active molecules operatively linked to promoters that are not subject to down-regulation *in vivo* upon implantation into a mammalian host. The devices provide for delivery of the molecules from living cells to specific sites within a recipient. In addition, see U.S. Patent Nos. 4,892,538, 5,011,472 and 5,106,627. A system for encapsulating living cells is described in PCT Application no. PCT/US91/00157 of Aebischer *et al.* See also, PCT Application no. PCT/US91/00155 of Aebischer et al.*;* Winn et al., Exper. Neurol., 113:322-329 (1991), Aebischer et al., Exper. Neurol., 111:269-275 (1991); and Tresco et al., ASAIO, 38:17-23 (1992).

*In vivo* and *in vitro* gene therapy delivery of IL-17 like polypeptides is also envisioned. One example of a gene therapy technique is to use the IL-17 like gene (either genomic DNA, cDNA and/or synthetic DNA) encoding an IL-17 like polypeptide which may be operably linked to a constitutive or inducible promoter to form a "gene therapy DNA construct". The promoter may be homologous or heterologous to the endogenous IL-17 like gene, provided that it is active in the cell or tissue type into which the construct will be inserted. Other components of the gene therapy DNA construct may optionally include, DNA molecules designed for site-specific integration *(e.g.,* endogenous sequences useful for homologous recombination); tissue-specific promoter, enhancer(s) or silencer(s); DNA molecules capable of providing a selective advantage over the parent cell; DNA molecules useful as labels to identify transformed cells; negative selection systems; cell-specific binding agents (as, for example, for cell targeting); cell-specific internalization factors; and transcription factors to enhance expression by a vector, as well as factors to enable vector manufacture.

A gene therapy DNA construct can then be introduced into cells (either *ex vivo* or *in vivo*) using viral or non-viral vectors. One means for introducing the gene therapy DNA construct is by means of viral vectors as described herein. Certain vectors, such as retroviral vectors, will deliver the DNA construct to the chromosomal DNA of the cells, and the gene can integrate into the chromosomal DNA. Other vectors will function as episomes, and the gene therapy DNA construct will remain in the cytoplasm.

In yet other embodiments, regulatory elements can be included for the controlled expression of the IL-17 like gene in the target cell. Such elements are turned on in response to an appropriate effector. In this way, a therapeutic polypeptide can be expressed when desired. One conventional control means involves the use of small molecule dimerizers or rapalogs (as described in WO 9641865 (PCT/US96/099486); WO 9731898 (PCT/US97/03137) and WO 9731899 (PCT/US95/03157)) used to dimerize chimeric proteins which contain a small molecule-binding domain and a domain capable of initiating biological process, such as a DNA-binding protein or a transcriptional activation protein. The dimerization of the proteins can be used to initiate transcription of the transgene.

An alternative regulation technology uses a method of storing proteins expressed from the gene of interest inside the cell as an aggregate or cluster. The gene of interest is expressed as a fusion protein that includes a conditional aggregation domain which results in the retention of the aggregated protein in the endoplasmic reticulum. The stored proteins are stable and inactive inside the cell. The proteins can be released, however, by administering a drug (*e.g.,* small molecule ligand) that removes the conditional aggregation domain and thereby specifically breaks apart the aggregates or clusters so that the proteins may be secreted from the cell. See, Science 287:816-817 and 826-830 (2000).

Other suitable control means or gene switches include, but are not limited to, the following systems. Mifepristone (RU486) is used as a progesterone antagonist. The binding of a modified progesterone receptor ligand-binding domain to the progesterone antagonist activates transcription by forming a dimer of two transcription factors which then pass into the nucleus to bind DNA. The ligand-binding domain is modified to eliminate the ability of the receptor to bind to the natural ligand. The modified steroid hormone receptor system is further described in U.S. 5,364,791; WO 9640911 and WO 9710337.

Yet another control system uses ecdysone (a fruit fly steroid hormone) which binds to and activates an ecdysone receptor (cytoplasmic receptor). The receptor then translocates to the nucleus to bind a specific DNA response element (promoter from ecdysone-responsive gene). The ecdysone receptor includes a transactivation domain/DNA-binding domain/ligand-binding domain to initiate transcription. The ecdysone system is further described in U.S. 5,514,578; WO 9738117; WO 9637609 and WO9303162.

Another control means uses a positive tetracycline-controllable transactivator. This system involves a mutated tet repressor protein DNA-binding domain (mutated tet R-4 amino acid changes which resulted in a reverse tetracycline-regulated transactivator protein, *i.e.,* it binds to a tet operator in the presence of tetracycline) linked to a polypeptide which activates transcription. Such systems are described in U.S. Patent Nos. 5,464,758; 5,650,298 and 5,654,168.

Additional expression control systems and nucleic acid constructs are described in U.S. Patent Nos. 5,741,679 and 5,834,186, to Innovir Laboratories Inc.

In vivo gene therapy may be accomplished by introducing the gene encoding an IL-17 like polypeptide into cells via local injection of an IL-17 like nucleic acid molecule or by other appropriate viral or non-viral delivery vectors (Hefti, Neurobiology, 25:1418-1435 (1994)). For example, a nucleic acid molecule encoding an IL-17 like polypeptide may be contained in an adeno-associated virus (AAV) vector for delivery to the targeted cells (e.g., Johnson, International Publication No. WO 95/34670 and International Application No. PCT/US95/07178). The recombinant AAV genome typically contains AAV inverted terminal repeats flanking a DNA sequence encoding an IL-17 like polypeptide operably linked to functional promoter and polyadenylation sequences.

Alternative suitable viral vectors include, but are not limited to, retrovirus, adenovirus, herpes simplex virus, lentivirus, hepatitis virus, parvovirus, papovavirus, poxvirus, alphavirus, coronavirus, rhabdovirus, paramyxovirus, and papilloma virus vectors. U.S. Patent No. 5,672,344 describes an *in vivo* viral-mediated gene transfer system involving a recombinant neurotrophic HSV-1 vector. U.S. Patent No. 5,399,346 provides examples of a process for providing a patient with a therapeutic protein by the delivery of human cells which have been treated *in vitro* to insert a DNA segment encoding a therapeutic protein. Additional methods and materials for the practice of gene therapy techniques are described in U.S. Patent No. 5,631,236 involving adenoviral vectors; U.S. Patent No. 5,672,510 involving retroviral vectors; and U.S. 5,635,399 involving retroviral vectors expressing cytokines.

Nonviral delivery methods include, but are not limited to, liposome-mediated transfer, naked DNA delivery (direct injection), receptor-mediated transfer (ligand-DNA complex), electroporation, calcium phosphate precipitation, and microparticle bombardment (e.g., gene gun). Gene therapy materials and methods may also include the use of inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, and transcription factors to enhance expression by a vector as well as methods of vector manufacture. Such additional methods and materials for the practice of gene therapy techniques are described in U.S. Patent No. 4,970,154 involving electroporation techniques; WO96/40958 involving nuclear ligands; U.S. Patent No. 5,679,559 describing a lipoprotein-containing system for gene delivery; U.S. Patent No. 5,676,954 involving liposome carriers; U.S. Patent No. 5,593,875 concerning methods for calcium phosphate transfection; and U.S. Patent No. 4,945,050 wherein biologically active particles are propelled at cells at a speed whereby the particles penetrate the surface of the cells and become incorporated into the interior of the cells.

It is also contemplated that IL-17 like gene therapy or cell therapy can further include the delivery of one or more additional polypeptide(s) in the same or a different cell(s). Such cells may be separately introduced into the patient, or the cells may be contained in a single implantable device, such as the encapsulating membrane described above, or the cells may be separately modified by means of viral vectors.

A means to increase endogenous IL-17 like polypeptide expression in a cell via gene therapy is to insert one or more enhancer element(s) into the IL-17 like polypeptide promoter, where the enhancer element(s) can serve to increase transcriptional activity of the IL-17 like gene. The enhancer element(s) used will be selected based on the tissue in which one desires to activate the gene(s); enhancer element(s)known to confer promoter activation in that tissue will be selected. For example, if a gene encoding an IL-17 like polypeptide is to be "turned on" in T-cells, the *lck* promoter enhancer element may be used. Here, the functional portion of the transcriptional element to be added may be inserted into a fragment of DNA containing the IL-17 like polypeptide promoter (and optionally, inserted into a vector and/or 5' and/or 3' flanking sequence(s), etc.) using standard cloning techniques. This construct, known as a "homologous recombination construct", can then be introduced into the desired cells either *ex vivo* or *in vivo.*

Gene therapy also can be used to decrease IL-17 like polypeptide expression by modifying the nucleotide sequence of the endogenous promoter(s). Such modification is typically accomplished via homologous recombination methods. For example, a DNA molecule containing all or a portion of the promoter of the IL-17 like gene(s) selected for inactivation can be engineered to remove and/or replace pieces of the promoter that regulate transcription. For example the TATA box and/or the binding site of a transcriptional activator of the promoter may be deleted using standard molecular biology techniques; such deletion can inhibit promoter activity thereby repressing the transcription of the corresponding IL-17 like gene. The deletion of the TATA box or the transcription activator binding site in the promoter may be accomplished by generating a DNA construct comprising all or the relevant portion of the IL-17 like polypeptide promoter(s) (from the same or a related species as the IL-17 like gene(s) to be regulated) in which one or more of the TATA box and/or transcriptional activator binding site nucleotides are mutated via substitution, deletion and/or insertion of one or more nucleotides. As a result, the TATA box and/or activator binding site has decreased activity or is rendered completely inactive. The construct will typically contain at least about 500 bases of DNA that correspond to the native (endogenous) 5' and 3' DNA sequences adjacent to the promoter segment that has been modified. The construct may be introduced into the appropriate cells (either ex vivo or in vivo) either directly or via a viral vector as described herein. Typically, the integration of the construct into the genomic DNA of the cells will be via homologous recombination, where the 5' and 3' DNA sequences in the promoter construct can serve to help integrate the modified promoter region via hybridization to the endogenous chromosomal DNA.

**<u>Additional Uses of IL-17 like Nucleic Acids and Polypeptides</u>**

Nucleic acid molecules as disclosed herein (including those that do not themselves encode biologically active polypeptides) may be used to map the locations of the IL-17 like gene and related genes on chromosomes. Mapping may be done by techniques known in the art, such as PCR amplification and in situ hybridization.

IL-17 like nucleic acid molecules (including those that do not themselves encode biologically active polypeptides), may be useful as hybridization probes in diagnostic assays to test, either qualitatively or quantitatively, for the presence of an IL-17 like DNA or corresponding RNA in mammalian tissue or bodily fluid samples.

The IL-17 like polypeptides may be used (simultaneously or sequentially) in combination with one or more cytokines, growth factors, antibiotics, anti-inflammatories and/or chemotherapeutic agents as is appropriate for the indication being treated.

Other methods may also be employed where it is desirable to inhibit the activity of one or more IL-17 like polypeptides. Such inhibition may be effected by nucleic acid molecules which are complementary to and which hybridize to expression control sequences (triple helix formation) or to IL-17 like mRNA. For example, antisense DNA or RNA molecules, which have a sequence that is complementary to at least a portion of the selected IL-17 like gene(s) can be introduced into the cell. Antisense probes may be designed by available techniques using the sequence of IL-17 like polypeptide disclosed herein. Typically, each such antisense molecule will be complementary to the start site (5' end) of each selected IL-17 like gene. When the antisense molecule then hybridizes to the corresponding IL-17 like mRNA, translation of this mRNA is prevented or reduced. Antisense inhibitors provide information relating to the decrease or absence of an IL-17 like polypeptide in a cell or organism.

Alternatively, gene therapy may be employed to create a dominant-negative inhibitor of one or more IL-17 like polypeptides. In this situation, the DNA encoding a mutant polypeptide of each selected IL-17 like polypeptide can be prepared and introduced into the cells of a patient using either viral or non-viral methods as described herein. Each such mutant is typically designed to compete with endogenous polypeptide in its biological role.

In addition, an IL-17 like polypeptide, whether biologically active or not, may be used as an immunogen, that is the polypeptide contains at least one epitope to which antibodies may be raised. Selective binding agents that bind to an IL-17 like polypeptide (as described herein) may be used for *in vivo* and *in vitro* diagnostic purposes, including but not limited to, use in labeled form to detect the presence of IL-17 like polypeptide in a body fluid or cell sample. The antibodies may also be used to prevent, treat or diagnose a number of diseases and disorders, including those recited herein. The antibodies may bind to an IL-17 like polypeptide so as to diminish or block at least one activity characteristic of an IL-17 like polypeptide, or may bind to a polypeptide to increase at least one activity characteristic of an IL-17 like polypeptide (including by increasing the pharmacokinetics of the IL-17 like polypeptide).

The following examples are for illustration purposes.

## EXAMPLE 1

### Cloning of Human-IL17E

An IL-8 family profile search of the Amgen and Genbank dbEST database was performed, resulting in the identification of the mouse EST, zmgb-ai430337. (Smith et al. (1994), Cell, 76: 959-62; Luethy et al. (1994), Protein Science, 3: 139-46). The overall homology between the zmgb-ai430337 predicted amino acid sequence and the human IL-8 was low; however, the conservation of cysteines and other key residues suggested that zmgb-ai430337 was a novel member of the IL-17 family. The clone corresponding to the mouse EST sequence was obtained from the NIH I.M.A.G.E. Consortium through Research Genetics (an Invitrogen Company; Huntsville, AL) and was fully sequenced. A BLAST search revealed that the mouse EST sequence corresponded to regions of the GenBank BAC clone sequence CNS0000B, which is genomic DNA sequence derived from human chromosome 14. Additional BLAST searches also revealed matches with Celera human genomic sequences. Similarity to both the GenBank and Celera sequences suggested there was a human counterpart to the identified novel mouse IL-17 family member.

Two PCR primers based on the human genomic sequence were designed to screen various cDNA libraries (Clontech) for the IL-17 related cDNA. The forward primer, designated 2392-73 has the sequence AGA GTC CTG TAG GGC CAG TGA AGA TGG (SEQ ID NO: 15). The reverse primer, designated 2374-88, has the sequence TAC AGC CTG CGC TCC AGG CAG TAG CC (SEQ ID NO: 16). This screening indicated that the human testis were the most abundant source among those cDNA samples screened.

In order to obtain a full-length human cDNA clone, the Rapid Screen human testis cDNA library (LTS-1001) was obtained from Origene Technologies Inc. (Rockville, MD). The Origene cDNA library was in the vector pCMV6-XL4. Standard PCR conditions were used for all of the cDNA library screening. According to the Origene product specifications, the library contained 500,000 clones arrayed in a 96-well primary plate. Each well of the primary plate therefore contained about 5,000 clones. Well 3B of the primary plate was positive. The secondary sub-plate corresponidng to the positive well 3B on the primary plate was purchased from Origene. The subsequent screening demonstrated that well number 55 on the 3B secondary sub-plate was positive.

The Origene secondary sub-plate contained glycerol stocks of E. coli amplified from 50 original clones. Serial dilutions of the positive well number 55 were plated at densities of 1:10, 1:100 and 1:1,000 on LB Amp plates. Colonies (96) from the LB Amp plates were individually picked and were used for PCR screening with primers 2392-73 and 2374-88 (described above). Four positive colonies were identified using this procedure. The positive colonies were designated as clone 70, 78, 85 and 89. The plasmid DNAs corresponding to these colonies were prepared and were sequenced using a combination of vector primers and gene-specific primers. Clone 89 was fully sequenced and the other clones were sequenced at the ends and in the coding sequence. The data revealed that the four clones had identical coding sequences.

The human IL-17 like cDNA (clone Origene-89) is 3987 bp in length and is set out as SEQ ID NO: 1. This cDNA encodes an open reading frame of 161 amino acids with a predicted signal peptide of 16 amino acids and a predicted mature protein of 145 amino acids (SEQ ID NO: 2). A FASTA search of the SwissProt database with the predicted IL-17 like protein sequence indicated that SEQ ID NO: 2 exhibited 25.0% identity within 160 amino acid overlap with IL-17, 36.7% identity within 90 amino acid overlap with IL-20, 35.6% identity within 90 amino acid overlap with IL-17B and 34.5% identity within 171 amino acid overlap with IL-17C. Similar to other IL-17 family members, the novel human IL-17 like polypeptide (also denoted IL-17E herein) is predicted to be a secreted protein and is predicted to be a cytokine.

## EXAMPLE 2

### IL-17 like Polynucleotide Overexpressing Transgenic Mice

#### A. Transgene Preparation.

The coding region of human IL-17 like cDNA (SEQ ID NO: 1)with an altered Kozak sequence, CCACC, immediately upstream of the initiating ATG, was ligated into a liver-specific expression vector. The expression vector consisted of a 774-bp DNA fragment containing the hepatocyte control region (HCR) from the human apolipoprotein (apo) C-I/C-I' intergenic region on chromosome 19 (Simonet et al., J. Biol. Chem., 268:8221-8229, 1993). The vector also contained a 1450-bp continuous piece of DNA which consisted of the human apoE gene 5'-flanking sequence, the first exon, the first intron and a portion of the second exon of the apoE gene (Simonet et al., J. Clin. Invest., 94:1310-1319, 1994). An SV40 polyadenylation signal was located downstream of the cDNA insert sites. The integrity of the cDNA was verified by sequencing using standard methods known in the art.

**B. Preparation and Analysis of Transgenic Mice.**

The resulting plasmid (denoted herein as ApoE-hIL-17) was purified and the transgene insert was isolated for microinjection. Single-cell embryos from BDF1 x BDF1-bred mice were injected essentially as described in Brinster et al. (Proc. Natl. Acad. Sci. USA, 82:4438-4442, 1985). Embryos were cultured overnight in a 37°C and 5% $CO_2$ incubator. Subsequently, 15 to 20 2-cell embryos were transferred to the oviducts of thirteen pseudopregnant CD1 female mice. Transgenic offsprings were identified by PCR screening with primers that amplify a 368-bp fragment of the human apoE first intron from DNA prepared from ear biopsies as described in Simonet et al. (J. Clin. Invest., 94:1310-1319, 1994).

**EXAMPLE 3**

**Necropsy Analysis of the Transgenic Mice.**

At 8-10 weeks of age, 10 IL-17 like transgenic mice and five non-transgenic littermates were necropsied. Liver samples from the mice were flash frozen in liquid nitrogen at the time of necropsy. RNAs were isolated from each sample using the Perfect RNA Kit (Eppendorf) according to the manufacturer's instructions and analyzed by Northern blot analysis.

The Northern blot was generated by running 10 μg of RNA diluted in 1x RNA Loading Dye (Sigma) on a 1% formaldehyde-agarose gel. The gel was denatured in 50 mM NaOH and 150 and 55 mM NaCl. Subsequently, the gel was neutralized in 0.1 M Tris-HCl (pH 7.0) and 150 mM NaCl and blotted onto a Duralon membrane according to the manufacturer's instructions (Stratagene). The Northern blot was probed with a $^{32}P$-labeled human IL-17 like cDNA generated by the Rediprime System (Amersham). Hybridization was carried out in Express Hyb Solution and then washed according to the manufacturer's instructions. The hybridized blot was exposed to X-ray film (Kodak) for 72 hours at -80°C and then developed.

The Northern blot analysis indicated that the transgenic founder mice had increased expression of the IL-17 like RNA as compared with the non-transgenic littermates. Of the 10 mice tested, those denoted as nos. 29,52, 55, 61 and 66 had the highest level of IL-17 like RNA expression. (See figure 7)

**B. Expression Analysis on the Remaining Founders**

Livers from the remaining transgenic founder mice along with control mice, were obtained by partial hepatectomy. The mice were anesthesized by isoflourane and a small transverse incision below the xyphoid process on the sternum was made to expose the liver. A suture was placed around the lobe of liver selected for excision at the point of attachment. The lobe of liver was ligated and removed by cutting below the ligature and flash frozen in liquid nitrogen. The mouse was then checked for bleeding and the skin incision was closed with 1-2 autoclips (skin staples). RNA was isolation from the liver and Northern blot analysis was carried out as described above. The hybridized blot was exposed to X-ray film (Kodak) for 24 hours at -80°C and then developed.

Northern blot analysis on the remaining founders indicated that these mice expressed higher levels of IL-17 like RNA in the liver as compared with non-transgenic littermates. The mice denoted as nos. 11, 30, 33, 46 and 68 expressed the highest levels of IL-17 RNA. (See figure 8).

**EXAMPLE 4**

**Pathological Analysis of IL-17 like Transgenic Mice**

**A. Necropsy**

In this study, seven, 6-8 week old, IL-17 like mice as well as five, 6-8 week old, non-transgenic littermates (two males and three females) were pathologically analyzed for a potential IL-17 like phenotype. Mice nos. 29, 52, 61 and 66 were strongly positive for hepatic expression of IL-17 like mRNA, while mice nos. 1, 16 and 55 were weakly positive. The five non-trangenic control mice (nos. 2, 17, 28, 53 and 65) were negative. At necropsy, mice were weighed, blood was drawn for hematology and serum chemistries, and liver, spleen, kidney, heart, and thymus were weighed. Sections of liver, spleen, lung, brain, heart, kidney, adrenal, stomach, small intestine, pancreas, cecum, colon, mesenteric lymph node, skin, mammary gland, trachea, esophagus, thyroid, parathyroid, salivary gland, urinary bladder, ovary or testis, uterus or seminal vesicle, skeletal muscle, bone, and bone marrow were harvested for histologic analysis.

## B. Histology

Sections of liver, spleen, lung, brain, heart, kidney, adrenal, stomach, small intestine, pancreas, cecum, colon, mesenteric lymph node, skin, mammary gland, trachea, esophagus, thyroid, parathyroid, salivary gland, urinary bladder, ovary or testis, uterus or seminal vesicle, skeletal muscle, bone, and bone marrow from the IL-17 like transgenic and non-transgenic mice were fixed overnight in 10% neutral buffered zinc formalin (Anatech, Battle Creek, Michigan), paraffin embedded, sectioned at 3 μm, and stained with hematoxylin and eosin (H&E) for routine histologic examination.

## C. Immunohistochemistry

Immunohistochemical staining was performed on 4 μm thick paraffin embedded sections using an automated DPC Mark 5 Histochemical Staining System (Diagnostic Products Corp, Randolph, NJ). Deparaffinized tissue sections were blocked with CAS BLOCK (Zymed Laboratories, San Francisco, CA), incubated with a rat anti-mouse monoclonal antibody directed against macrophages (F4/80, Serotec Inc., Raleigh, NC) or a rat anti-mouse CD45R/B220 monoclonal antibody directed against all types of B cells (PharMingen, San Diego, CA). The primary antibody was detected using a biotinylated rabbit anti-rat immunoglobulin secondary antibody (Vector Laboratories, Burlingame, CA). Sections were then quenched with 3% hydrogen peroxide and reacted with an avidin-biotin complex tertiary (Vector Laboratories). The staining reaction was visualized with diaminobenzidine (DAB, Dako Carpinteria, CA) and sections were counterstained with hematoxylin.

## D. Gross Pathology Findings

Mesenteric lymph nodes from the four high expressing IL-17 like transgenic mice (nos. 29, 52, 61 and 66) plus one of the low expressing mice (no. 55) were markedly increased in size. Similarly, the spleens

**Table 1 Raw Organ Weights for IL-17 like Transgenic Mice vs. Non-Transgenic Mice**

| Group | Sex | TBW | Liver | **%BW** | Spln | **%BW** | Heart | **%BW** |
|---|---|---|---|---|---|---|---|---|
| Non-Transgenic | | | | | | | | |
| 2 | F | 21.8 | 0.923 | 4.23 | 0.070 | 0.32 | 0.121 | 0.56 |
| 17 | F | 20.5 | 0.912 | 4.45 | 0.089 | 0.43 | 0.112 | 0.55 |
| 28 | F | 22.5 | 1.125 | 5 | 0.123 | 0.55 | 0.127 | 0.56 |
| 53 | M | 25.8 | 1.315 | 5.1 | 0.076 | 0.29 | 0.140 | 0.54 |
| 65 | M | 29 | 1.45 | 5 | 0.082 | 0.28 | 0.169 | 0.58 |
| Mean | | | | 4.76 | | 0.37 | | 0.56 |
| St. Dev. | | | | 0.39 | | 0.12 | | 0.01 |
| IL-17 like Trans-genic | | | | | | | | |
| 1 | F | 31.9 | 1.406 | 4.41 | 0.118 | 0.37 | 0.151 | 0.47 |
| 16 | F | 22.5 | 1.121 | 4.98 | 0.085 | 0.38 | 0.115 | 0.51 |
| 29 | F | 24.4 | 1.439 | 5.90 | 0.333 | 1.36 | 0.123 | 0.5 |
| 52 | M | 25.6 | 1.583 | 6.18 | 0.223 | 0.87 | 0.129 | 0.5 |
| 55 | F | 19.1 | 1.181 | 6.18 | 0.196 | 1.03 | 0.122 | 0.64 |
| 61 | F | 24.5 | 1.401 | 5.72 | 0.190 | 0.78 | 0.118 | 0.48 |
| 66 | M | 25 | 1.47 | 5.88 | 0.338 | 1.35 | 0.162 | 0.65 |
| Mean | | | | 5.61 | | 0.88 | | 0.54 |
| St. Dev. Group | | | | 0.67 | | 0.41 | | 0.08 |
| Non-Transgenic | | | Heart | **%BW** | Kidneys | **%BW** | Thymus | **%BW** |
| 2 | F | 21.8 | 0.121 | 0.56 | 0.351 | 1.61 | 0.061 | 0.28 |
| 17 | F | 20.5 | 0.112 | 0.55 | 0.273 | 1.33 | 0.048 | 0.23 |
| 28 | F | 22.5 | 0.127 | 0.56 | 0.398 | 1.77 | 0.058 | 0.26 |
| 53 | M | 25.8 | 0.140 | 0.54 | 0.423 | 1.64 | 0.031 | 0.12 |

(continued)

| Non-Transgenic | | | Heart | %BW | Kidneys | %BW | Thymus | %BW |
|---|---|---|---|---|---|---|---|---|
| 65 | M | 29 | 0.169 | 0.58 | 0.523 | 1.8 | 0.055 | 0.19 |
| Mean | | | | 0.56 | | 1.63 | | 0.22 |
| St. Dev. | | | | 0.01 | | 0.19 | | 0.06 |
| IL-17 like Trans-genic | | | | | | | | |
| 1 | F | 31.9 | 0.151 | 0.47 | 0.433 | 1.36 | 0.071 | 0.22 |
| 16 | F | 22.5 | 0.115 | 0.51 | 0.350 | 1.56 | 0.061 | 0.27 |
| 29 | F | 24.4 | 0.123 | 0.5 | 0.861 | 3.53 | 0.061 | 0.25 |
| 52 | M | 25.6 | 0.129 | 0.5 | 0.356 | 1.39 | 0.074 | 0.29 |
| 55 | F | 19.1 | 0.122 | 0.64 | 0.388 | 2.03 | 0.04 | 0.21 |
| 61 | F | 24.5 | 0.118 | 0.48 | 0.372 | 1.52 | 0.059 | 0.24 |
| 66 | M | 25 | 0.162 | 0.65 | 0.433 | 1.73 | 0.026 | 0.1 |
| Mean | | | | 0.54 | | 1.87 | | 0.23 |
| St. Dev. | | | | 0.08 | | 0.76 | | 0.06 |

from these five IL-17 like transgenic mice were enlarged and exhibited a significant increase in weight (1.08 ± 0.27 SD % of body weight vs. 0.37 ± 0.12 SD % of body weight in non-transgenic control mice, p=0.0007). Mesenteric lymph nodes and spleens from two other low expressing transgenic mice (nos. 1 and 16) appeared normal. The raw organ weight data is shown in Table 1 and significant differences are summarized in Table 3.

### E. Hematology Findings

Four of the five IL-17 like transgenic mice with enlarged mesenteric lymph nodes and spleens (the blood from mice nos. 29, 52, 55, 61 and 66 clotted and could not be evaluated) had moderate to marked increases in total leukocytes, neutrophils, lymphocytes, eosinophils, and large unstained cells (possibly large granular lymphocytes). The mean total leukocyte count for these four IL-17 like transgenic mice was 11.93 x $10^3$ (± 4.47 x $10^3$ SD) while non-transgenic control mice had a mean total leukocyte count of 3.09 x $10^3$ (± 0.79 x $10^3$ SD, p=0.003). The mean neutrophil count in these four IL-17 like transgenic mice was 2.29 x $10^3$ (± 0.67 x $10^3$ SD) vs. 0.92 x $10^3$ (± 0.53 x $10^3$ SD) in non-transgenic control mice, p=0.032. These four IL-17 like transgenic mice had a mean lymphocyte count of 6.76 x $10^3$ (± 2.32 x $10^3$) vs. 1.99 x $10^3$ (± 0.38 x $10^3$ SD) in non-transgenic control mice, p=0.0025, a mean eosinophil count of 1.35 x $10^3$ (± 0.96 x $10^3$ SD) vs. 0.03 x $10^3$ (± 0.01 x $10^3$ SD) in non-transgenic control mice, p=0.017, and a mean large unstained cell count of 1.41 x $10^3$ (± 1.11 x $10^3$ SD) vs. 0.10 x $10^3$ (± 0.05 x $10^3$ SD) in non-transgenic control mice, p=0.031. Two of the IL-17 like transgenic mice (nos. 55 and 66) also had a mild anemia characterized by a slight decrease in red blood cell number, hemoglobin, and hematocrit as well as slightly elevated platelet counts. The raw hematology data is shown in Table 2 and significant differences are summarized in Table 3.

**Table 2 Raw Hematology Data for IL-17 like Transgenic Mice vs. Non-Transgenic Mice**

| Group | WBC | RBC | HGB | HCT | PLT | MPV | Neut | Lymph | Mono | Eos | Baso | LUC |
|---|---|---|---|---|---|---|---|---|---|---|---|---|
| **Non-Transgenic** | | | | | | | | | | | | |
| 2 | 2.52 | 9.39 | 13.9 | 48.9 | 1179 | 5.0 | 0.69 | 1.64 | 0.02 | 0.03 | 0.01 | 0.13 |
| 17 | 3.48 | 10.12 | 15.1 | 50.9 | 938 | 5.1 | 0.72 | 2.63 | 0.02 | 0.04 | 0.01 | 0.06 |
| 28 | 2.45 | 9.51 | 14.8 | 49.5 | 1013 | 5.7 | 0.37 | 2.00 | 0.02 | 0.01 | 0.01 | 0.05 |
| 53 | 2.70 | 10.67 | 16.1 | 55.9 | 1353 | 5.0 | 0.61 | 1.88 | 0.04 | 0.04 | 0.01 | 0.11 |
| 65 | 4.30 | 11.55 | 17.8 | 61.4 | 1362 | 4.5 | 2.20 | 1.81 | 0.11 | 0.02 | 0.01 | 0.16 |
| **Mean** | **3.09** | **10.25** | **15.5** | **53.3** | **1169** | **5.1** | **0.92** | **1.99** | **0.04** | **0.03** | **0.01** | **0.10** |
| **St. Dev.** | **0.79** | **0.89** | **1.5** | **5.3** | **193** | **0.4** | **0.73** | **0.38** | **0.04** | **0.01** | **0.00** | **0.05** |

(continued)

| IL-17 like Transgenic | | | | | | | | | | | | |
|---|---|---|---|---|---|---|---|---|---|---|---|---|
| 1 | 2.80 | 10.80 | 16.3 | 56.8 | 1113 | 5.2 | 0.69 | 1.91 | 0.03 | 0.02 | 0.01 | 0.14 |
| 16 | 3.49 | 10.29 | 15.8 | 54.7 | 1134 | 4.8 | 1.30 | 2.01 | 0.05 | 0.04 | 0.01 | 0.07 |
| 29 | No Sample | | | | | | | | | | | |
| 52 | 13.32 | 8.81 | 12.5 | 45.8 | 977 | 6.3 | 3.25 | 6.61 | 0.17 | 2.12 | 0.04 | 1.13 |
| 55 | 16.89 | 7.89 | 12.0 | 36.6 | 2758 | 5.4 | 1.84 | 9.80 | 0.09 | 2.14 | 0.04 | 2.99 |
| 61 | 11.32 | 9.18 | 14.1 | 50.0 | 1102 | 5.2 | 2.66 | 6.47 | 0.08 | 0.96 | 0.03 | 1.12 |
| 66 | 6.19 | 6.24 | 7.8 | 31.7 | 2195 | 4.4 | 1.42 | 4.16 | 0.05 | 0.16 | 0.01 | 0.40 |
| **Mean** | **9.00** | **8.87** | **13.1** | **45.9** | **1547** | **5.2** | **1.86** | **5.16** | **0.08** | **0.91** | **0.02** | **0.98** |
| **SD** | **5.71** | **1.66** | **3.1** | **10.0** | **744** | **0.6** | **0.94** | **3.06** | **0.05** | **1.01** | **0.02** | **1.09** |

**Table 3 Summary Data for Significant Differences in Organ Weights and CBC Values between IL-17 like Transgenic Mice and Non-Transgenic mice**

| | **HEAGP Transgenic Mice (n=4 or 5*)** | **Non-Transgenic Mice (n=5)** | **p value (t Test)** |
|---|---|---|---|
| **Spleen Weight as % Body Weight** | 1.08 ± 0.27 SD* | 0.37 ± 0.12 SD | 0.0007 |
| **Total Leukocytes (WBCs)** | 11.93 x $10^3$ ± 4.47 x $10^3$ SD | 3.09 x $10^3$ ± 0.79 x $10^3$ SD | 0.003 |
| **Neutrophils** | 2.29 x $10^3$ ± 0.67 x $10^3$ SD | 0.92 x $10^3$ ± 0.53 x $10^3$ SD | 0.032 |
| **Lymphocytes** | 6.76 x $10^3$ ± 2.32 x $10^3$ vs SD | 1.99 x $10^3$ ± 0.38 x $10^3$ SD | 0.0025 |
| **Eosinophils** | 1.99 x $10^3$ ± 0.38 x $10^3$ SD | 0.03 x $10^3$ ± 0.01 x $10^3$ SD | 0.017 |
| **Large Unstained Cells (LUC - Possibly Large Granular Lymphocytes)** | 1.41 x $10^3$ ± 1.11 x $10^3$ SD | 0.10 x $10^3$ ± 0.05 x $10^3$ SD | 0.031 |

### F. Histopathologic Findings

Hematoxylin and eosin stained sections of liver, spleen, lung, brain, heart, kidney, adrenal, stomach, small intestine, pancreas, cecum, colon, mesenteric lymph node, skin, mammary gland, trachea, esophagus, thyroid, parathyroid, salivary gland, urinary bladder, ovary or testis, uterus or seminal vesicle, skeletal muscle, bone, and bone marrow were examined from seven IL-17 like transgenic mice and five non-transgenic control littermates. B220 (specific for all B cells) and F4/80 (specific for macrophages) immunostained sections of lymph node and spleen were also examined from all mice. Five of the IL-17 like transgenic mice (nos. 29, 52, 55, 61 and 66) had similar histologic findings characterized by marked mesenteric lymphadenopathy, splenic lymphoid hyperplasia and red pulp eosinophilic myeloid hyperplasia, and bone marrow eosinophilic hyperplasia. The most striking histologic finding was the mesenteric lymphadenopathy, which was characterized by massive nodal enlargement with loss of normal nodal architecture and medullary expansion by a mixed population of inflammatory cells containing a large number of eosinophils, reactive B cells (stained with B220) and plasma cells, macrophages (stained with F4/80) and multinucleated inflammatory giant cells (See figure 9). These five IL-17 like transgenic mice also exhibited marked bone marrow eosinophilic myeloid hyperplasia (figure 10B) as well as moderate to marked splenic B cell lymphoid hyperplasia and red pulp eosinophilic myeloid hyperplasia (figure 10F). In addition, one of the IL-17 like transgenic mice (no.29) also exhibited marked, chronic eosinophilic and suppurative pyelonephritis with renal pelvic dilation in one kidney and moderate chronic eosinophilic and suppurative pyelitis in the other kidney (figure 10J), while another IL-17 like transgenic mouse (no. 55) exhibited severe, chronic eosinophilic and suppurative urinary cystitis with mild bilateral chronic eosinophilic and suppurative pyelitis. Lastly, four of the IL-17 like transgenic mice (nos. 29, 55, 61 and 66) exhibited minimal to mild eosinophilic and lymphoplasmacytic colitis and/or ileitis.

### G. Summary of Phenotypic Findings in Transgenic Mice Overexpressing Human IL-17 Like Polypeptide

Five of the IL-17 like transgenic mice (nos. 29, 52, 55, 61 and 66) had a similar phenotype, characterized by a leukocytosis with marked elevations in eosinophils, lymphocytes, and large unstained cells which may be large granular lymphocytes, a marked lymphadenopathy with a marked eosinophilic component, bone marrow eosinophilic myeloid hyperplasia, and splenic B cell lymphoid hyperplasia and eosinophilic myeloid hyperplasia. Two of the IL-17 like transgenic mice (nos. 55 and 66) also exhibited mild anemia and thrombocytosis. In addition, IL-17 like transgenic mice nos. 55 and 29, exhibited eosinophilic and superlative inflammation of their kidneys and/or urinary bladder. Lastly, four of the IL-17 like transgenic mice (nos. 29, 55, 61 and 66) had minimal to mild eosinophilic and lymphoplasmacytic colitis and/or ileitis. All of these findings suggest that the IL-17 like protein plays a role in inflammation and myelopoiesis, particularly in the development, stimulation, and/or recruitment of eosinophils and B lymphocytes.

## EXAMPLE 5

### Transgenic Phenotype of IL-17 like Polypeptide Overexpressing Mice

Phenotype analysis was performed on 10 transgenic mice and 5 non-transgenic littermates. A femur, peripheral blood (obtained by cardiac puncture) and a longitudinal half section of spleen were obtained from each transgenic mouse and their littermate control. Five of the trangenic mice analyzed (nos. 29, 52,55,61 and 66) exhibited phenotypic changes.

To analyze the phenotype of the transgenic mice, the major hematopoietic populations including activated T cells were quantitated. In addition, the tissue and lineage specific expression of IL-17 like receptor, IL-17RB, was quantitated as described in Example 6 herein.

The following antibody panel was designed to make the above-identified measurements with fluorescent activated cell sorting (FACS). CD4-PE antibody was used to detect helper T cells. CD69 is an early activation marker and CD69-FITC antibody was used to detect activated cells. CD3-FITC antibody was used to detect all T cells. CD8-PE antibody was used to detect killer T cells. CD14-FITC antibody was used to detect cells of Monocyte lineage. CD19-PE antibody was used to detect B lineage cells (preB to mature surface immunoglobulin positive B cell). GR-1-FITC antibody was used to detect granulocytes. NK1.1-PE antibody was used to detect natural killer cells. The expression pattern of the IL-17 like cytokine receptor (IL17RB) was detected by binding of recombinant IL-17 like-Fc fusion protein and developed with appropriate anti-human-FITC antibodies. CD45R-PE antibody was used to detect B cells. CD11-PE antibody was used to detect dendritic cells. CD5 antibody was used as a possible indicator of leukemia/lymphoma when co-expressed with CD19. CD34 antibody was also used as a possible indicator of leukemia/lymphoma when co-expressed with CD19. (as described in Example 10). All of the antibodies were obtained from BD-Pharmingen, San Diego, CA.

The transgenic mice and non-transgenic littermates were sacrificed and the femurs and spleens were dissected. Cell suspension from the femoral bone marrow and the spleen were made, washed twice and resuspended in PBS/ 0.5% BSA. The cell number of each cell suspension was quantitated with a Coulter Z1 Coulter Counter using a 100 $\mu$m aperture and a lower threshold setting of 4 $\mu$m. A 10 $\mu$l alloquot of each cell suspension was added to 10 ml of Isoton buffer containing 3 drops of Zapoglobin (to lyse the red blood cells) and counted. The cell suspensions were incubated with Fc-block (CD 16/32) for 15 minutes at 4°C. Subsequently, $1x10^6$ cells (suspended in PBS/0.5% BSA) were added to each antibody-containing well on a 96 well plate.

In addition, peripheral blood samples from the transgenic mice and non-transgenic littermates were obtained by cardiac puncture and CBC analysis was performed. Subsequently, the remaining blood was divided equally among 8 wells containing the antibodies on a 96 well plate.

The cell suspensions and blood samples were incubated in the presence of the antibodies for 30 minutes at room temperature. Subsequently, the cells were washed twice and lysed with FACS lysing buffer (200 $\mu$l/well; Becton Dickinson) for 15 minutes at room temperature in order to eliminate the red blood cells. After lysing, the cells were washed and resuspended in 400 $\mu$l of FACS buffer and analyzed by flow cytometry.

In the 5 transgenic mice which exhibited a phenotype (nos. 29, 52, 55, 61 and 66), there was a striking increase in CD19+ cells (B cells) in the peripheral blood. As shown in figure 11, the absolute number of CD19+ cells was increased up to 5 fold compared to controls. In addition, there was a 2-4 fold increase in absolute number of CD19+ cells in the spleen as shown in figure 12. In the femoral bone marrow, there was a slight decrease in CD19+ cells (figure 13). Staining for CD45r followed a similar trend. The peripheral blood and spleens isolated from the transgenic mice also exhibited a 2-3 fold increase in the absolute number of helper T cells (CD4+ T lymphocytes). (See figures 14 and 15; respectively)

The transgenic mice had a consistent appearance of a large population of cells (e.g., 33% granulocytes) bearing light scatter properties similar to those of eosinophils (figures 16 and 17). In addition, the cells do not express the granulocytic marker. There was also a consistant appearance of a smaller but distinct population of granulocyte like cells (e.g., 8-17% of granulocytes) that express the IL-17RB in blood and bone marrow. (See figures 18 and 19). Based on correlations with scatter plots, the transgenic mice seem to have the following multi-lineage phenotype: CD4+, CD45R+, CD11c+, and are large and granular.

This analysis indicated that within the transgenic mice there was a clear emergence of an eosinophil-like population in the femoral bone marrow and peripheral blood. As shown in figure 20, the scatter profile of these cells closely resembles a "text-book" example of the forward vs. side scatter (size vs. granularity) properties of eosinophils.

There was also an important increase in the absolute number (and compartmental percentage) of circulating and splenic CD19+ B cells. Although the CD19+ lymphocytes were not positive for the activation marker CD69+, their increase in absolute number in the periphery and slight decrease in the bone marrow is suggestive of migration to peripheral tissues where proliferation is taking place.

The appearance of a multi-lineage phenotype in blood and bone marrow is suggestive of a lymphoma like phenotype. These results are further described in Example 10. Furthermore, since IL-17RB seems to be upregulated on these cells, it is suggestive that this population may be reactive to the omnipresence of IL-17 like protein. Together with the fact that there is clear eosinophilia in these mice, the multi-lineage phenotype closely fits the description of an acute myelomonocytic leukemia (M4 AML) (Campena & Behm, J. Immunol. Meth. 234:59-75, 2000).

## EXAMPLE 6

**Recombinant Human IL-17 like-Fc Fusion Protein:**

An Epogen signal peptide (EpoSP) fused in frame to the predicted mature protein of the human IL-17 like (SEQ ID NO: 2) that was fused in frame to the IgG1 heavy chain constant region (Fc) was engineered to make recombinant mature human IL-17 like protein. The EpoSP DNA encoding for the amino acid sequence MGVHECPAWLWLLLSLLSLPLGLPVLG (SEQ ID NO: 11) was inserted into the pCEP4 expression vector (Invitrogen) in between a consensus Kozak sequence (CCACC) at its 5' end and an AscI site at its 3' end. In addition, the Fc DNA fragment encoding for the amino acid sequence set out in SEQ ID NO: 12 and a NotI restriction site at the 5' end of the sequence was inserted at the 3' end of the EpoSP (SEQ ID NO: 11). A thymidine was inserted immediately after the NotI restriction site in order to keep the coding frame the same. The resulting vector containing the EpoSP and the Fc in pCEP4 is referred to as pCEP4-EpoSP-Fc vector.

A DNA fragment, containing an AscI restriction site at the 5' end and a NotI restriction site at the 3' end, coding for the mature human IL-17 like protein (SEQ ID NO: 2) without the stop codon was generated by PCR. The mature human IL-17 like protein starts at amino acid number 17 (aa17) with the starting methionine as amino acid number one. The AscI site, which contains a thymidine, was inserted immediately before the codon containing residue 17 in order to keep the coding frame the same. The human IL-17 like fragment was directionally ligated into the pCEP4-EpoSP-Fc expression vector using the AscI and NotI restriction sites and was denoted as pCEP4-EpoSP-huIL-17 like-Fc. The integrity of the DNA and the junction sites were confirmed by DNA sequencing using standard methods known in the art.

The pCEP4-EpoSP-huIL-17 like-Fc plasmid was transiently transfected into human 293/EBNA cells using Superfect (Qiagen) according to the manufacturer's instructions. The serum-free conditioned media was harvested from the cells 72 hours after transfection. The recombinant human IL-17 like-Fc fusion protein, predicted to have the amino acid sequence APS located at the amino-terminus of the mature protein, was isolated by affinity chromatography using a HiTrap Protein G column (Amersham Pharmacia). The recombinant human IL-17 like-Fc fusion protein was then dialyzed against PBS buffer for 72 hours at 4°C using Spectra/Pore Membrane MWCO 10,000 (Spectrum Laboratories). Subsequently, the recombinant human IL-17 like-Fc fusion protein was electrophoresed on a 10% acrylamide gel (Novex) and stained with Coomassie-Blue. The stained gel was scanned with a denstitometer to determine the percent representation of the protein band of interest. Modified Lowry Protein Assay Reagent (Pierce) was used to determine the total protein concentration according to the manufacturer's instructions. Then, the amount of human IL-17 like-Fc fusion protein was calculated by multiplying the percentage of IL-17 like-Fc fusion protein by the total protein concentration.

## EXAMPLE 7

**Recombinant Human IL-17 Receptor B-Fc Fusion Protein:**

IL-17 receptor-B polypeptides were cloned as described in U.S. Patent Application serial no. 09/723,232 filed November 27, 2000, the disclosure of which is incorporated herein by reference in its entirety. To prepare IL-17 receptor B-Fc fusion proteins (IL-17RB-Fc), the extra-cellular domain of the human IL-17 receptor like polypeptide (amino acid #1-292 for IL-17RB-2, amino acid #1-350 for IL-17RB-3 of SEQ ID NOS: 18 and 20, respectively) was fused to the human IgG1 heavy chain constant region (Fc). The DNA fragment encoding the human Fc (amino acid sequence set out in SEQ ID NO: 12) with a NotI restriction site at its 5' end and XhoI restriction site at its 3' end were directionally ligated into pCEP4 vector using NotI and XhoI sites. The resulting vector containing the Fc coding sequence in pCEP4 is referred to as pCEP4-Fc vector. DNA fragments encoding the extra-cellular domain of the human IL-17RB-2 or IL-17RB-3 (SEQ ID NOS: 18 and 20 respectively), with an Hind III restriction site and kozak sequence (CCACC) at their 5' end and a NotI restriction site at their 3' end, were generated by PCR. These DNA fragments were directionally ligated into the pCEP4-Fc expression vector using the Hind III and NotI restriction sites and were denoted as pCEP4-huIL-17RB-2 like-Fc or pCEP4-huIL-17RB-3 like-Fc. The integrity of the DNA and the junction sites were confirmed by DNA sequencing using standard methods known in the art.

The pCEP4-huIL-17RB-2 like-Fc plasmid or pCEP4-huIL-17RB-3 like-Fc plasmid (also denoted HIL-17RB-2-Fc and HIL17RB-3-Fc, respectively, and deposited on March 14, 2001 with the American Type Culture Collection, 10801 University Blvd., Manassas, VA 20110, U.S.A. under Accession Nos. _____ and ______, respectively) were transiently transfected into human 293/EBNA cells using Superfect (Qiagen) according to the manufacturer's instructions. The serum-free conditioned media was harvested from the cells 72 hours after transfection. The recombinant human IL-17RB like-Fc fusion proteins, predicted to have the amino acid sequence APS located at the amino-terminus of the mature protein, were isolated by affinity chromatography using a HiTrap Protein G column (Amersham Pharmacia). The amino acid sequences of the resulting fusion proteins are set out in as SEQ ID NOS: 21 and 22.

The recombinant human IL-17RB like-Fc fusion proteins were dialyzed against PBS buffer for 72 hours at 4°C using Spectra/Pore Membrane MWCO 10,000 (Spectrum Laboratories). Subsequently, the recombinant human IL-17RB like-Fc fusion proteins were electrophoresed on a 10% acrylamide gel (Novex) and stained with Coomassie-Blue. The stained gel was scanned with a denstitometer to determine the percent representation of the protein band of interest. Modified Lowry Protein Assay Reagent (Pierce) was used to determine the total protein concentration according to the manufacturer's instructions. Then, the amount of human IL-17 receptor like-Fc fusion protein were calculated by multiplying the percentage of IL-17RB like-Fc fusion proteins by the total protein concentration.

The IL-17RB fusion proteins can also be generated with an Epogen signal peptide (MGVHECPAWLWLLLSLLSLPLGLPVLG (SEQ ID NO: 11) fused in frame into the predicted mature protein instead of fusing to the native extra-cellular domain as described above.

## EXAMPLE 8

### IL-17 like Polypeptide Binds to the IL-17 receptor B

To determine if IL-17 like polypeptide is a ligand for the IL-17 receptor B (IL-17RB) polypeptides (SEQ ID NOS: 18 and 20), competitive binding assays were performed with the human B-lymphoblast cell line GM3104A which has been shown to express IL-17RB by Northern blot and RT-PCR analyses. The conditioned media from 293E cells transfected to express IL-17 like-Fc fusion protein (described above in Example 6) was collected, concentrated and used for the binding assay. Specificity of ligand binding was determined by competition with soluble blocking receptors, either IL-17RB-2 or IL-17RB-3. IL-17R-Fc fusion protein (containing the extracellular portion of IL-17 receptor) was purified from conditioned media collected from transfected 293E cells. Conditioned media from 293E cells transfected with IL-17RB-2-Fc or IL-17-RB-3-Fc (deposited with the ATCC on March 14, 2001 under Accession Nos. ____ and ____ respectively) as described above in Example 7 was concentrated (5x) with an Amicon 3Kd cut-off Centracon (#4203) and also used for blocking.

Prior to the binding assay, 0.5 ml of IL-17 like-Fc fusion protein containing (1x) conditioned media was added into vials each containing 0.5 ml 5x conditioned media of IL-17RB-2-Fc, IL-17RB-3-Fc, or 0.5 ml of 5 μg/ml IL-17R-Fc protein in RPMI 1640. Each vial was incubated on ice for 2 hours in order to pre-block non-specific binding sites.

Subsequently, GM3104A cells ($1x10^6$ cells per sample) were incubated with 1 ml of 8% FBS/PBS, at 4°C for 1 hour. The cells were then washed with 0.5% BSA/PBS and incubated with 1 ml of control conditioned media, conditioned media containing IL-17 like-Fc or conditioned media supplemented with blocking receptor (IL-17RB-2 or IL-17RB-3) for 2 hours at 4°C with gentle shaking. After the incubation, the cells were washed 3 times with 1 ml of ice-cold 0.5% BSA/PBS.

Each cell sample was stained with 2μg/100μl goat anti human IgG-Fc-FITC (Chemicon, AP113F)diluted in 0.5% BSA/PBS. The cells were incubated on ice for 1 hour and washed 3 times with 1 ml of ice-cold 0.5%BSA/PBS. Subsequently, ligand binding was detected with Fluorescence-activated cell sorter analysis using FACScan (Becton Dickinson). This analysis indicated that IL-17 like-FC fusion protein bound to GM3104A cells. This binding was inhibited by IL-17RB-2 and IL-17RB-3 but not IL-17 R.

## EXAMPLE 9

### IL-17 like Polypeptide Induces Expression of Proinflammatory Cytokines

The conditioned media from 293E cells expressing either IL-17 like-Fc fusion protein, IL-17B-Fc, IL-17C-Fc or IL-17C-Fc, was collected to use as ligand in the assay. Conditioned media containing IL-17C-Fc, IL-17D-Fc, and IL-17 like-Fc were then concentrated (15x) using a 3Kd cut-off Centracon(Amicon, #4032), and reconstituted to 1x medium by adding fresh 20% FBS/1640 media.

Human T-lymphoblast cells (GM3104A, 1x$10^6$ cell/sample) were cultured in reconstituted concentrated condition media which contained each IL-17 ligand (IL-17 like polypeptide, IL-17B, IL-17 C, IL-17D and human Fc). After incubation for 18 hours at 37°C and 5% $CO_2$, the media were collected and the amount of IL-1$\alpha$, IL-1$\beta$, IL-6, IFN-$\gamma$, G-CSF, and TNF-$\alpha$ released into the media was measured with the appropriate Quantikine Immunoassay kit (R&D Systems) following the manufacturer's instructions. The results are summarized in table 4. IL-17 like-FC fusion protein induced the release of TNF-$\alpha$, IL-1$\alpha$, and IL-6 to a much greater extent that the other IL-17 ligands tested. Induction of IL-1$\beta$, IFN-$\gamma$, and G-CSF was not detected for any of the ligands.

**Table 4**

| Ligand | TNF-$\alpha$ (pg/ml) | IL-1$\alpha$ (pg/ml) | IL-6 (pg/ml) |
|---|---|---|---|
| Mock CM | 190 | 6 | 157.6 |
| Human Fc | 210 | 8 | 199 |
| IL-17B | 180 | 11 | 138 |
| IL-17C | 170 | 8 | 152 |
| IL-17D | 180 | 22 | 155 |
| IL-17 like | 460 | 25 | 362 |

**EXAMPLE 10**

**Immunophenotying the F1 Generation of IL-17 like Polypeptide Overexpressing Transgenic Mice**

The immunophenotype of the IL-17 like polypeptide overexpressing transgenic mice was analyzed using FACS analysis. The populations of CD5 on CD19+ lymphocytes and CD34 on CD19+ lymphocytes in the lymph nodes of non-transgenic control and transgenic mice were measured. In addition, CD4 expression on eosinophils in the bone marrow of non-transgenic and transgenic mice was also measured.

The profiles of CD5, CD34, and CD4 expression on cells from the specified lymphoid tissues isolated from IL-17 like polypeptide overexpressing transgenic mice(8-10 weeks) and non-trangenic controls, FACS analysis was carried out on cell suspensions as described in Example 4. Cells (1x$10^6$) were incubated with 1 $\mu$g/$10^6$ cells of conjugated antibodies against the follow mouse surface markers: CD5-FITC, CD34-FITC, CD19-PE, and CD4-Cychrome. All antibodies were obtained from BD-Pharmingen (San Diego, CA). Staining procedures were performed as previously described (See Example 4) and read on a FACScan (Beckman). Marker expression level was measured on either lymphocytes or eosinophils(Eos)gates on scatter plots. (See figure 21) Percentages included refer to double positive populations.

Absolute numbers of cells for CD5+/CD19+, CD34+/CD19+, and CD4+Eos. populations are represented in Table 5. To measure the lymphocytes, the FACS was gated for lymphocytes and the data is shown as percent of absolute number of lymphocytes. To measure eosinophils the FACS was gated for all cell types and therefore the data is shown as percent of total number of cells.

**TABLE 5**

| Lymphocytes | Percent of Absolute Number of Lymphocytes Non-Transgenic | Trangenic | Fold Increase |
|---|---|---|---|
| CD5+CD19+Lymph Node | 0.97% | 43.11% | >100-fold increase |
| CD34+CD19+Lymph Node | 1.39% | 46.49% | >90-fold increase |
| **Eosinophils** | **Percent of Total Cells Non-Transgenic** | **Trangenic** | **Fold Increase** |
| CD4+Eos Bone Marrow | 0.67% | 15.14% | >50-fold increase |

As shown in Table 5 above, the F1 IL-17 like polypeptide overexpressing transgenic mice revealed lymphocytic and eosinophilic populations expressing hematopoietic markers that have been identified in numerous leukemias. Specifically, CD19+ lymphocytes in the lymph node from the transgenic mice expressed CD5 and CD34 markers which are not expressed in the control mice. Both CD5 and CD34 expression is at approximately 100-fold increase over the controls (Table 5). Upregulation of CD5 and CD19 has been identified in B-cell chronic leukemia (B-CLL), while upregulation of CD34 has been reported for acute myeloid leukemia (AML.) *(See,* Xia et al., Cytometry 42: 114-7, 2000; Caldwell and Lascombe, Evaluation of Peripheral Blood Lymphocytosis, Acedemic Information Systems, Inc., 2000; Neuber et al., Dermatology 192: 110-5, 1996).

Also, CD4 expression was found on eosinophils in the bone marrow in the transgenic mice, at 15% increased over the controls, which resulted in a 50-fold increase in absolute numbers of CD4-expressing eosinophils. This aberrrant CD4 expression on eosinophils has been reported in adult T cell leukemia patients, with higher CD4+ and HLA-DR+ eosinophils than control groups (Sakamoto et al., Intl. Archives of Allergy and Immunology. 111 (Suppl.1) : 26-8, 1996). From these expression patterns, it appears that the lymph node in the F1 generation of IL-17 like polypeptide overexpressing transgenic mice bear early symptoms of pre-leukemic conditions. These mice may deteriorate in health.as they age, developing any of these possible leukemias which may be in early stages in these mice.

These results suggest IL-17 like polypeptides and polynucleotides may be useful in the diagnosis, treatment and prevention of lymphomas including non-hodgkin's lymphoma and Hodgkin's Disease; acute myelogenous leukemias (AML and CML) including premyelocytic leukemia (M3 AML), myelomonocytic leukemia (M4 AML), erythroleukemia (M6 AML) and megakaryocytic leukemia(M7 AML); acute lymphocytic leukemia including acute lymphoblastic leukemia; chronic lymphocytic leukemia; hairy cell leukemia; and multiple myeloma.

## Claims

1. Use of an antagonistic antibody specific for a polypeptide consisting of the amino acid sequence of SEQ ID NO: 2, or a fragment thereof comprising 25, 50, 75, 100 or 150 contiguous amino acids, for preparation of a medicament for treating cystic fibrosis, acute respiratory disease syndrome or emphysema.

2. An antagonistic antibody specific for a polypeptide consisting of the amino acid sequence of SEQ ID NO: 2, or a fragment thereof comprising 25, 50, 75, 100 or 150 contiguous amino acids, for use in the treatment of cystic fibrosis, acute respiratory disease syndrome or emphysema.

3. Use of a pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for preparation of a medicament for treating cystic fibrosis, acute respiratory disease syndrome or emphysema.

4. A pharmaceutical composition comprising an antagonistic antibody specific for a polypeptide consisting of the amino acid sequence of SEQ ID NO: 2, or a fragment thereof comprising 25, 50, 75, 100 or 150 contiguous amino acids, and a pharmaceutically acceptable formulation agent, for use in the treatment of cystic fibrosis, acute respiratory disease syndrome or emphysema.

## Patentansprüche

1. Verwendung eines antagonistischen Antikörpers mit Spezifität für ein Polypeptid, bestehend aus der Aminosäuresequenz der SEQ ID NO: 2, oder ein Fragment davon, umfassend 25, 50, 75, 100 oder 150 zusammenhängende Aminosäuren, zur Herstellung eines Medikaments zum Behandeln von Mukoviszidose, akutem Atemwegserkrankungssyndrom oder Emphysem.

2. Antagonistischer Antikörper mit Spezifität für ein Polypeptid, bestehend aus der Aminosäuresequenz der SEQ ID NO: 2, oder ein Fragment davon, umfassend 25, 50, 75, 100 oder 150 zusammenhängende Aminosäuren, zur Verwendung bei der Behandlung von Mukoviszidose, akutem Atemwegserkrankungssyndrom oder Emphysem.

3. Verwendung einer pharmazeutischen Zusammensetzung, umfassend einen antagonistischen Antikörper mit Spezifität für ein Polypeptid, bestehend aus der Aminosäuresequenz der SEQ ID NO: 2, oder ein Fragment davon, umfassend 25, 50, 75, 100 oder 150 zusammenhängende Aminosäuren, und ein pharmazeutisch verträgliches Formulierungsmittel, zur Herstellung eines Medikaments zum Behandeln von Mukoviszidose, akutem Atemwegserkrankungssyndrom oder Emphysem.

4. Pharmazeutische Zusammensetzung, umfassend einen antagonistischen Antikörper mit Spezifität für ein Polypeptid, bestehend aus der Aminosäuresequenz der SEQ ID NO: 2, oder ein Fragment davon, umfassend 25, 50, 75, 100 oder 150 zusammenhängende Aminosäuren, und ein pharmazeutisch verträgliches Formulierungsmittel, zur Verwendung bei der Behandlung von Mukoviszidose, akutem Atemwegserkrankungssyndrom oder Emphysem.

**Revendications**

1. Utilisation d'un anticorps antagoniste spécifique d'un polypeptide constitué de la séquence d'acides aminés de SEQ ID NO: 2, ou d'un de ses fragments comprenant 25, 50, 75, 100 ou 150 acides aminés contigus, pour la préparation d'un médicament destiné au traitement de la fibrose kystique, du syndrome de détresse respiratoire aiguë ou de l'emphysème.

2. Anticorps antagoniste spécifique d'un polypeptide constitué de la séquence d'acides aminés de SEQ ID NO: 2, ou d'un de ses fragments comprenant 25, 50, 75, 100 ou 150 acides aminés contigus, destiné à être utilisé dans le traitement de la fibrose kystique, du syndrome de détresse respiratoire aiguë ou de l'emphysème.

3. Utilisation d'une composition pharmaceutique comprenant un anticorps antagoniste spécifique d'un polypeptide constitué de la séquence d'acides aminés de SEQ ID NO: 2, ou d'un de ses fragments comprenant 25, 50, 75, 100 ou 150 acides aminés contigus, et un agent de formulation pharmaceutiquement acceptable, pour la préparation d'un médicament destiné au traitement de la fibrose kystique, du syndrome de détresse respiratoire aiguë ou de l'emphysème.

4. Composition pharmaceutique comprenant un anticorps antagoniste spécifique d'un polypeptide constitué de la séquence d'acides aminés de SEQ ID NO: 2, ou d'un de ses fragments comprenant 25, 50, 75, 100 ou 150 acides aminés contigus, et un agent de formulation pharmaceutiquement acceptable, destinée à être utilisée dans le traitement de la fibrose kystique, du syndrome de détresse respiratoire aiguë ou de l'emphysème.

Figure 1

Map of Human IL-17 Like cDNA (SEQ ID NO:1) and
Amino Acid (SEQ ID NO:2) Sequences

1 CTCAAGTCACTCCCTAAAAAGACAGTGGAAATAAATTTGAATAAACAAAACAGGCTTGCT
61 GAAAATAAAATCAGGACTCCTAACCTGCTCCAGTCAGCCTGCTTCCACGAGGCCTGTCAG
121 TCAGTGCCCCACTTGTGACTGAGTGTGCAGTGCCCAGCATGTACCAGGTGGTTGCATTCT
1 **M** Y Q V V A F L 8
181 TGGCAATGGTCATGGGAACCCACACCTACAGCCACTGGCCCAGCTGCTGCCCCAGCAAAG
9 A M V M G T H T Y S H W P S C C P S K G 28
241 GGCAGGACACCTCTGAGGAGCTGCTGAGGTGGAGCACTGTGCCTGTGCCTCCCCTAGAGC
29 Q D T S E E L L R W S T V P V P P L E P 48
301 CTGCTAGGCCCAACCGCCACCCAGAGTCCTGTAGGGCCAGTGAAGATGGACCCCTCAACA
49 A R P N R H P E S C R A S E D G P L N S 68
361 GCAGGGCCATCTCCCCCTGGAGATATGAGTTGGACAGAGACTTGAACCGGCTCCCCCAGG
69 R A I S P W R Y E L D R D L N R L P Q D 88
421 ACCTGTACCACGCCCGTTGCCTGTGCCCGCACTGCGTCAGCCTACAGACAGGCTCCCACA
89 L Y H A R C L C P H C V S L Q T G S H M 108
481 TGGACCCCCGGGGCAACTCGGAGCTGCTCTACCACAACCAGACTGTCTTCTACCGGCGGC
109 D P R G N S E L L Y H N Q T V F Y R R P 128
541 CATGCCATGGCGAGAAGGGCACCCACAAGGGCTACTGCCTGGAGCGCAGGCTGTACCGTG
129 C H G E K G T H K G Y C L E R R L Y R V 148
601 TTTCCTTAGCTTGTGTGTGTGTGCGGCCCCGTGTGATGGGCTAG 643
149 S L A C V C V R P R V M G * 162

Figure 2A

(Map of Mouse IL-17 Like cDNA (SEQ ID NO:3) and Amino Acid (SEQ ID NO:4) Sequences with Predicted Signal Peptide

```
1     ATGTACCAGGCTGTTGCATTCTTGGCAATGATCGTGGGAACCCACACCGTCAGCTTG
       M  Y  Q  A  V  A  F  L  A  M  I  V  G  T  H  T  V  S  L    19
58    CGGATCCAGGAGGGCTGCAGTCACTTGCCCAGCTGCTGCCCCAGCAAAGAGCAAGAACCC
11    R  I  Q  E  G  C  S  H  L  P  S  C  C  P  S  K  E  Q  E  P  39
118   CCGGAGGAGTGGCTGAAGTGGAGCTCTGCATCTGTGTCCCCCCCAGAGCCTCTGAGCCAC
31    P  E  E  W  L  K  W  S  S  A  S  V  S  P  P  E  P  L  S  H  59
178   ACCCACCACGCAGAATCCTGCAGGGCCAGCAAGGATGGCCCCCTCAACAGCAGGGCCATC
51    T  H  H  A  E  S  C  R  A  S  K  D  G  P  L  N  S  R  A  I  79
238   TCTCCTTGGAGCTATGAGTTGGACAGGGACTTGAATCGGGTCCCCCAGGACCTGTACCAC
71    S  P  W  S  Y  E  L  D  R  D  L  N  R  V  P  Q  D  L  Y  H  99
298   GCTCGATGCCTGTGCCCACACTGCGTCAGCCTACAGACAGGCTCCCACATGGACCCGCTG
91    A  R  C  L  C  P  H  C  V  S  L  Q  T  G  S  H  M  D  P  L  119
358   GGCAACTCCGTCCCACTTTACCACAACCAGACGGTCTTCTACCGGCGGCCATGCCATGGC
111   G  N  S  V  P  L  Y  H  N  Q  T  V  F  Y  R  R  P  C  H  G  139
418   GAGGAAGGTACCCATCGCCGCTACTGCTTGGAGCGCAGGCTCTACCGAGTCTCCTTGGCT
131   E  E  G  T  H  R  R  Y  C  L  E  R  R  L  Y  R  V  S  L  A  159
478   TGTGTGTGTGTGCGGCCCCGGGTCATGGCTTAGTCATGCTCACCACCTGCCTGAGGCTGA
160   C  V  C  V  R  P  R  V  M  A  *                                170
538   TGCCCGGTTGGGAGAGAGGGCCAGGTGTACAATCACCTTGCCAATGCGGGCCGGGTTCAA
598   GCCCTCCAAAGCCCTACCTGAAGCAGCAGGCTCCCGGGACAAGATGGAGGACTTGGGGAG
658   AAACTCTGACTTTTGCACTTTTTGGAAGCACTTTTGGGAAGGAGCAGGTTCCGCTTGTGC
```

Figure 2B

```
718   TGCTAGAGGATGCTGTTGTGGCATTTCTACTCAGGAACGGACTCCAAAGGCCTGCTGACC
778   CTGGAAGCCATACTCCTGGCTCCTTTCCCCTGAATCCCCCAACTCCTGGCACAGGCACTT
838   TCTCCACCTCTCCCCCTTTGCCTTTTGTTGTGTTTGTTTGTGCATGCCAACTCTGCGTGC
898   AGCCAGGTGTAATTGCCTTGAAGGATGGTTCTGAGGTGAAAGCTGTTATCGAAAGTGAAG
958   AGATTTATCCAAATAAACATCTGTGTTTAAAAAAAAAAAAAAAAAAAAAAAAAAAAA 1496
```

Map of Non-Secreted Form of Mouse IL-17 Like cDNA (SEQ ID NO:9), and Corresponding Amino Acid Sequence (SEQ ID NO:10)

```
  1   CCGGGCAGGTGCCCTCGGCGCGTCCCAAAGCTTAGGGAAGCTCCAGGTGTCTTGGGAAAT
 61   GAAGAAAAAGGCCACCGAGCAAAAAGGAACAGAGAAGGGGAGGAGCAGTGCTGTGGGCTC
121   GCCTAGGGTCGAGGGCCATTATCACCTACAAATCAGAATGTGGGAGTGCTATTCTAGAGG
181   TCTCCATCTTTGCCATTGCTGGGTCGCTCAGAAAAGTGTGATGGGGTTGTCCCATTGCCA
241   AGAACAGCTTCTGCTTACCAGCAGGTGCTGACCTCTTTCCCCAGAGGCACAGGGAAGGAA
301   TTCCAGCCCCGGTTGGCTGCCAGAGGCTTCCTCTGGCGTTGGGTACAGAGGCAGAGAAAG
361   AAACCCCAAATGTCTCCTATGAAAAACAATGTCCCCGTCATCCAGGCCAGATCATTCTGC
421   AGTGTCAACAGTTGAGACAAGAAGCTGGGGTCATTTTCTGTGCCTAAGAGTGCCTGTTCT
481   GCACTGGCCAAGCTGTTGCATTCTTGGCAATGATCGTGGGAACCCACACCGTCAGCTTG
                                                M  I  V  G  T  H  T  V  S  L     10
541   CGGATCCAGGAGGGCTGCAGTCACTTGCCCAGCTGCTGCCCCAGCAAAGAGCAAGAACCC
 11   R  I  Q  E  G  C  S  H  L  P  S  C  C  P  S  K  E  Q  E  P    30
```

Figure 2C

```
601   CCGGAGGAGTGGCTGAAGTGGAGCTCTGCATCTGTGTCCCCCCAGAGCCTCTGAGCCAC
 31   P  E  E  W  L  K  W  S  S  A  S  V  S  P  P  E  P  L  S  H     50
661   ACCCACCACGCAGAATCCTGCAGGGCCAGCAAGGATGGCCCCCTCAACAGCAGGGCCATC
 51   T  H  H  A  E  S  C  R  A  S  K  D  G  P  L  N  S  R  A  I     70
721   TCTCCTTGGAGCTATGAGTTGGACAGGGACTTGAATCGGGTCCCCCAGGACCTGTACCAC
 71   S  P  W  S  Y  E  L  D  R  D  L  N  R  V  P  Q  D  L  Y  H     90
781   GCTCGATGCCTGTGCCCACACTGCGTCAGCCTACAGACAGGCTCCCACATGGACCCGCTG
 91   A  R  C  L  C  P  H  C  V  S  L  Q  T  G  S  H  M  D  P  L     110
841   GGCAACTCCGTCCCACTTTACCACAACCAGACGGTCTTCTACCGGCGGCCATGCCATGGC
111   G  N  S  V  P  L  Y  H  N  Q  T  V  F  Y  R  R  P  C  H  G     130
901   GAGGAAGGTACCCATCGCCGCTACTGCTTGGAGCGCAGGCTCTACCGAGTCTCCTTGGCT
131   E  E  G  T  H  R  R  Y  C  L  E  R  R  L  Y  R  V  S  L  A     150
961   TGTGTGTGTGTGCGGCCCCGGGTCATGGCTTAGTCATGCTCACCACCTGCCTGAGGCTGA
151   C  V  C  V  R  P  R  V  M  A  *                                161
1021  TGCCCGGTTGGGAGAGAGGGCCAGGTGTACAATCACCTTGCCAATGCGGGCCGGGTTCAA
1081  GCCCTCCAAAGCCCTACCTGAAGCAGCAGGCTCCCGGGACAAGATGGAGGACTTGGGGAG
1141  AAACTCTGACTTTTGCACTTTTTGGAAGCACTTTTGGGAAGGAGCAGGTTCCGCTTGTGC
1201  TGCTAGAGGATGCTGTTGTGGCATTTCTACTCAGGAACGGACTCCAAAGGCCTGCTGACC
1261  CTGGAAGCCATACTCCTGGCTCCTTTCCCCTGAATCCCCCAACTCCTGGCACAGGCACTT
1321  TCTCCACCTCTCCCCCTTTGCCTTTTGTTGTGTTTGTTTGTGCATGCCAACTCTGCGTGC
1381  AGCCAGGTGTAATTGCCTTGAAGGATGGTTCTGAGGTGAAAGCTGTTATCGAAAGTGAAG
1441  AGATTTATCCAAATAAACATCTGTGTTTAAAAAAAAAAAAAAAAAAAAAAAAAAAAA  1496
```

Figure 3A

Pile-Up of Human IL-17 Like Amino Acid Sequence, hIL-17L (SEQ ID NO:2), with a Known Human IL-17 Family Member Amino Acid Sequence, hIL-17 (SEQ ID NO:5)

Smith-Waterman score: 155;   25.0% identity in 160 AA overlap

```
                  10        20        30        40        50        60
hIL-17L   XNQDSXPAPVSLLPRGLSVSAPLVTECAVPSMYQVVAFLAMVMGTHTYSHWPSCCPSKGQ
                                          |:  :::: |:| :  |  : |:|  |: :
hIL-17                         MTPGKTSLVSLLLLLSLEAIVKAGITIPRNPGCPNSEDK
                                         10        20        30

                  70        80        90       100       110       120
hIL-17L   DTSEELLRWSTVPVPPLEPARPNRHPESCRASEDGPLNSRAISPWRYELDRDLNRLPQDL
          :  : ::   ::     :    | :|:  |:|:     :|: |||  : ::| :| |: :
hIL-17    NFPRTVMVNLNI-----HNRNTNTNPK--RSSD---YYNRSTSPWNLHRNEDPERYPSVI
          40       50             60             70        80
```

Figure 3B

```
                      130       140  .      150       160       170       180
hIL-17L     YHARCLCPHCVSLQTGSHMDPLGNSVPLYHNQTVFYRRPCHGEEGTHRRYCLERRLYRVS
            ::|:|    |::  : :::|   ||||: ::  |: |:| |  ::    : ||: |  ||
hIL-17      WEAKCRHLGCIN--ADGNVDYHMNSVPIQQEILVLRREPPHCPNS----FRLEKIL--VS
            90       100         110       120       130            140

                 190
hIL-17L     LACVCVRPRVMA
            ::|:|| | |
hIL-17      VGCTCVTPIVHHVA
                 150
```

Figure 4

Pile-Up of Human IL-17 like Amino Acid Sequence, hIL-17L, (SEQ ID NO:2) with a Known Human IL-20 Amino Acid Sequence, hIL-20 (SEQ ID NO:6)

SÇORES Init1: 124 Initn: 124 Opt: 175
Smith-Waterman score: 175; 36.7% identity in 90 aa overlap

```
                70        80        90        100       110       120
hIL-17L         RWSTVPVPPLEPARPNRHPESCRASEDGPLNSRAISPWRYELDRDLNRLPQDLYHARCLC
                                              |:|::||| | :::| :|:| || :|||||
hIL-20          RNIEEMVAQLRNSSELAQRKCEVNLQLWMSNKRSLSPWGYSINHDPSRIPVDLPEARCLC
                      70        80        90        100       110       120

                130       140       150       160       170       180
hIL-17L         PHCVSLQTGSHMDPLGNSVPLYHNQTVFYRRPCHGEEGTHRRYCLERRLYRVSLA-CVCV
                  ||:  | :: |    |||:: :|:   || |     |    | :| :::: :| |:|:
hIL-20          LGCVNPFTMQE-DRSMVSVPVF-SQVPVRRRLCPPPPRTGP--CRQRAVMETIVAGCTCI
                    130        140        150       160         170

                190
hIL-17L         RPRVMA

hIL-20          F
```

Figure 5

Pile-Up of Human IL-17 Like Amino Acid Sequence, hIL-17L, (SEQ ID NO:2) with a Known Human IL-17 Amino Acid Sequence, hIL-17b (SEQ ID NO:7) Family Member

```
SCORES   Init1: 124   Initn: 124   Opt: 178
Smith-Waterman score: 178;    35.6% identity in 90 aa overlap

            70        80        90       100        110      120
hIL-17L     RWSTVPVPPLEPARPNRHPESCRASEDGPLNSRAISPWRYELDRDLNRLPQDLYHARCLC
                                          |:|::||| | :::| :|:| || :|||||
hIL-17b     RNIEEMVAQLRNSSELAQRKCEVNLQLWMSNKRSLSPWGYSINHDPSRIPVDLPEARCLC
                 70        80        90       100       110       120

            130       140       150       160       170        180
hIL-17L     PHCVSLQTGSHMDPLGNSVPLYHNQTVFYRRPCHGEEGTHRRYCLERRLYR-VSLACVCV
              ||:  | :: |    |||:: :|:   || |     |    | :|:::  ::::|:|:
hIL-17b     LGCVNPFTMQE-DRSMVSVPVF-SQVPVRRRLCPPPPRTGP--CRQRAVMETIAVGCTCI
                130       140       150       160        170

            190
hIL-17L     RPRVMA

hIL-17b     F
            180
```

Figure 6A

Pile-Up of Human IL-17 Like Amino Acid Sequence, hIL-17L, (SEQ ID NO:2) with Amino Acid Sequence of a Known Human IL-17 Family Member, hIL-17c (SEQ ID NO:8)

SCORES   Init1: 149   Initn: 194   Opt: 236

Smith-Waterman score: 243;   34.5% identity in 171 aa overlap

```
             20        30        40        50             60       69
hIL-17L    GLSVSAPLVTECAVPSMYQVVAFLAMVMGTHTYSHW-PSCCPSK----GQDTSEELLR--
                                          | :||  | |  ::    ||   : | |
hIL-17c        MTLLPGLLFLTWLHTCLAHHDPSLRGHPHSHGTPHCYSAEELPLGQAPPHLLARGA
                       10        20        30        40        50

           70            80              90            100       110
hIL-17L    -WS-TVPVP---PLEPARP-NRH--PES---C---RASE--DGPLNSRAISPWRYELDRD
            |: ::||     || |   :||  | :   |   |  |  ::  ::|:||||||::| |
hIL-17c    KWGQALPVALVSSLEAASHRGRHERPSATTQCPVLRPEEVLEADTHQRSISPWRYRVDTD
             60        70        80        90       100       110
```

Figure 6B

```
               120       130       140       150       160          169
hIL-17L     LNRLPQDLYHARCLCPHCVSLQTGSHMDPLGNSVPLYHNQTVFYRRPCHGEEG----THR
             :| || |  |:|||  |:: :|| :   | ||| | ::  |: |||| :::|    |
hIL-17c     EDRYPQKLAFAECLCRGCIDARTGRETAAL-NSVRLLQSLLVLRRRPC-SRDGSGLPTPG
             120       130       140        150       160        170

            170       180       190
hIL-17L     RYCLERRLYRVSLACVCVRPRVMA
             : :: :: :| ::|:|| ||
hIL-17c     AFAFHTEFIHVPVGCTCVLPRSV
               180       190
```

Figure 7

Northern Blot Expression Analysis of TH00-018
Necropsied Transgenic Founders

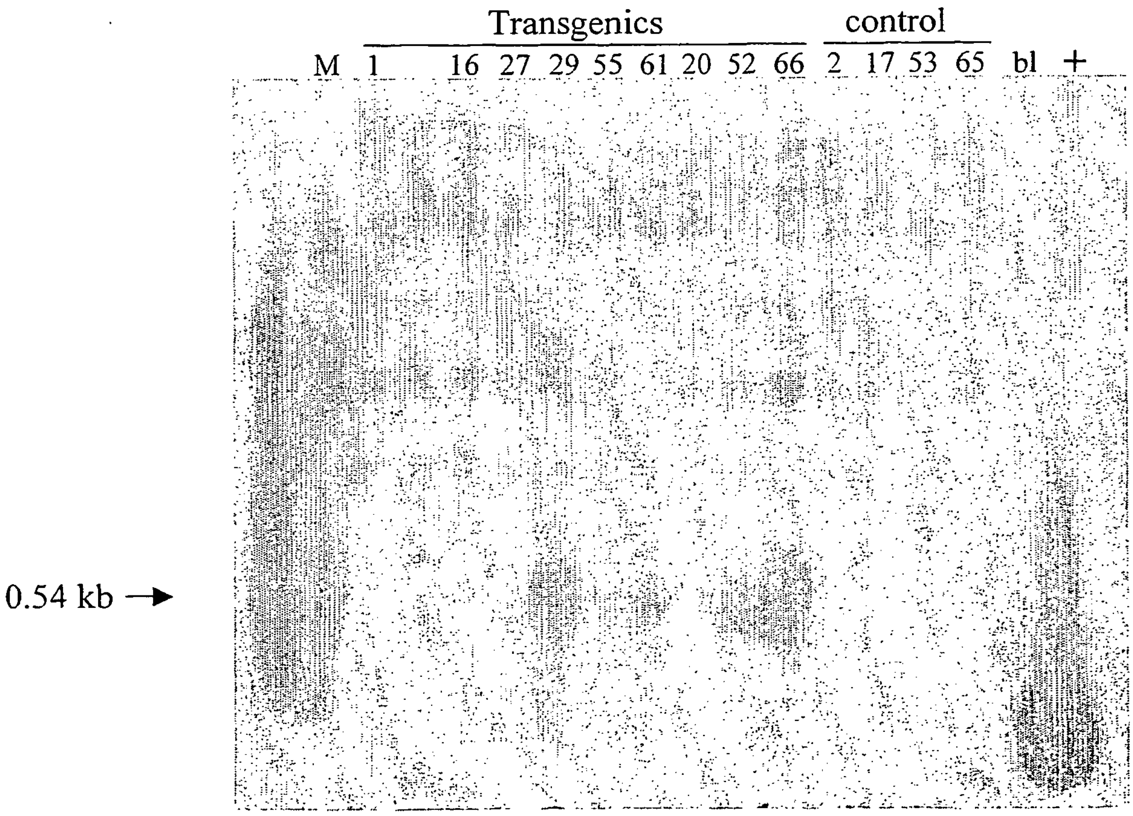

Figure 8

Northern Blot Expression Analysis of TH00-018 Hepatectomized Transgenic Founders

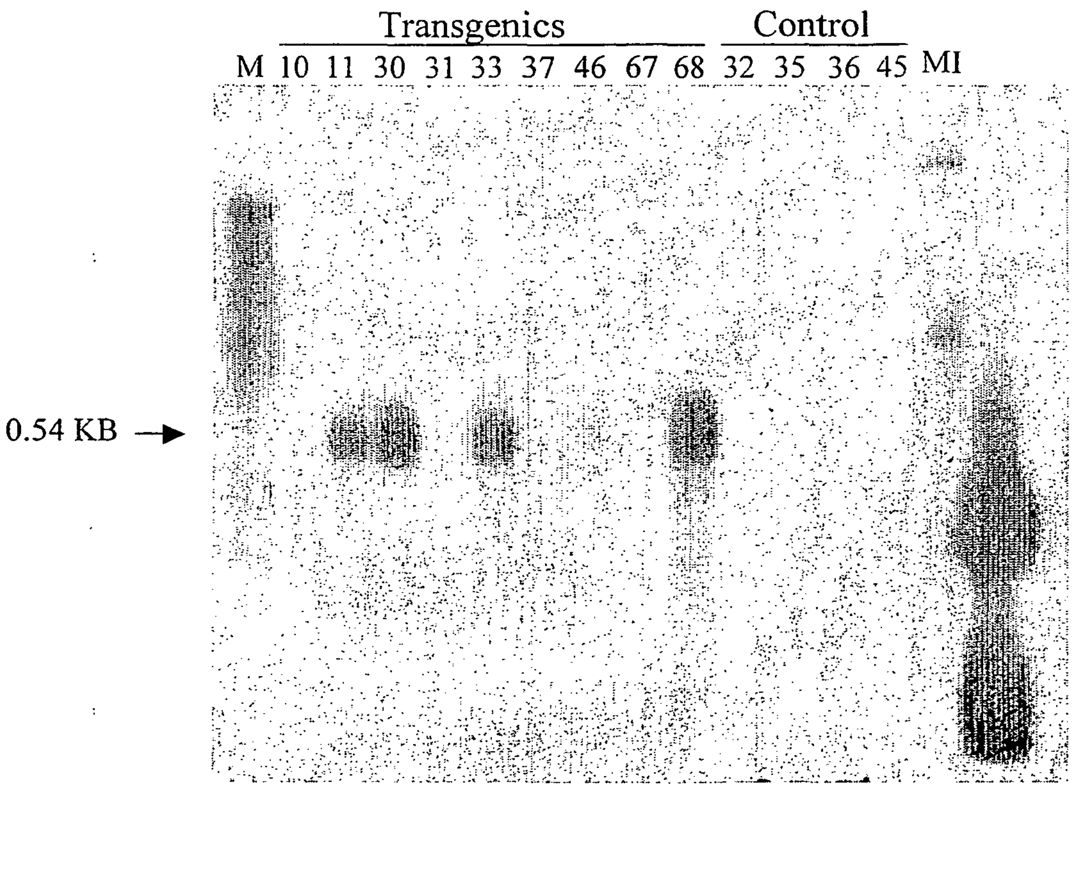

Figure 9

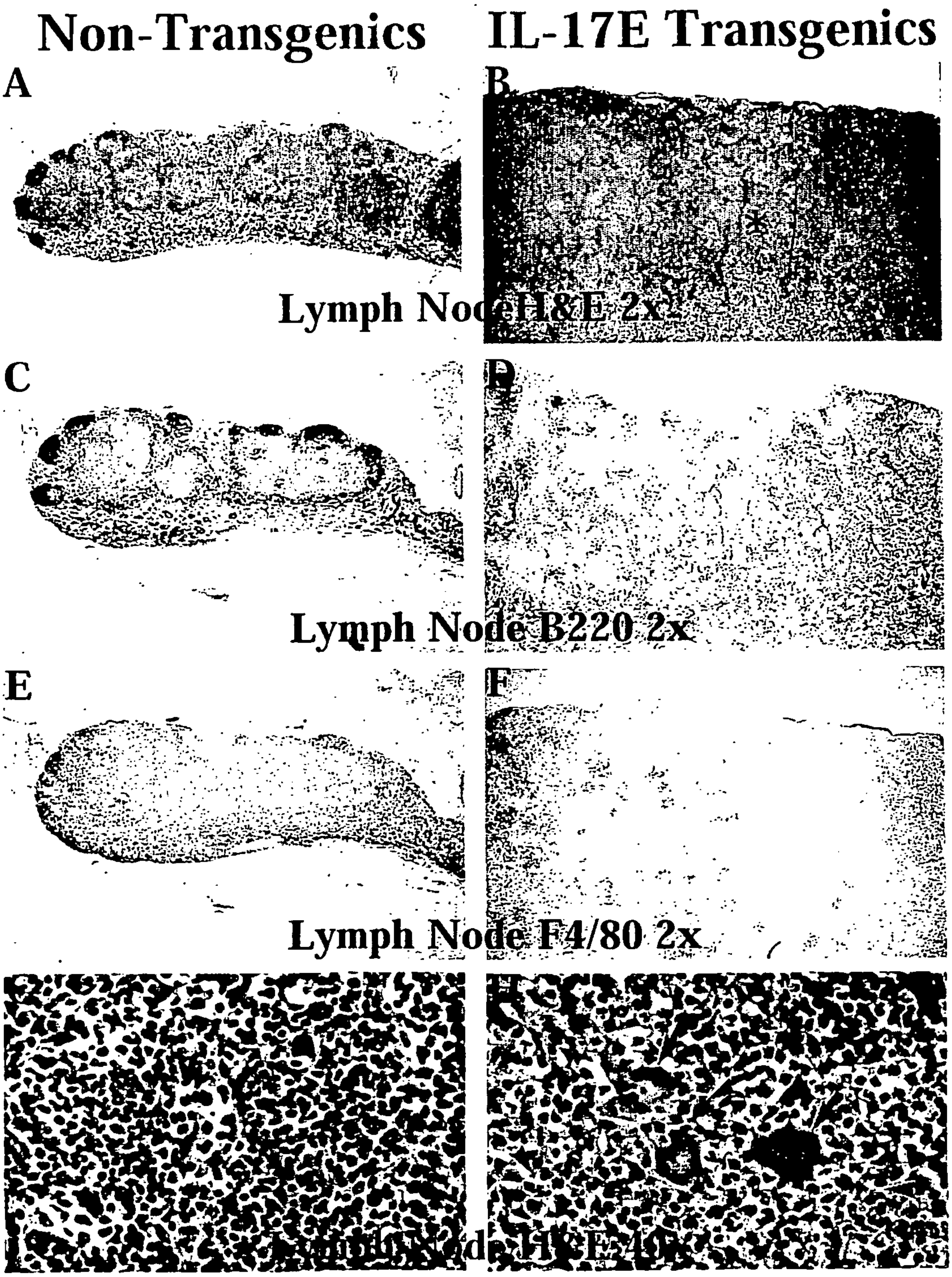
Non-Transgenics
IL-17E Transgenics
A
B
Lymph Node H&E 2x
C
D
Lymph Node B220 2x
E
F
Lymph Node F4/80 2x

# Figure 10

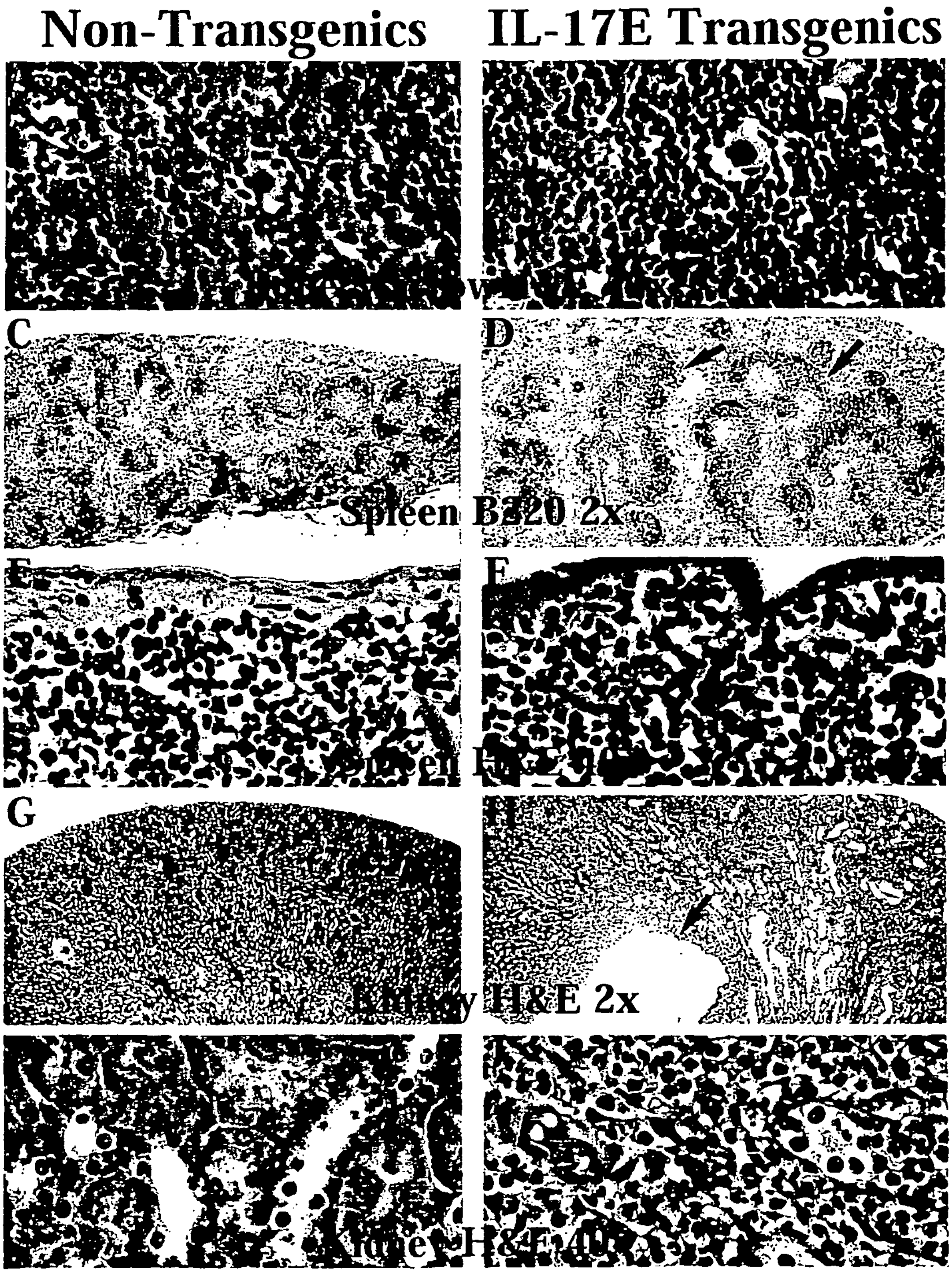
Non-Transgenics
IL-17E Transgenics
C
D
Spleen B220 2x
F
E
G
H
Kidney H&E 2x

Figure 11

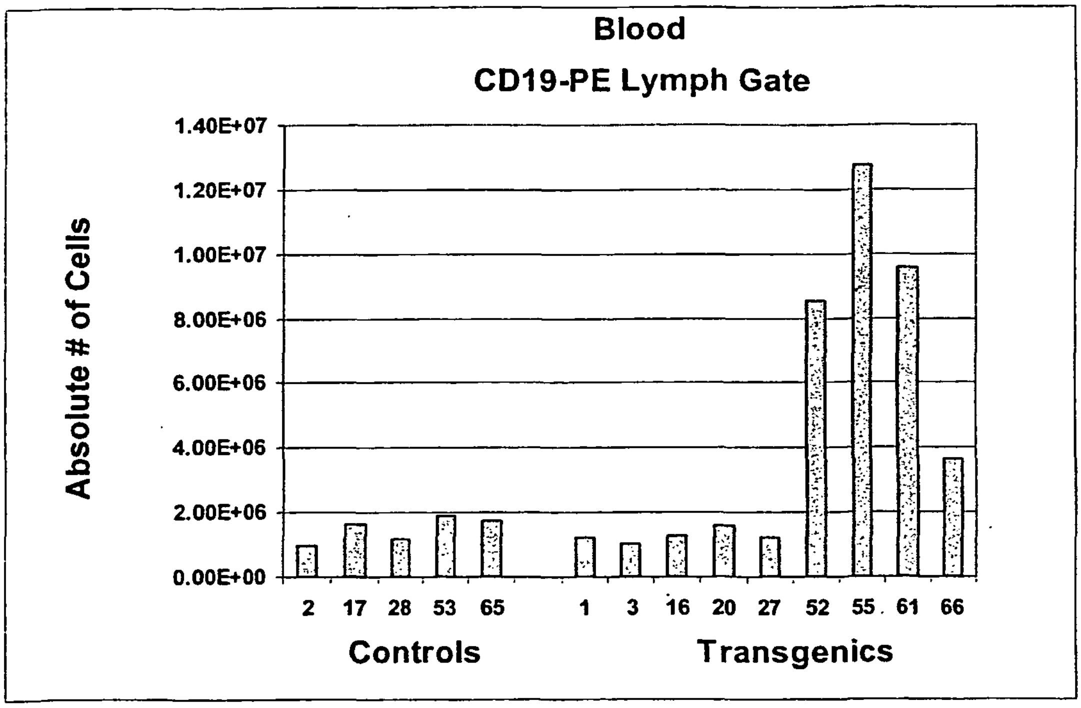
Blood
CD19-PE Lymph Gate
Absolute # of Cells
1.40E+07
1.20E+07
1.00E+07
8.00E+06
6.00E+06
4.00E+06
2.00E+06
0.00E+00
2
17
28
53
65
1
3
16
20
27
52
55
61
66
Controls
Transgenics

Figure 12

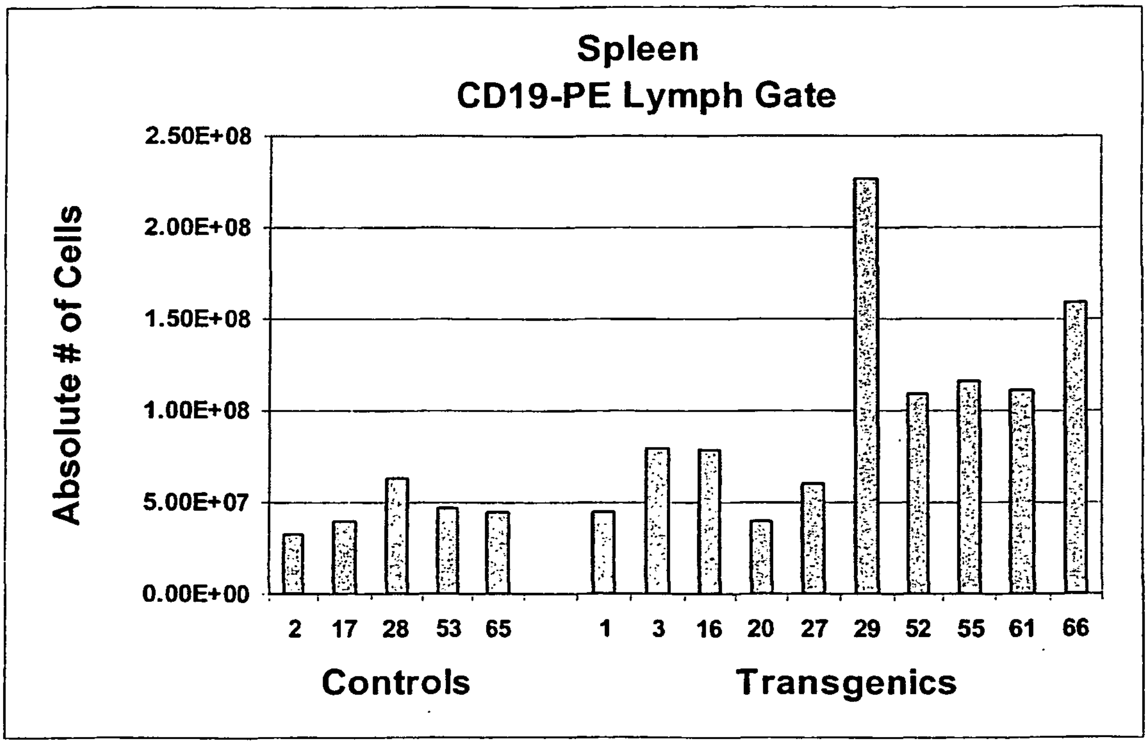
Spleen
CD19-PE Lymph Gate
Absolute # of Cells
2.50E+08
2.00E+08
1.50E+08
1.00E+08
5.00E+07
0.00E+00
2
17
28
53
65
1
3
16
20
27
29
52
55
61
66
Controls
Transgenics

Figure 13

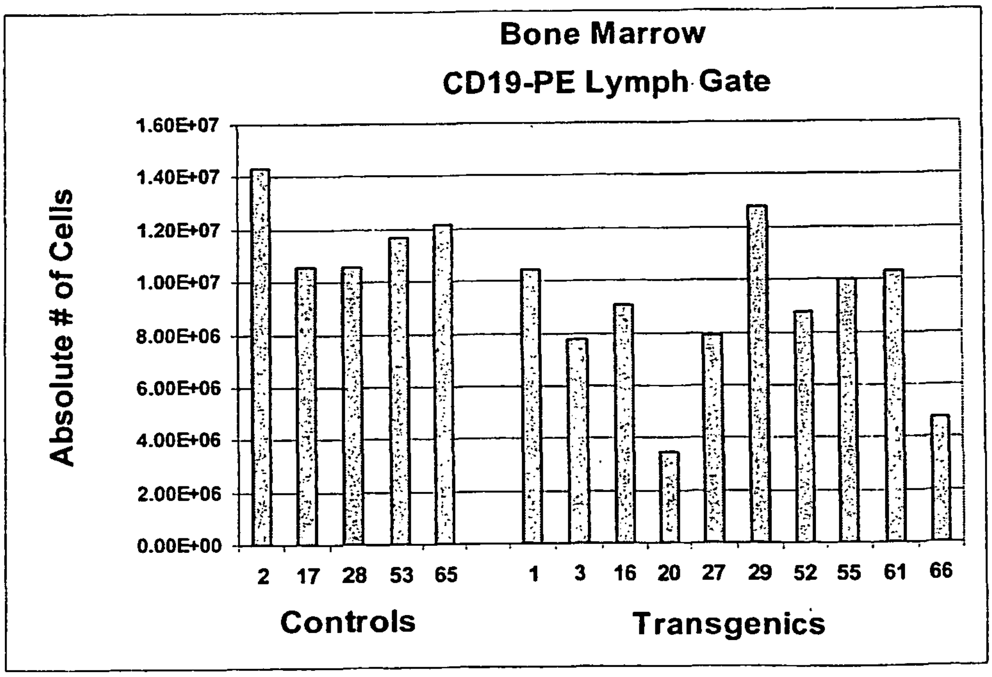
Bone Marrow
CD19-PE Lymph Gate
Absolute # of Cells
1.60E+07
1.40E+07
1.20E+07
1.00E+07
8.00E+06
6.00E+06
4.00E+06
2.00E+06
0.00E+00
2 17 28 53 65
1 3 16 20 27 29 52 55 61 66
Controls
Transgenics

Figure 14

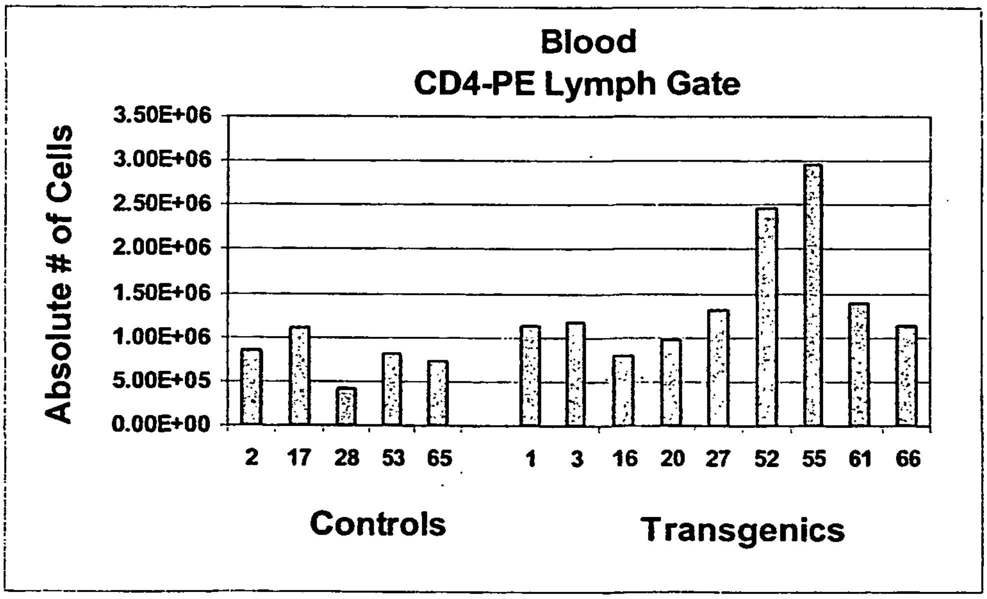
Blood
CD4-PE Lymph Gate
Absolute # of Cells
3.50E+06
3.00E+06
2.50E+06
2.00E+06
1.50E+06
1.00E+06
5.00E+05
0.00E+00
2
17
28
53
65
1
3
16
20
27
52
55
61
66
Controls
Transgenics

Figure 15

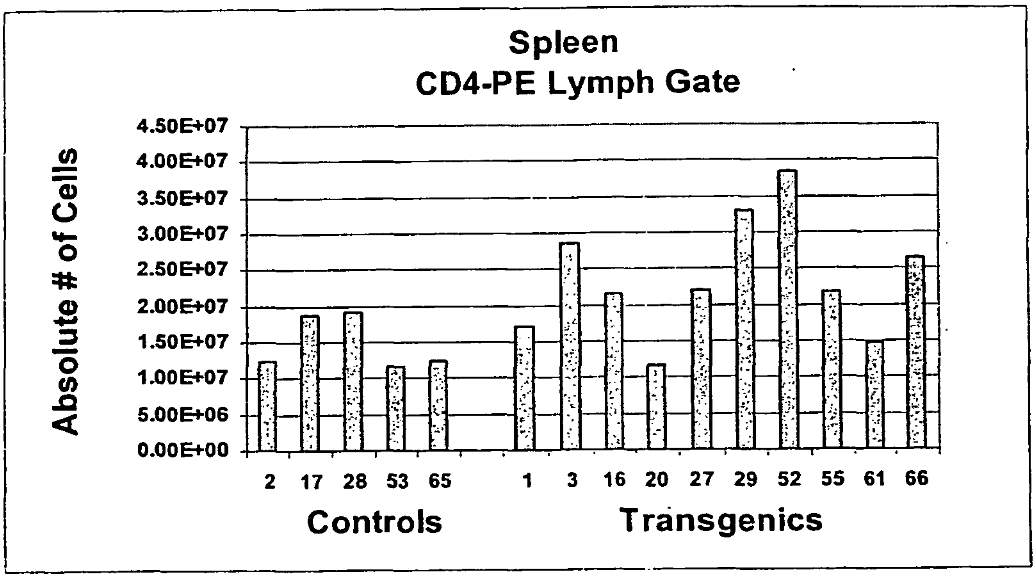
Spleen
CD4-PE Lymph Gate
Absolute # of Cells
4.50E+07
4.00E+07
3.50E+07
3.00E+07
2.50E+07
2.00E+07
1.50E+07
1.00E+07
5.00E+06
0.00E+00
2 17 28 53 65
1 3 16 20 27 29 52 55 61 66
Controls
Transgenics

Figure 16

# CD45R+ CELLS EXPRESSING IL17Br IN TRANSGENIC BONE MARROW

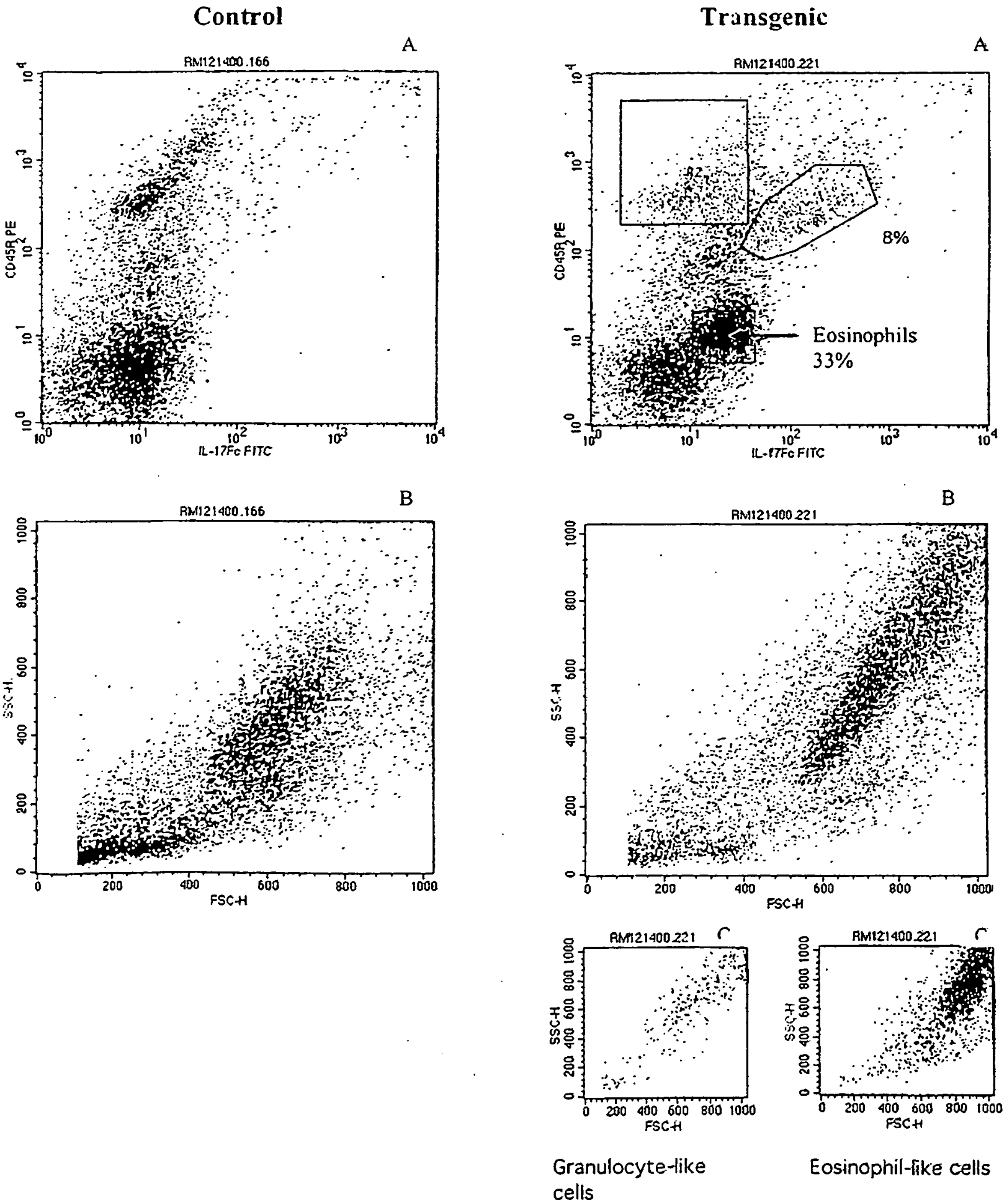

Figure 17

CD4+ CELLS EXPRESSING IL17Br IN TRANSGENIC BONE MARROW

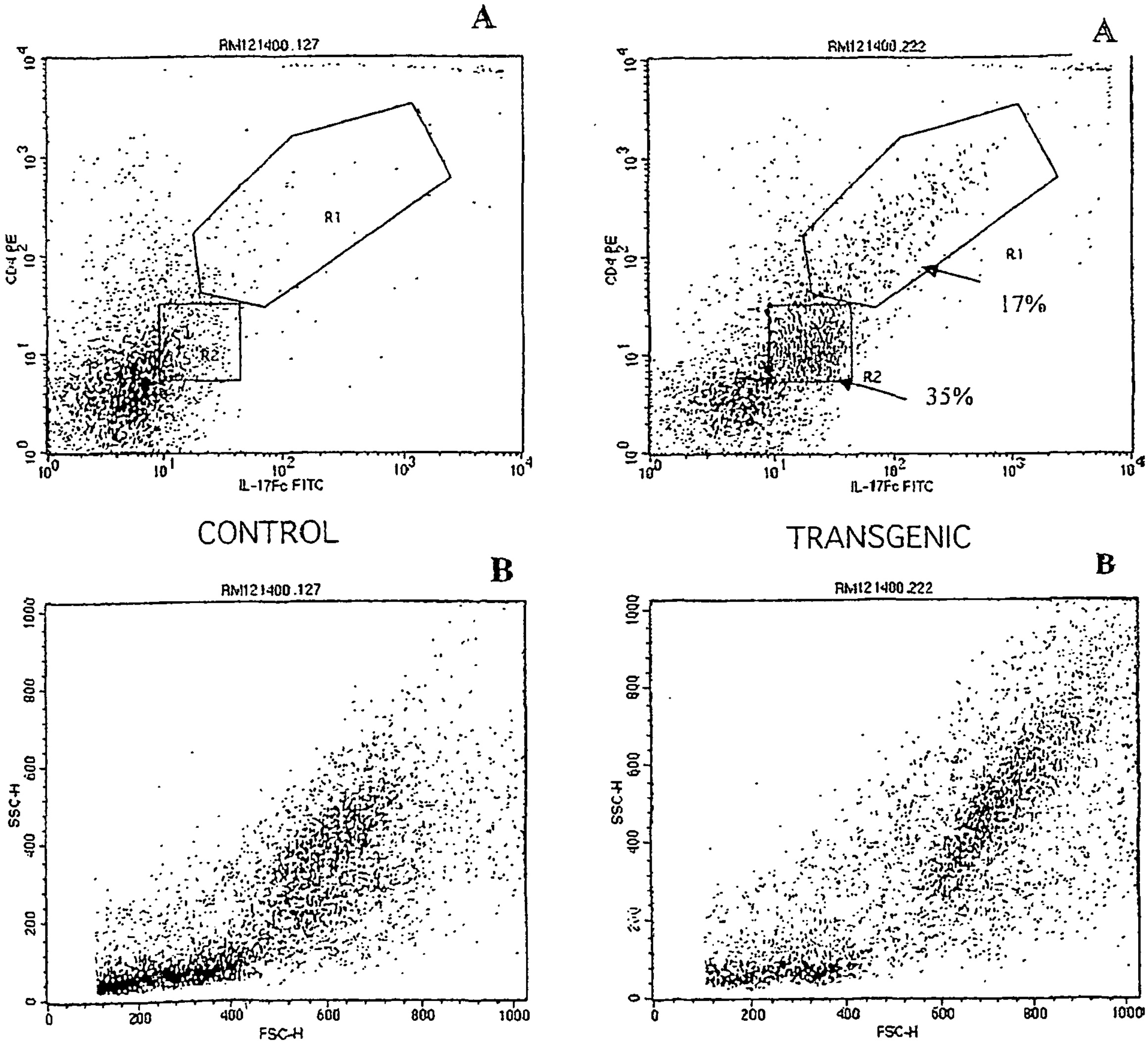

Figure 18

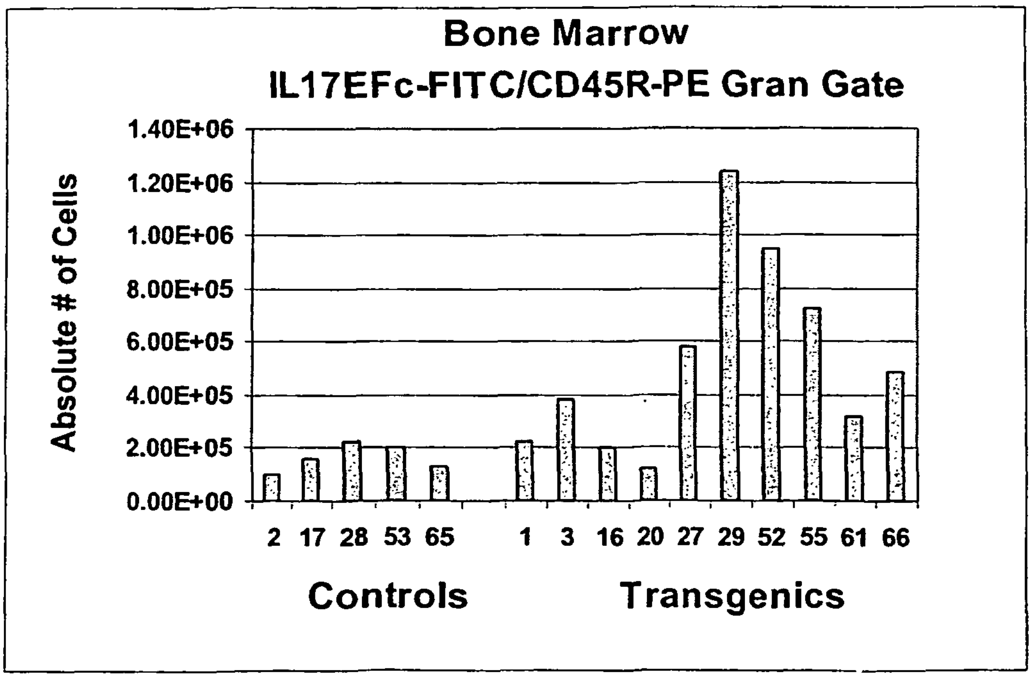
Bone Marrow
IL17EFc-FITC/CD45R-PE Gran Gate
Absolute # of Cells
1.40E+06
1.20E+06
1.00E+06
8.00E+05
6.00E+05
4.00E+05
2.00E+05
0.00E+00
2 17 28 53 65
1 3 16 20 27 29 52 55 61 66
Controls
Transgenics

Figure 19

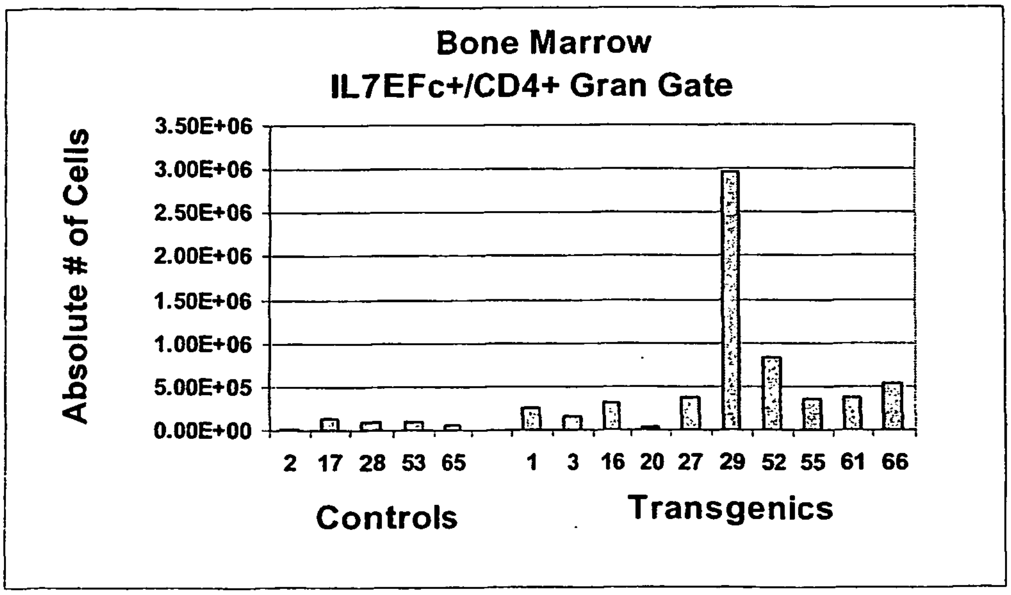

Bone Marrow
IL7EFc+/CD4+ Gran Gate
Absolute # of Cells
3.50E+06
3.00E+06
2.50E+06
2.00E+06
1.50E+06
1.00E+06
5.00E+05
0.00E+00
2 17 28 53 65
1 3 16 20 27 29 52 55 61 66
Controls
Transgenics

Figure 20

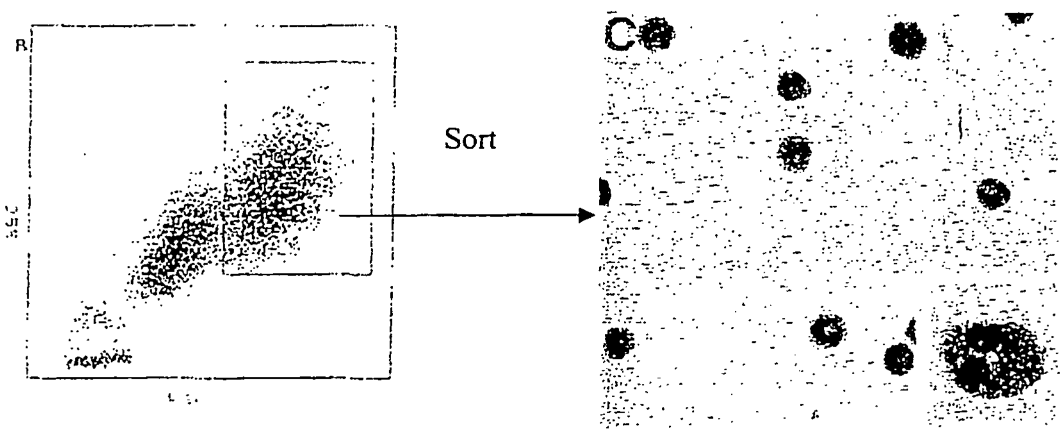

Figure 21A

## Non-transgenic Control

## Transgenic

### CD5+ on CD19+ Lymphocytes in Lymph Node

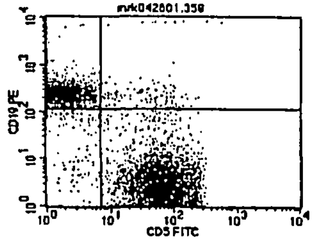


0.97%

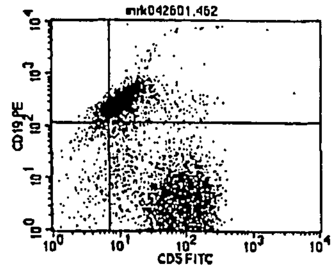


43.11%

### CD34+ on CD19+ Lymphocytes in Lymph Node

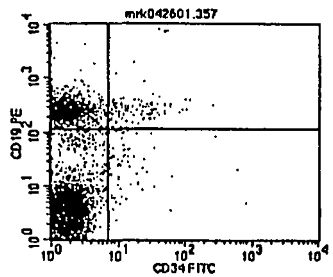


1.39%

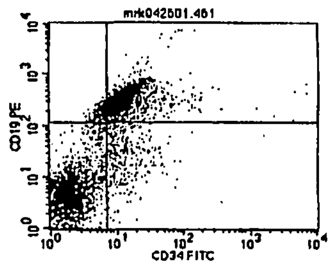


46.49%

Figure 21B

## CD4 Expression on Eosinophils in Bone Marrow

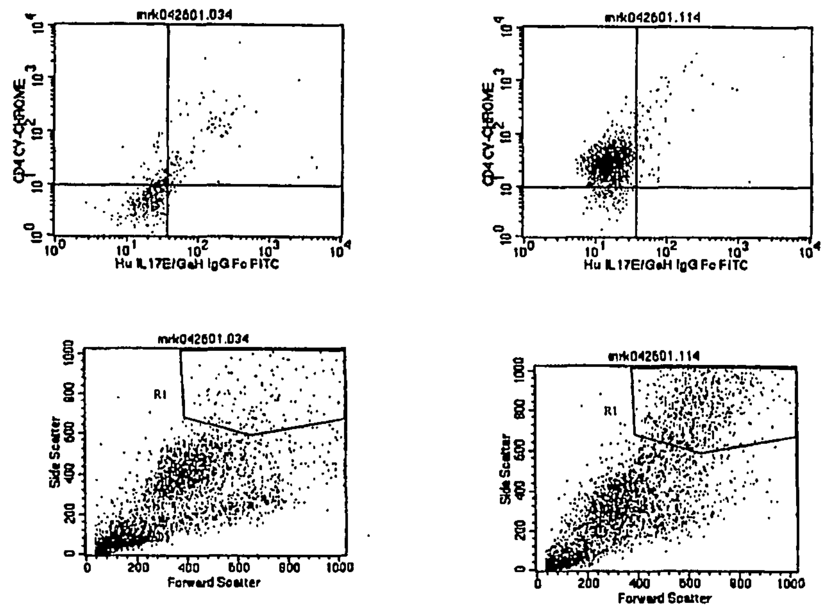

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5480981 A
- US 5808029 A
- WO 9723614 A
- US 9723183 W
- WO 9014363 A
- US 5824469 A
- US 5763192 A
- US 5814476 A
- US 5723323 A
- US 5817483 A
- US 9820094 W
- WO 9915650 A
- US 5234784 A
- EP 0154316 A
- EP 0401384 A
- US 4179337 A
- US 5252714 A
- US 5557032 A
- US 5489743 A
- WO 9428122 A
- US 4816567 A
- US 5585089 A
- US 5693762 A
- US 9605928 W
- US 9306926 W
- US 5545807 A
- US 91245 W
- GB 8901207 W
- EP 546073 B1
- EP 546073 A1
- US 9817364 W
- US 4376110 A
- US 5593992 A
- WO 9314081 A
- WO 9718626 A
- WO 9621452 A
- WO 9621654 A
- WO 9640143 A
- WO 9705878 A
- US 3929807 A
- WO 9705877 A
- WO 9716426 A
- WO 9716441 A
- WO 9716442 A
- WO 9822457 A
- WO 9824780 A
- WO 9824782 A
- WO 9924404 A
- WO 9932448 A
- EP 623674 A
- US 5492888 A
- US 5488032 A
- US 5464937 A
- US 5319071 A
- US 5180812 A
- WO 9501997 A
- WO 9402627 A
- WO 9006371 A
- US 4935343 A
- EP 364778 A
- EP 267611 A
- EP 220063 A
- WO 9623067 A
- US 5747444 A
- US 5359032 A
- US 5608035 A
- US 5843905 A
- US 5866576 A
- US 5869660 A
- US 5869315 A
- US 5872095 A
- US 5955480 A
- WO 9821957 A
- WO 9609323 A
- WO 9117184 A
- WO 9640907 A
- WO 9832733 A
- WO 9842325 A
- WO 9844940 A
- WO 9847892 A
- WO 9856377 A
- WO 9903837 A
- WO 9906426 A
- WO 9906042 A
- WO 9117249 A
- WO 9817661 A
- WO 9708174 A
- WO 9534326 A
- WO 9936426 A
- WO 9936415 A
- EP 534978 A
- EP 894795 A
- FR 2762514
- US 5075222 A
- WO 9108285 A
- AU 9173636
- WO 9216221 A
- WO 9321946 A

- WO 9406457 A
- WO 9421275 A
- FR 2706772
- WO 9421235 A
- DE 4219626
- WO 9420517 A
- WO 9622793 A
- WO 9728828 A
- WO 9936541 A
- EP 308378 A
- EP 422339 A
- GB 2218101 A
- EP 393438 A
- WO 9013575 A
- EP 398327 A
- EP 412486 A
- WO 9103553 A
- EP 418014 A
- JP 3127800 A
- EP 433900 A
- US 5136021 A
- GB 2246569 A
- EP 464533 A
- WO 9201002 A
- WO 9213095 A
- EP 512528 A
- EP 526905 A
- WO 9307863 A
- EP 568928 A
- WO 9319777 A
- EP 417563 A
- WO 9406476 A
- US 9712244 W
- US 5116964 A
- WO 8909622 A
- WO 9116437 A
- EP 315062 A
- WO 9620206 A
- EP 510691 A
- US 94001875 W
- US 9300829 W
- US 3773919 A
- EP 058481 A
- EP 133988 A
- EP 036676 A
- EP 088046 A
- EP 143949 A
- US 5272071 A
- EP 9193051 A
- EP 505500 A
- US 9007642 W
- WO 9109955 A
- WO 9505452 A, Baetge
- US 9409299 W
- US 4892538 A
- US 5011472 A
- US 5106627 A
- US 9100157 W
- US 9100155 W, Aebischer
- WO 9641865 A
- US 96099486 W
- WO 9731898 A
- US 9703137 W
- WO 9731899 A
- US 9503157 W
- US 5364791 A
- WO 9640911 A
- WO 9710337 A
- US 5514578 A
- WO 9738117 A
- WO 9637609 A
- WO 9303162 A
- US 5464758 A
- US 5650298 A
- US 5654168 A
- US 5741679 A
- US 5834186 A
- WO 9534670 A, Johnson
- US 9507178 W
- US 5672344 A
- US 5399346 A
- US 5631236 A
- US 5672510 A
- US 5635399 A
- US 4970154 A
- WO 9640958 A
- US 5679559 A
- US 5676954 A
- US 5593875 A
- US 4945050 A
- US 72323200 A
- US 09810384 B
- US 60266159 B
- US 60213125 B

**Non-patent literature cited in the description**


- **H. SIMONSEN ; H.F. LODISH.** *Trends in Pharmacological Sciences,* 1994, vol. 15, 437-441
- **TARTAGLIA et al.** *Cell,* 1995, vol. 83, 1263-1271
- **GRAHAM et al.** *Virology,* 1973, vol. 52, 456
- **SAMBROOK et al.** Molecular Cloning, a Laboratory Manual. Cold Spring Harbor Laboratories, 1989
- **DAVIS et al.** Basic Methods in Molecular Biology. Elsevier, 1986
- **CHU et al.** *Gene,* 1981, vol. 13, 197

- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridization: a practical approach. IRL Press Limited, 1999
- **SUGGS et al.** Developmental Biology Using Purified Genes. 1981, 683
- **KYTE et al.** *J. Mol. Biol.,* 1982, vol. 157, 105-131
- **MOULT J.** *Curr. Op. in Biotech.,* 1996, vol. 7 (4), 422-427
- **CHOU et al.** *Biochemistry,* 1974, vol. 13 (2), 222-245
- **CHOU et al.** *Biochemistry,* 1974, vol. 113 (2), 211-222
- **CHOU et al.** *Adv. Enzymol. Relat. Areas Mol. Biol.,* 1978, vol. 47, 45-148
- **CHOU et al.** *Ann. Rev. Biochem.,* vol. 47, 251-276
- **CHOU et al.** *Biophys. J.,* 1979, vol. 26, 367-384
- **HOLM et al.** *Nucl. Acid. Res.,* 1999, vol. 27 (1), 244-247
- **BRENNER et al.** *Curr. Op. Struct. Biol.,* 1997, vol. 7 (3), 369-376
- **JONES, D.** *Curr. Opin. Struct. Biol.,* 1997, vol. 7 (3), 377-87
- **SIPPL et al.** *Structure,* 1996, vol. 4 (1), 15-19
- **BOWIE et al.** *Science,* 1991, vol. 253, 164-170
- **GRIBSKOV et al.** *Meth. Enzym.,* 1990, vol. 183, 146-159
- **GRIBSKOV et al.** *Proc. Nat. Acad. Sci.,* 1987, vol. 84 (13), 4355-4358
- **CAPON et al.** *Nature,* 1989, vol. 337, 525-31
- **ZHENG et al.** *J. Immunol.,* 1995, vol. 154, 5590-5600
- **FISHER et al.** *N. Engl. J. Med.,* 1996, vol. 334, 1697-1702
- **VAN ZEE et al.** *J. Immunol.,* vol. 156, 2221-2230
- **CAPON et al.** *Nature,* 1989, vol. 337, 525-531
- **HARVILL et al.** *Immunotech.,* 1995, vol. 1, 95-105
- **LINSLEY.** *J. Exp. Med.,* 1991, vol. 174, 561-569
- Computational Molecular Biology. Oxford University Press, 1988
- Biocomputing: Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M. Stockton Press, 1991
- **CARILLO et al.** *SIAM J. Applied Math.,* 1988, vol. 48, 1073
- **DEVEREUX et al.** *Nucl. Acid. Res.,* 1984, vol. 12, 387
- **ALTSCHUL et al.** *J. Mol. Biol.,* 1990, vol. 215, 403-410
- **ALTSCHUL et al.** BLAST Manual. NCB/NLM/NIH
- **DAYHOFF et al.** Atlas of Protein Sequence and Structure. 1978, vol. 5
- **HENIKOFF et al.** *Proc. Natl. Acad. Sci USA,* 1992, vol. 89, 10915-10919
- **NEEDLEMAN et al.** *J. Mol. Biol.,* 1970, vol. 48, 443-453
- Program Manual, Wisconsin Package. September 1997
- **SAMBROOK et al.** Molecular Cloning: A Laboratory Manual. Cold Spring Harbor Laboratory Press, 1989
- Current Protocols in Molecular Biology. Green Publishers Inc. and Wiley and Sons, 1994
- **ENGELS et al.** *Angew. Chem. Intl. Ed.,* 1989, vol. 28, 716-734
- Meth. Enz. Academic Press Inc, 1990, vol. 185
- **BERNOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. USA,* 1981, vol. 78, 144-145
- **BRINSTER et al.** *Nature,* 1982, vol. 296, 39-42
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **SWIFT et al.** *Cell,* 1984, vol. 38, 639-646
- **ORNITZ et al.** *Cold Spring Harbor Symp. Quant. Biol.,* 1986, vol. 50, 399-409
- **MACDONALD.** *Hepatology,* 1987, vol. 7, 425-515
- **HANAHAN.** *Nature,* 1985, vol. 315, 115-122
- **GROSSCHEDL et al.** *Cell,* 1984, vol. 38, 647-658
- **ADAMES et al.** *Nature,* 1985, vol. 318, 533-538
- **ALEXANDER et al.** *Mol. Cell. Biol.,* 1987, vol. 7, 1436-1444
- **LEDER et al.** *Cell,* 1986, vol. 45, 485-495
- **PINKERT et al.** *Genes and Devel.,* 1987, vol. 1, 268-276
- **KRUMLAUF et al.** *Mol. Cell. Biol.,* 1985, vol. 5, 1639-1648
- **HAMMER et al.** *Science,* 1987, vol. 235, 53-58
- **KELSEY et al.** *Genes and Devel.,* 1987, vol. 1, 161-171
- **MOGRAM et al.** *Nature,* 1985, vol. 315, 338-340
- **KOLLIAS et al.** *Cell,* 1986, vol. 46, 89-94

- **READHEAD et al.** *Cell,* 1987, vol. 48, 703-712
- **SANI.** *Nature,* 1985, vol. 314, 283-286
- **MASON et al.** *Science,* 1986, vol. 234, 1372-1378
- **URLAUB et al.** *Proc. Natl. Acad. Sci. USA,* 1980, vol. 97, 4216-4220
- **KITTS et al.** *Biotechniques,* 1993, vol. 14, 810-817
- **LUCKLOW.** *Curr. Opin. Biotechnol.,* 1993, vol. 4, 564-572
- **LUCKLOW et al.** *J. Virol.,* 1993, vol. 67, 4566-4579
- **MARSTON et al.** *Meth. Enz.,* 1990, vol. 182, 264-275
- Current Protocols in Molecular Biology. John Wiley & Sons, 1993
- **MERRIFIELD et al.** *J. Am. Chem. Soc.,* 1963, vol. 85, 2149
- **HOUGHTEN et al.** *Proc. Natl. Acad. Sci. USA,* 1985, vol. 82, 5132
- **STEWART ; YOUNG.** Solid Phase Peptide Synthesis. Pierce Chemical Co, 1984
- **ROBERTS et al.** *Proc. Natl. Acad. Sci. USA,* 1997, vol. 94, 12297-12303
- **ROBERTS, R.** *Curr. Opin. Chem. Biol.,* 1999, vol. 3, 268-273
- **FRANCIS et al.** *Focus on Growth Factors,* 1992, vol. 3, 4-10
- **KOHLER et al.** *Nature,* 1975, vol. 256, 495-497
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MORRISON et al.** *Proc. Natl. Acad. Sci. USA,* 1985, vol. 81, 6851-6855
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **JAKOBOVITS et al.** *Proc. Natl. Acad. Sci. USA,* 1993, vol. 90, 2551-2555
- **JAKOBOVITS et al.** *Nature,* 1993, vol. 362, 255-258
- **BRUGGERMANN et al.** *Year in Immunol.,* 1993, vol. 7, 33
- **HOOGENBOOM et al.** *J. Mol. Biol.,* 1991, vol. 227, 381
- **MARKS et al.** *J. Mol. Biol.,* 1991, vol. 222, 581
- **SOLA.** Monoclonal Antibodies: A Manual of Techniques. CRC Press, Inc, 1987, 147-158
- **BAYER et al.** *Meth. Enz.,* 1990, vol. 184, 138-163
- Current Protocols in Molecular Biology. John Wiley & Sons, 1995
- **LEE et al.** *Nature,* 1994, vol. 372, 739
- **HAN et al.** *Biochimica Biophysica Acta,* 1995, vol. 1265, 224-227
- **FALWELL et al.** *Proc. Natl. Acad. Sci. USA,* 1994, vol. 91, 664-668
- **SCHWARZE et al.** *Science,* 1999, vol. 285, 1569-1572
- **NAGAHARA et al.** *Nature Medicine,* 1998, vol. 4, 1449-1452
- **STRAUSS, E.** Introducing Proteins Into the Body's Cells. *Science,* 1999, vol. 285, 1466-1467
- **NEVE et al.** *Cytokine,* 1996, vol. 8 (5), 365-370
- **HOLLER et al.** *1st International Symposium on Cytokines in Bone Marrow Transplantation,* 1993, 147
- **RANKIN et al.** *British Journal of Rheumatology,* 1995, vol. 34, 334-342
- **KIEFT et al.** *7th European Congress of Clinical Microbiology and Infectious Diseases,* 1995, 9
- **ELLIOTT et al.** *Lancet,* 1994, vol. 344, 1125-1127
- **ELLIOTT et al.** *Lancet,* 1994, vol. 344, 1105-1110
- **MOUNTZ et al.** *J. Immunology,* vol. 155, 4829-4837
- The Merck Manual of Diagnosis and Therapy. Merck, Sharp & Dohme Research Laboratories, Merck & Co, 1992
- **GOODMAN ; GILMAN.** The Pharmacological Basis of Therapeutics. MacMillan, 1985
- Remington's Pharmaceutical Sciences. Mack Publishing Company, 1990
- **SIDMAN et al.** *Biopolymers,* 1983, vol. 22, 547-556
- **LANGER et al.** *J. Biomed. Mater. Res.,* 1981, vol. 15, 167-277
- **LANGER.** *Chem. Tech.,* 1982, vol. 12, 98-105
- **EPPSTEIN et al.** *Proc. Natl. Acad. Sci . USA,* 1985, vol. 82, 3688-3692
- **KUCHERLAPATI.** *Prog. in Nucl. Acid Res. & Mol. Biol.,* 1989, vol. 36, 301
- **THOMAS et al.** *Cell,* 1986, vol. 44, 419-428
- **THOMAS ; CAPECCHI.** *Cell,* 1987, vol. 51, 503-512
- **DOETSCHMAN et al.** *Proc. Natl. Acad. Sci.,* 1988, vol. 85, 8583-8587
- **DOETSCHMAN et al.** *Nature,* 1987, vol. 330, 576-578
- **SAUER.** *Current Opinion In Biotechnology,* 1994, vol. 5, 521-527
- **SAUE.** *Methods In Enzymology,* 1993, vol. 225, 890-900
- **BAUBONIS ; SAUER.** *Nucleic Acids Res.,* 1993, vol. 21, 2025-2029
- **O'GORMAN et al.** *Science,* 1991, vol. 251, 1351-1355
- **SAUER.** *Current Opinion In Biotechnology,* 1994
- **SAUER.** *Methods In Enzymology,* 1993

- **WINN et al.** *Exper. Neurol.,* 1991, vol. 113, 322-329
- **AEBISCHER et al.** *Exper. Neurol.,* 1991, vol. 111, 269-275
- **TRESCO et al.** *ASAIO,* 1992, vol. 38, 17-23
- *Science,* 2000, vol. 287, 816-817826-830
- **HEFTI.** *Neurobiology,* 1994, vol. 25, 1418-1435
- **SMITH et al.** *Cell,* 1994, vol. 76, 959-62
- **LUETHY et al.** *Protein Science,* 1994, vol. 3, 139-46
- **SIMONET et al.** *J. Biol. Chem.,* 1993, vol. 268, 8221-8229
- **SIMONET et al.** *J. Clin. Invest.,* 1994, vol. 94, 1310-1319
- **BRINSTER et al.** *Proc. Natl. Acad. Sci. USA,* 1985, vol. 82, 4438-4442
- **CAMPENA ; BEHM.** *J. Immunol. Meth.,* 2000, vol. 234, 59-75
- **XIA et al.** *Cytometry,* 2000, vol. 42, 114-7
- **CALDWELL ; LASCOMBE.** Evaluation of Peripheral Blood Lymphocytosis. Acedemic Information Systems, Inc, 2000
- **NEUBER et al.** *Dermatology,* 1996, vol. 192, 110-5
- **SAKAMOTO et al.** *Intl. Archives of Allergy and Immunology,* 1996, vol. 111 (1), 26-8

SEQUENCE LISTING

<110> Amgen, Inc.

<120> IL-17 Like Molecules and Uses Thereof

<130> 01017/37128B

<140>
<141>

<150> 09/810,384
<151> 2001-03-16

<150> 60/266,159
<151> 2001-02-02

<150> 60/213,125
<151> 2000-06-22

<160> 22

<170> PatentIn Ver. 2.0

<210> 1
<211> 644
<212> DNA
<213> Homo sapiens

<220>
<221> CDS
<222> (159)..(641)

<400> 1

```
ctcaagtcac tccctaaaaa gacagtggaa ataaatttga ataaacaaaa caggcttgct 60

gaaaataaaa tcaggactcc taacctgctc cagtcagcct gcttccacga ggcctgtcag 120

tcagtgcccc acttgtgact gagtgtgcag tgcccagc atg tac cag gtg gtt gca 176
                                          Met Tyr Gln Val Val Ala
                                           1               5

ttc ttg gca atg gtc atg gga acc cac acc tac agc cac tgg ccc agc   224
Phe Leu Ala Met Val Met Gly Thr His Thr Tyr Ser His Trp Pro Ser
            10                  15                  20

tgc tgc ccc agc aaa ggg cag gac acc tct gag gag ctg ctg agg tgg   272
Cys Cys Pro Ser Lys Gly Gln Asp Thr Ser Glu Glu Leu Leu Arg Trp
        25                  30                  35

agc act gtg cct gtg cct ccc cta gag cct gct agg ccc aac cgc cac   320
Ser Thr Val Pro Val Pro Pro Leu Glu Pro Ala Arg Pro Asn Arg His
    40                  45                  50

cca gag tcc tgt agg gcc agt gaa gat gga ccc ctc aac agc agg gcc   368
Pro Glu Ser Cys Arg Ala Ser Glu Asp Gly Pro Leu Asn Ser Arg Ala
55                  60                  65                  70

atc tcc ccc tgg aga tat gag ttg gac aga gac ttg aac cgg ctc ccc   416
Ile Ser Pro Trp Arg Tyr Glu Leu Asp Arg Asp Leu Asn Arg Leu Pro
                75                  80                  85

cag gac ctg tac cac gcc cgt tgc ctg tgc ccg cac tgc gtc agc cta   464
Gln Asp Leu Tyr His Ala Arg Cys Leu Cys Pro His Cys Val Ser Leu
            90                  95                  100

cag aca ggc tcc cac atg gac ccc cgg ggc aac tcg gag ctg ctc tac   512
Gln Thr Gly Ser His Met Asp Pro Arg Gly Asn Ser Glu Leu Leu Tyr
        105                 110                 115

cac aac cag act gtc ttc tac cgg cgg cca tgc cat ggc gag aag ggc   560
His Asn Gln Thr Val Phe Tyr Arg Arg Pro Cys His Gly Glu Lys Gly
    120                 125                 130

acc cac aag ggc tac tgc ctg gag cgc agg ctg tac cgt gtt tcc tta   608
Thr His Lys Gly Tyr Cys Leu Glu Arg Arg Leu Tyr Arg Val Ser Leu
135                 140                 145                 150

gct tgt gtg tgt gtg cgg ccc cgt gtg atg ggc tag                   644
Ala Cys Val Cys Val Arg Pro Arg Val Met Gly
                155                 160
```

<210> 2
<211> 161
<212> PRT
<213> Homo sapiens

<400> 2

```
Met Tyr Gln Val Val Ala Phe Leu Ala Met Val Met Gly Thr His Thr
1               5                   10                  15

Tyr Ser His Trp Pro Ser Cys Cys Pro Ser Lys Gly Gln Asp Thr Ser
            20                  25                  30

Glu Glu Leu Leu Arg Trp Ser Thr Val Pro Val Pro Pro Leu Glu Pro
        35                  40                  45

Ala Arg Pro Asn Arg His Pro Glu Ser Cys Arg Ala Ser Glu Asp Gly
    50                  55                  60

Pro Leu Asn Ser Arg Ala Ile Ser Pro Trp Arg Tyr Glu Leu Asp Arg
65                  70                  75                  80

Asp Leu Asn Arg Leu Pro Gln Asp Leu Tyr His Ala Arg Cys Leu Cys
                85                  90                  95

Pro His Cys Val Ser Leu Gln Thr Gly Ser His Met Asp Pro Arg Gly
            100                 105                 110

Asn Ser Glu Leu Leu Tyr His Asn Gln Thr Val Phe Tyr Arg Arg Pro
        115                 120                 125

Cys His Gly Glu Lys Gly Thr His Lys Gly Tyr Cys Leu Glu Arg Arg
    130                 135                 140

Leu Tyr Arg Val Ser Leu Ala Cys Val Cys Val Arg Pro Arg Val Met
145                 150                 155                 160

Gly
```

<210> 3
<211> 1013
<212> DNA
<213> Mus musculus

<220>
<221> CDS
<222> (1)..(507)

<400> 3

```
atg tac cag gct gtt gca ttc ttg gca atg atc gtg gga acc cac acc    48
Met Tyr Gln Ala Val Ala Phe Leu Ala Met Ile Val Gly Thr His Thr
 1               5                  10                  15

gtc agc ttg cgg atc cag gag ggc tgc agt cac ttg ccc agc tgc tgc    96
Val Ser Leu Arg Ile Gln Glu Gly Cys Ser His Leu Pro Ser Cys Cys
            20                  25                  30

ccc agc aaa gag caa gaa ccc ccg gag gag tgg ctg aag tgg agc tct   144
Pro Ser Lys Glu Gln Glu Pro Pro Glu Glu Trp Leu Lys Trp Ser Ser
        35                  40                  45

gca tct gtg tcc ccc cca gag cct ctg agc cac acc cac cac gca gaa   192
Ala Ser Val Ser Pro Pro Glu Pro Leu Ser His Thr His His Ala Glu
    50                  55                  60

tcc tgc agg gcc agc aag gat ggc ccc ctc aac agc agg gcc atc tct   240
Ser Cys Arg Ala Ser Lys Asp Gly Pro Leu Asn Ser Arg Ala Ile Ser
 65                  70                  75                  80

cct tgg agc tat gag ttg gac agg gac ttg aat cgg gtc ccc cag gac   288
Pro Trp Ser Tyr Glu Leu Asp Arg Asp Leu Asn Arg Val Pro Gln Asp
                85                  90                  95

ctg tac cac gct cga tgc ctg tgc cca cac tgc gtc agc cta cag aca   336
Leu Tyr His Ala Arg Cys Leu Cys Pro His Cys Val Ser Leu Gln Thr
            100                 105                 110

ggc tcc cac atg gac ccg ctg ggc aac tcc gtc cca ctt tac cac aac   384
Gly Ser His Met Asp Pro Leu Gly Asn Ser Val Pro Leu Tyr His Asn
        115                 120                 125

cag acg gtc ttc tac cgg cgg cca tgc cat ggc gag gaa ggt acc cat   432
Gln Thr Val Phe Tyr Arg Arg Pro Cys His Gly Glu Glu Gly Thr His
    130                 135                 140

cgc cgc tac tgc ttg gag cgc agg ctc tac cga gtc tcc ttg gct tgt   480
Arg Arg Tyr Cys Leu Glu Arg Arg Leu Tyr Arg Val Ser Leu Ala Cys
145                 150                 155                 160

gtg tgt gtg cgg ccc cgg gtc atg gct tagtcatgct caccacctgc         527
Val Cys Val Arg Pro Arg Val Met Ala
                165

ctgaggctga tgcccggttg ggagagaggg ccaggtgtac aatcaccttg ccaatgcggg 587

ccgggttcaa gccctccaaa gccctacctg aagcagcagg ctcccgggac aagatggagg 647

acttggggag aaactctgac ttttgcactt tttggaagca cttttgggaa ggagcaggtt 707

ccgcttgtgc tgctagagga tgctgttgtg gcatttctac tcaggaacgg actccaaagg 767

cctgctgacc ctggaagcca tactcctggc tcctttcccc tgaatccccc aactcctggc 827

acaggcactt tctccacctc tcccccttg cctttttgttg tgtttgtttg tgcatgccaa 887

ctctgcgtgc agccaggtgt aattgccttg aaggatggtt ctgaggtgaa agctgttatc 947

gaaagtgaag agatttatcc aaataaacat ctgtgtttaa aaaaaaaaaa aaaaaaaaaa 1007

aaaaaa                                                            1013
```

<210> 4
<211> 169

<212> PRT
<213> Mus musculus

<400> 4

```
Met Tyr Gln Ala Val Ala Phe Leu Ala Met Ile Val Gly Thr His Thr
1               5                   10                  15

Val Ser Leu Arg Ile Gln Glu Gly Cys Ser His Leu Pro Ser Cys Cys
            20                  25                  30

Pro Ser Lys Glu Gln Glu Pro Pro Glu Glu Trp Leu Lys Trp Ser Ser
        35                  40                  45

Ala Ser Val Ser Pro Pro Glu Pro Leu Ser His Thr His His Ala Glu
    50                  55                  60

Ser Cys Arg Ala Ser Lys Asp Gly Pro Leu Asn Ser Arg Ala Ile Ser
65                  70                  75                  80

Pro Trp Ser Tyr Glu Leu Asp Arg Asp Leu Asn Arg Val Pro Gln Asp
                85                  90                  95

Leu Tyr His Ala Arg Cys Leu Cys Pro His Cys Val Ser Leu Gln Thr
            100                 105                 110

Gly Ser His Met Asp Pro Leu Gly Asn Ser Val Pro Leu Tyr His Asn
        115                 120                 125

Gln Thr Val Phe Tyr Arg Arg Pro Cys His Gly Glu Glu Gly Thr His
    130                 135                 140

Arg Arg Tyr Cys Leu Glu Arg Arg Leu Tyr Arg Val Ser Leu Ala Cys
145                 150                 155                 160

Val Cys Val Arg Pro Arg Val Met Ala
                165
```

<210> 5
<211> 155
<212> PRT
<213> Homo sapiens

<400> 5

```
Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45
```

```
Leu Asn Ile His Asn Arg Asn Thr Asn Thr Asn Pro Lys Arg Ser Ser
     50                  55                  60

Asp Tyr Tyr Asn Arg Ser Thr Ser Pro Trp Asn Leu His Arg Asn Glu
 65                  70                  75                  80

Asp Pro Glu Arg Tyr Pro Ser Val Ile Trp Glu Ala Lys Cys Arg His
                 85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155
```

<210> 6
<211> 117
<212> PRT
<213> Homo sapiens

<400> 6

```
Arg Asn Ile Glu Glu Met Val Ala Gln Leu Arg Asn Ser Ser Glu Leu
  1               5                  10                  15

Ala Gln Arg Lys Cys Glu Val Asn Leu Gln Leu Trp Met Ser Asn Lys
             20                  25                  30

Arg Ser Leu Ser Pro Trp Gly Tyr Ser Ile Asn His Asp Pro Ser Arg
         35                  40                  45

Ile Pro Val Asp Leu Pro Glu Ala Arg Cys Leu Cys Leu Gly Cys Val
     50                  55                  60

Asn Pro Phe Thr Met Gln Glu Asp Arg Ser Met Val Ser Val Pro Val
 65                  70                  75                  80

Phe Ser Gln Val Pro Val Arg Arg Arg Leu Cys Pro Pro Pro Pro Arg
                 85                  90                  95

Thr Gly Pro Cys Arg Gln Arg Ala Val Met Glu Thr Ile Val Ala Gly
            100                 105                 110

Cys Thr Cys Ile Phe
        115
```

<210> 7
<211> 117
<212> PRT
<213> Homo sapiens

<400> 7

```
Arg Asn Ile Glu Glu Met Val Ala Gln Leu Arg Asn Ser Ser Glu Leu
  1               5                  10                  15
```

```
Ala Gln Arg Lys Cys Glu Val Asn Leu Gln Leu Trp Met Ser Asn Lys
            20                  25                  30

Arg Ser Leu Ser Pro Trp Gly Tyr Ser Ile Asn His Asp Pro Ser Arg
        35                  40                  45

Ile Pro Val Asp Leu Pro Glu Ala Arg Cys Leu Cys Leu Gly Cys Val
    50                  55                  60

Asn Pro Phe Thr Met Gln Glu Asp Arg Ser Met Val Ser Val Pro Val
65                  70                  75                  80

Phe Ser Gln Val Pro Val Arg Arg Arg Leu Cys Pro Pro Pro Pro Arg
                85                  90                  95

Thr Gly Pro Cys Arg Gln Arg Ala Val Met Glu Thr Ile Ala Val Gly
            100                 105                 110

Cys Thr Cys Ile Phe
        115
```

<210> 8
<211> 197
<212> PRT
<213> Homo sapiens

<400> 8

```
Met Thr Leu Leu Pro Gly Leu Leu Phe Leu Thr Trp Leu His Thr Cys
1               5                   10                  15

Leu Ala His His Asp Pro Ser Leu Arg Gly His Pro His Ser His Gly
            20                  25                  30

Thr Pro His Cys Tyr Ser Ala Glu Glu Leu Pro Leu Gly Gln Ala Pro
        35                  40                  45

Pro His Leu Leu Ala Arg Gly Ala Lys Trp Gly Gln Ala Leu Pro Val
    50                  55                  60

Ala Leu Val Ser Ser Leu Glu Ala Ala Ser His Arg Gly Arg His Glu
65                  70                  75                  80

Arg Pro Ser Ala Thr Thr Gln Cys Pro Val Leu Arg Pro Glu Glu Val
                85                  90                  95

Leu Glu Ala Asp Thr His Gln Arg Ser Ile Ser Pro Trp Arg Tyr Arg
            100                 105                 110

Val Asp Thr Asp Glu Asp Arg Tyr Pro Gln Lys Leu Ala Phe Ala Glu
        115                 120                 125

Cys Leu Cys Arg Gly Cys Ile Asp Ala Arg Thr Gly Arg Glu Thr Ala
    130                 135                 140

Ala Leu Asn Ser Val Arg Leu Leu Gln Ser Leu Leu Val Leu Arg Arg
145                 150                 155                 160

Arg Pro Cys Ser Arg Asp Gly Ser Gly Leu Pro Thr Pro Gly Ala Phe
                165                 170                 175

Ala Phe His Thr Glu Phe Ile His Val Pro Val Gly Cys Thr Cys Val
            180                 185                 190
```

```
Leu Pro Arg Ser Val
            195
```

<210> 9
<211> 1496
<212> DNA
<213> Mus musculus

<220>
<221> CDS
<222> (511)..(987)

<400> 9

```
ccgggcaggt gccctcggcg cgtcccaaag cttagggaag ctccaggtgt cttgggaaat 60

gaagaaaaag gccaccgagc aaaaaggaac agagaagggg aggagcagtg ctgtgggctc 120

gcctagggtc gagggccatt atcacctaca aatcagaatg tgggagtgct attctagagg 180

tctccatctt tgccattgct gggtcgctca gaaaagtgtg atggggttgt cccattgcca 240

agaacagctt ctgcttacca gcaggtgctg acctctttcc ccagaggcac agggaaggaa 300

ttccagcccc ggttggctgc cagaggcttc ctctggcgtt gggtacagag gcagagaaag 360

aaaccccaaa tgtctcctat gaaaaacaat gtccccgtca tccaggccag atcattctgc 420

agtgtcaaca gttgagacaa gaagctgggg tcattttctg tgcctaagag tgcctgttct 480

gcactggcca aggctgttgc attcttggca atg atc gtg gga acc cac acc gtc  534
                                 Met Ile Val Gly Thr His Thr Val
                                 1               5

agc ttg cgg atc cag gag ggc tgc agt cac ttg ccc agc tgc tgc ccc  582
Ser Leu Arg Ile Gln Glu Gly Cys Ser His Leu Pro Ser Cys Cys Pro
    10                  15                  20

agc aaa gag caa gaa ccc ccg gag gag tgg ctg aag tgg agc tct gca  630
Ser Lys Glu Gln Glu Pro Pro Glu Glu Trp Leu Lys Trp Ser Ser Ala
25                  30                  35                  40

tct gtg tcc ccc cca gag cct ctg agc cac acc cac cac gca gaa tcc  678
Ser Val Ser Pro Pro Glu Pro Leu Ser His Thr His His Ala Glu Ser
                45                  50                  55

tgc agg gcc agc aag gat ggc ccc ctc aac agc agg gcc atc tct cct  726
Cys Arg Ala Ser Lys Asp Gly Pro Leu Asn Ser Arg Ala Ile Ser Pro
            60                  65                  70

tgg agc tat gag ttg gac agg gac ttg aat cgg gtc ccc cag gac ctg  774
Trp Ser Tyr Glu Leu Asp Arg Asp Leu Asn Arg Val Pro Gln Asp Leu
        75                  80                  85

tac cac gct cga tgc ctg tgc cca cac tgc gtc agc cta cag aca ggc  822
Tyr His Ala Arg Cys Leu Cys Pro His Cys Val Ser Leu Gln Thr Gly
    90                  95                  100

tcc cac atg gac ccg ctg ggc aac tcc gtc cca ctt tac cac aac cag  870
Ser His Met Asp Pro Leu Gly Asn Ser Val Pro Leu Tyr His Asn Gln
105                 110                 115                 120
```

```
acg gtc ttc tac cgg cgg cca tgc cat ggc gag gaa ggt acc cat cgc    918
Thr Val Phe Tyr Arg Arg Pro Cys His Gly Glu Glu Gly Thr His Arg
                125                 130                 135

cgc tac tgc ttg gag cgc agg ctc tac cga gtc tcc ttg gct tgt gtg    966
Arg Tyr Cys Leu Glu Arg Arg Leu Tyr Arg Val Ser Leu Ala Cys Val
            140                 145                 150

tgt gtg cgg ccc cgg gtc atg gcttagtcat gctcaccacc tgcctgaggc      1017
Cys Val Arg Pro Arg Val Met
        155

tgatgcccgg ttgggagaga gggccaggtg tacaatcacc ttgccaatgc gggccgggtt 1077

caagccctcc aaagccctac ctgaagcagc aggctcccgg gacaagatgg aggacttggg 1137

gagaaactct gacttttgca ctttttggaa gcacttttgg gaaggagcag gttccgcttg 1197

tgctgctaga ggatgctgtt gtggcatttc tactcaggaa cggactccaa aggcctgctg 1257

accctggaag ccatactcct ggctcctttc ccctgaatcc cccaactcct ggcacaggca 1317

ctttctccac ctctccccct ttgccttttg ttgtgtttgt ttgtgcatgc caactctgcg 1377

tgcagccagg tgtaattgcc ttgaaggatg gttctgaggt gaaagctgtt atcgaaagtg 1437

aagagattta tccaaataaa catctgtgtt taaaaaaaaa aaaaaaaaaa aaaaaaaaa  1496
```

<210> 10
<211> 159
<212> PRT
<213> Mus musculus

<400> 10

```
Met Ile Val Gly Thr His Thr Val Ser Leu Arg Ile Gln Glu Gly Cys
1               5                   10                  15

Ser His Leu Pro Ser Cys Cys Pro Ser Lys Glu Gln Glu Pro Pro Glu
            20                  25                  30

Glu Trp Leu Lys Trp Ser Ser Ala Ser Val Ser Pro Pro Glu Pro Leu
        35                  40                  45

Ser His Thr His His Ala Glu Ser Cys Arg Ala Ser Lys Asp Gly Pro
    50                  55                  60

Leu Asn Ser Arg Ala Ile Ser Pro Trp Ser Tyr Glu Leu Asp Arg Asp
65                  70                  75                  80

Leu Asn Arg Val Pro Gln Asp Leu Tyr His Ala Arg Cys Leu Cys Pro
                85                  90                  95

His Cys Val Ser Leu Gln Thr Gly Ser His Met Asp Pro Leu Gly Asn
            100                 105                 110

Ser Val Pro Leu Tyr His Asn Gln Thr Val Phe Tyr Arg Arg Pro Cys
        115                 120                 125

His Gly Glu Glu Gly Thr His Arg Arg Tyr Cys Leu Glu Arg Arg Leu
    130                 135                 140
```

Tyr Arg Val Ser Leu Ala Cys Val Cys Val Arg Pro Arg Val Met
145 150 155

<210> 11
<211> 27
<212> PRT
<213> Artificial Sequence

<220>
<223> Description of Artificial Sequence: Epogen signal peptide

<400> 11

Met Gly Val His Glu Cys Pro Ala Trp Leu Trp Leu Leu Leu Ser Leu
1 5 10 15

Leu Ser Leu Pro Leu Gly Leu Pro Val Leu Gly
20 25

<210> 12
<211> 233
<212> PRT
<213> Artificial Sequence

<220>
<223> Description of Artificial Sequence: peptideof Fc fragment

<400> 12

Glu Pro Lys Ser Ala Asp Lys Thr His Thr Cys Pro Pro Cys Pro Ala
1 5 10 15

Pro Glu Leu Leu Gly Gly Pro Ser Val Phe Leu Phe Pro Pro Lys Pro
20 25 30

Lys Asp Thr Leu Met Ile Ser Arg Thr Pro Glu Val Thr Cys Val Val
35 40 45

Val Asp Val Ser His Glu Asp Pro Glu Val Lys Phe Asn Trp Tyr Val
50 55 60

Asp Gly Val Glu Val His Asn Ala Lys Thr Lys Pro Arg Glu Glu Gln
65 70 75 80

Tyr Asn Ser Thr Tyr Arg Val Val Ser Val Leu Thr Val Leu His Gln
85 90 95

Asp Trp Leu Asn Gly Lys Glu Tyr Lys Cys Lys Val Ser Asn Lys Ala
100 105 110

Leu Pro Ala Pro Ile Glu Lys Thr Ile Ser Lys Ala Lys Gly Gln Pro
115 120 125

Arg Glu Pro Gln Val Tyr Thr Leu Pro Pro Ser Arg Asp Glu Leu Thr
130 135 140

Lys Asn Gln Val Ser Leu Thr Cys Leu Val Lys Gly Phe Tyr Pro Ser
145 150 155 160

Asp Asp Ile Ala Val Glu Trp Glu Ser Asn Gly Gln Pro Glu Asn Asn
165 170 175

Tyr Lys Thr Thr Pro Pro Val Leu Asp Ser Asp Gly Ser Phe Phe Leu
180 185 190

Tyr Ser Lys Leu Thr Val Asp Lys Ser Arg Trp Gln Gln Gly Asn Val
195 200 205

Phe Ser Cys Ser Val Met His Glu Ala Leu His Asn His Tyr Thr Gln
210 215 220

Lys Ser Leu Ser Leu Ser Pro Gly Lys
225 230

<210> 13
<211> 11
<212> PRT
<213> Artificial Sequence

<220>
<223> Description of Artificial Sequence: Peptide of HIV TAT protein

<400> 13

Tyr Gly Arg Lys Lys Arg Arg Gln Arg Arg Arg
1 5 10

<210> 14
<211> 19
<212> PRT
<213> Artificial Sequence

<220>
<223> Description of Artificial Sequence: Peptide of HIV TAT protein

<400> 14

Phe Ile Thr Cys Gly Gly Gly Gly Tyr Gly Arg Lys Lys Arg Arg Gln
1 5 10 15

Arg Arg Arg

<210> 15
<211> 18
<212> DNA
<213> Artificial Sequence

<220>
<223> Description of Artificial Sequence: PCR Primer

<400> 15
tagggccagt gaagatgg 18

<210> 16
<211> 26
<212> DNA
<213> Artificial Sequence

<220>
<223> Description of Artificial Sequence: PCR Primer

<400> 16
tacagcctgc gctccaggca gtagcc 26

<210> 17
<211> 1841
<212> DNA
<213> Homo sapiens

<220>
<221> CDS
<222> (50)..(1555)

<400> 17

```
ataaaagcgc agcgtgcggg tggcctggat cccgcgcagt ggcccggcg atg tcg ctc 58
                                                      Met Ser Leu
                                                      1

gtg ctg cta agc ctg gcc gcg ctg tgc agg agc gcc gta ccc cga gag   106
Val Leu Leu Ser Leu Ala Ala Leu Cys Arg Ser Ala Val Pro Arg Glu
    5                   10                  15

ccg acc gtt caa tgt ggc tct gaa act ggg cca tct cca gag tgg atg   154
Pro Thr Val Gln Cys Gly Ser Glu Thr Gly Pro Ser Pro Glu Trp Met
20                  25                  30                  35

cta caa cat gat cta atc ccc gga gac ttg agg gac ctc cga gta gaa   202
Leu Gln His Asp Leu Ile Pro Gly Asp Leu Arg Asp Leu Arg Val Glu
                40                  45                  50

cct gtt aca act agt gtt gca aca ggg gac tat tca att ttg atg aat   250
Pro Val Thr Thr Ser Val Ala Thr Gly Asp Tyr Ser Ile Leu Met Asn
            55                  60                  65

gta agc tgg gta ctc cgg gca gat gcc agc atc cgc ttg ttg aag gcc   298
Val Ser Trp Val Leu Arg Ala Asp Ala Ser Ile Arg Leu Leu Lys Ala
        70                  75                  80

acc aag att tgt gtg acg ggc aaa agc aac ttc cag tcc tac agc tgt   346
Thr Lys Ile Cys Val Thr Gly Lys Ser Asn Phe Gln Ser Tyr Ser Cys
    85                  90                  95

gtg agg tgc aat tac aca gag gcc ttc cag act cag acc aga ccc tct   394
Val Arg Cys Asn Tyr Thr Glu Ala Phe Gln Thr Gln Thr Arg Pro Ser
100                 105                 110                 115

ggt ggt aaa tgg aca ttt tcc tac atc ggc ttc cct gta gag ctg aac   442
Gly Gly Lys Trp Thr Phe Ser Tyr Ile Gly Phe Pro Val Glu Leu Asn
                120                 125                 130

aca gtc tat ttc att ggg gcc cat aat att cct aat gca aat atg aat   490
Thr Val Tyr Phe Ile Gly Ala His Asn Ile Pro Asn Ala Asn Met Asn
            135                 140                 145

gaa gat ggc cct tcc atg tct gtg aat ttc acc tca cca ggc tgc cta   538
Glu Asp Gly Pro Ser Met Ser Val Asn Phe Thr Ser Pro Gly Cys Leu
        150                 155                 160
```

```
gac cac ata atg aaa tat aaa aaa aag tgt gtc aag gcc gga agc ctg   586
Asp His Ile Met Lys Tyr Lys Lys Lys Cys Val Lys Ala Gly Ser Leu
    165                 170                 175

tgg gat ccg aac atc act gct tgt aag aag aat gag gag aca gta gaa   634
Trp Asp Pro Asn Ile Thr Ala Cys Lys Lys Asn Glu Glu Thr Val Glu
180                 185                 190                 195

gtg aac ttc aca acc act ccc ctg gga aac aga tac atg gct ctt atc   682
Val Asn Phe Thr Thr Thr Pro Leu Gly Asn Arg Tyr Met Ala Leu Ile
                200                 205                 210

caa cac agc act atc atc ggg ttt tct cag gtg ttt gag cca cac cag   730
Gln His Ser Thr Ile Ile Gly Phe Ser Gln Val Phe Glu Pro His Gln
            215                 220                 225

aag aaa caa acg cga gct tca gtg gtg att cca gtg act ggg gat agt   778
Lys Lys Gln Thr Arg Ala Ser Val Val Ile Pro Val Thr Gly Asp Ser
        230                 235                 240

gaa ggt gct acg gtg cag ctg act cca tat ttt cct act tgt ggc agc   826
Glu Gly Ala Thr Val Gln Leu Thr Pro Tyr Phe Pro Thr Cys Gly Ser
    245                 250                 255

gac tgc atc cga cat aaa gga aca gtt gtg ctc tgc cca caa aca ggc   874
Asp Cys Ile Arg His Lys Gly Thr Val Val Leu Cys Pro Gln Thr Gly
260                 265                 270                 275

gtc cct ttc cct ctg gat aac aac aaa agc aag ccg gga ggc tgg ctg   922
Val Pro Phe Pro Leu Asp Asn Asn Lys Ser Lys Pro Gly Gly Trp Leu
                280                 285                 290

cct ctc ctc ctg ctg tct ctg ctg gtg gcc aca tgg gtg ctg gtg gca   970
Pro Leu Leu Leu Leu Ser Leu Leu Val Ala Thr Trp Val Leu Val Ala
            295                 300                 305

ggg atc tat cta atg tgg agg cac gaa agg atc aag aag act tcc ttt   1018
Gly Ile Tyr Leu Met Trp Arg His Glu Arg Ile Lys Lys Thr Ser Phe
        310                 315                 320

tct acc acc aca cta ctg ccc ccc att aag gtt ctt gtg gtt tac cca   1066
Ser Thr Thr Thr Leu Leu Pro Pro Ile Lys Val Leu Val Val Tyr Pro
    325                 330                 335

tct gaa ata tgt ttc cat cac aca att tgt tac ttc act gaa ttt ctt   1114
Ser Glu Ile Cys Phe His His Thr Ile Cys Tyr Phe Thr Glu Phe Leu
340                 345                 350                 355

caa aac cat tgc aga agt gag gtc atc ctc gaa aag tgg cag aaa aag   1162
Gln Asn His Cys Arg Ser Glu Val Ile Leu Glu Lys Trp Gln Lys Lys
                360                 365                 370

aaa ata gca gag atg ggt cca gtg cag tgg ctt gcc act caa aag aag   1210
Lys Ile Ala Glu Met Gly Pro Val Gln Trp Leu Ala Thr Gln Lys Lys
            375                 380                 385

gca gca gac aaa gtc gtc ttc ctt ctt tcc aat gac gtc aac agt gtg   1258
Ala Ala Asp Lys Val Val Phe Leu Leu Ser Asn Asp Val Asn Ser Val
        390                 395                 400
```

```
tgc gat ggt acc tgt ggc aag agc gag ggc agt ccc agt gag aac tct     1306
Cys Asp Gly Thr Cys Gly Lys Ser Glu Gly Ser Pro Ser Glu Asn Ser
    405                 410                 415

caa gac ctc ttc ccc ctt gcc ttt aac ctt ttc tgc agt gat cta aga     1354
Gln Asp Leu Phe Pro Leu Ala Phe Asn Leu Phe Cys Ser Asp Leu Arg
420                 425                 430                 435

agc cag att cat ctg cac aaa tac gtg gtg gtc tac ttt aga gag att     1402
Ser Gln Ile His Leu His Lys Tyr Val Val Val Tyr Phe Arg Glu Ile
                440                 445                 450

gat aca aaa gac gat tac aat gct ctc agt gtc tgc ccc aag tac cac     1450
Asp Thr Lys Asp Asp Tyr Asn Ala Leu Ser Val Cys Pro Lys Tyr His
            455                 460                 465

ctc atg aag gat gcc act gct ttc tgt gca gaa ctt ctc cat gtc aag     1498
Leu Met Lys Asp Ala Thr Ala Phe Cys Ala Glu Leu Leu His Val Lys
        470                 475                 480

cag cag gtg tca gca gga aaa aga tca caa gcc tgc cac gat ggc tgc     1546
Gln Gln Val Ser Ala Gly Lys Arg Ser Gln Ala Cys His Asp Gly Cys
    485                 490                 495

tgc tcc ttg tagcccaccc atgagaagca agagacctta aaggcttcct             1595
Cys Ser Leu
500

atcccaccaa ttacagggaa aaaacgtgtg atgatcctga agcttactat gcagcctaca 1655

aacagcctta gtaattaaaa cattttatac caataaaatt ttcaaatatt gctaactaat 1715

gtagcattaa ctaacgattg gaaactacat ttacaacttc aaagctgttt tatacataga 1775

aatcaattac agctttaatt gaaaactgta accattttga taatgcaaca ataaagcatc 1835

ttcagc                                                            1841
```

<210> 18
<211> 502
<212> PRT
<213> Homo sapiens

<400> 18

```
Met Ser Leu Val Leu Leu Ser Leu Ala Ala Leu Cys Arg Ser Ala Val
 1               5                  10                  15

Pro Arg Glu Pro Thr Val Gln Cys Gly Ser Glu Thr Gly Pro Ser Pro
            20                  25                  30

Glu Trp Met Leu Gln His Asp Leu Ile Pro Gly Asp Leu Arg Asp Leu
        35                  40                  45

Arg Val Glu Pro Val Thr Thr Ser Val Ala Thr Gly Asp Tyr Ser Ile
    50                  55                  60

Leu Met Asn Val Ser Trp Val Leu Arg Ala Asp Ala Ser Ile Arg Leu
65                  70                  75                  80

Leu Lys Ala Thr Lys Ile Cys Val Thr Gly Lys Ser Asn Phe Gln Ser
                85                  90                  95
```

Tyr Ser Cys Val Arg Cys Asn Tyr Thr Glu Ala Phe Gln Thr Gln Thr
100 105 110

Arg Pro Ser Gly Gly Lys Trp Thr Phe Ser Tyr Ile Gly Phe Pro Val
115 120 125

Glu Leu Asn Thr Val Tyr Phe Ile Gly Ala His Asn Ile Pro Asn Ala
130 135 140

Asn Met Asn Glu Asp Gly Pro Ser Met Ser Val Asn Phe Thr Ser Pro
145 150 155 160

Gly Cys Leu Asp His Ile Met Lys Tyr Lys Lys Lys Cys Val Lys Ala
165 170 175

Gly Ser Leu Trp Asp Pro Asn Ile Thr Ala Cys Lys Lys Asn Glu Glu
180 185 190

Thr Val Glu Val Asn Phe Thr Thr Thr Pro Leu Gly Asn Arg Tyr Met
195 200 205

Ala Leu Ile Gln His Ser Thr Ile Ile Gly Phe Ser Gln Val Phe Glu
210 215 220

Pro His Gln Lys Lys Gln Thr Arg Ala Ser Val Val Ile Pro Val Thr
225 230 235 240

Gly Asp Ser Glu Gly Ala Thr Val Gln Leu Thr Pro Tyr Phe Pro Thr
245 250 255

Cys Gly Ser Asp Cys Ile Arg His Lys Gly Thr Val Val Leu Cys Pro
260 265 270

Gln Thr Gly Val Pro Phe Pro Leu Asp Asn Asn Lys Ser Lys Pro Gly
275 280 285

Gly Trp Leu Pro Leu Leu Leu Leu Ser Leu Leu Val Ala Thr Trp Val
290 295 300

Leu Val Ala Gly Ile Tyr Leu Met Trp Arg His Glu Arg Ile Lys Lys
305 310 315 320

Thr Ser Phe Ser Thr Thr Thr Leu Leu Pro Pro Ile Lys Val Leu Val
325 330 335

Val Tyr Pro Ser Glu Ile Cys Phe His His Thr Ile Cys Tyr Phe Thr
340 345 350

Glu Phe Leu Gln Asn His Cys Arg Ser Glu Val Ile Leu Glu Lys Trp
355 360 365

Gln Lys Lys Lys Ile Ala Glu Met Gly Pro Val Gln Trp Leu Ala Thr
370 375 380

Gln Lys Lys Ala Ala Asp Lys Val Val Phe Leu Leu Ser Asn Asp Val
385 390 395 400

Asn Ser Val Cys Asp Gly Thr Cys Gly Lys Ser Glu Gly Ser Pro Ser
405 410 415

Glu Asn Ser Gln Asp Leu Phe Pro Leu Ala Phe Asn Leu Phe Cys Ser
420 425 430

```
Asp Leu Arg Ser Gln Ile His Leu His Lys Tyr Val Val Val Tyr Phe
        435                 440                 445

Arg Glu Ile Asp Thr Lys Asp Asp Tyr Asn Ala Leu Ser Val Cys Pro
    450                 455                 460

Lys Tyr His Leu Met Lys Asp Ala Thr Ala Phe Cys Ala Glu Leu Leu
465                 470                 475                 480

His Val Lys Gln Gln Val Ser Ala Gly Lys Arg Ser Gln Ala Cys His
                485                 490                 495

Asp Gly Cys Cys Ser Leu
            500
```

<210> 19
<211> 2015
<212> DNA
<213> Homo sapiens

<220>
<221> CDS
<222> (50)..(1729)

<40.0> 19

```
ataaaagcgc agcgtgcggg tggcctggat cccgcgcagt ggcccggcg atg tcg ctc 58
                                                      Met Ser Leu
                                                      1

gtg ctg cta agc ctg gcc gcg ctg tgc agg agc gcc gta ccc cga gag   106
Val Leu Leu Ser Leu Ala Ala Leu Cys Arg Ser Ala Val Pro Arg Glu
    5                   10                  15

ccg acc gtt caa tgt ggc tct gaa act ggg cca tct cca gag tgg atg   154
Pro Thr Val Gln Cys Gly Ser Glu Thr Gly Pro Ser Pro Glu Trp Met
20                  25                  30                  35

cta caa cat gat cta atc ccc gga gac ttg agg gac ctc cga gta gaa   202
Leu Gln His Asp Leu Ile Pro Gly Asp Leu Arg Asp Leu Arg Val Glu
                40                  45                  50

cct gtt aca act agt gtt gca aca ggg gac tat tca att ttg atg aat   250
Pro Val Thr Thr Ser Val Ala Thr Gly Asp Tyr Ser Ile Leu Met Asn
            55                  60                  65

gta agc tgg gta ctc cgg gca gat gcc agc atc cgc ttg ttg aag gcc   298
Val Ser Trp Val Leu Arg Ala Asp Ala Ser Ile Arg Leu Leu Lys Ala
        70                  75                  80

acc aag att tgt gtg acg ggc aaa agc aac ttc cag tcc tac agc tgt   346
Thr Lys Ile Cys Val Thr Gly Lys Ser Asn Phe Gln Ser Tyr Ser Cys
    85                  90                  95

gtg agg ctg gag tgc agt ggt gcg atc atg gct cgc tgc gac ctc aat   394
Val Arg Leu Glu Cys Ser Gly Ala Ile Met Ala Arg Cys Asp Leu Asn
100                 105                 110                 115

ctt ctg ggc tca agc gat cgt tct gct tca gcc tcc cga gcg gct ggg   442
Leu Leu Gly Ser Ser Asp Arg Ser Ala Ser Ala Ser Arg Ala Ala Gly
                120                 125                 130
```

```
act gca ggc gtg ggc cac cag acc tgg cta att ttt gta gtt ttt gta    490
Thr Ala Gly Val Gly His Gln Thr Trp Leu Ile Phe Val Val Phe Val
            135                 140                 145

gag ggg ggt ttc acc gtg ttg ctg gtc ttg aat tcc agt gct cag gcg    538
Glu Gly Gly Phe Thr Val Leu Leu Val Leu Asn Ser Ser Ala Gln Ala
        150                 155                 160

atc tgc ctg cct cgg ctt ccc aaa gtg ctg gga tta cag tgg aca ttt    586
Ile Cys Leu Pro Arg Leu Pro Lys Val Leu Gly Leu Gln Trp Thr Phe
    165                 170                 175

tcc tac atc ggc ttc cct gta gag ctg aac aca gtc tat ttc att ggg    634
Ser Tyr Ile Gly Phe Pro Val Glu Leu Asn Thr Val Tyr Phe Ile Gly
180                 185                 190                 195

gcc cat aat att cct aat gca aat atg aat gaa gat ggc cct tcc atg    682
Ala His Asn Ile Pro Asn Ala Asn Met Asn Glu Asp Gly Pro Ser Met
                200                 205                 210

tct gtg aat ttc acc tca cca ggc tgc cta gac cac ata atg aaa tat    730
Ser Val Asn Phe Thr Ser Pro Gly Cys Leu Asp His Ile Met Lys Tyr
            215                 220                 225

aaa aaa aag tgt gtc aag gcc gga agc ctg tgg gat ccg aac atc act    778
Lys Lys Lys Cys Val Lys Ala Gly Ser Leu Trp Asp Pro Asn Ile Thr
        230                 235                 240

gct tgt aag aag aat gag gag aca gta gaa gtg aac ttc aca acc act    826
Ala Cys Lys Lys Asn Glu Glu Thr Val Glu Val Asn Phe Thr Thr Thr
    245                 250                 255

ccc ctg gga aac aga tac atg gct ctt atc caa cac agc act atc atc    874
Pro Leu Gly Asn Arg Tyr Met Ala Leu Ile Gln His Ser Thr Ile Ile
260                 265                 270                 275

ggg ttt tct cag gtg ttt gag cca cac cag aag aaa caa acg cga gct    922
Gly Phe Ser Gln Val Phe Glu Pro His Gln Lys Lys Gln Thr Arg Ala
                280                 285                 290

tca gtg gtg att cca gtg act ggg gat agt gaa ggt gct acg gtg cag    970
Ser Val Val Ile Pro Val Thr Gly Asp Ser Glu Gly Ala Thr Val Gln
            295                 300                 305

ctg act cca tat ttt cct act tgt ggc agc gac tgc atc cga cat aaa    1018
Leu Thr Pro Tyr Phe Pro Thr Cys Gly Ser Asp Cys Ile Arg His Lys
        310                 315                 320

gga aca gtt gtg ctc tgc cca caa aca ggc gtc cct ttc cct ctg gat    1066
Gly Thr Val Val Leu Cys Pro Gln Thr Gly Val Pro Phe Pro Leu Asp
    325                 330                 335

aac aac aaa agc aag ccg gga ggc tgg ctg cct ctc ctc ctg ctg tct    1114
Asn Asn Lys Ser Lys Pro Gly Gly Trp Leu Pro Leu Leu Leu Leu Ser
340                 345                 350                 355

ctg ctg gtg gcc aca tgg gtg ctg gtg gca ggg atc tat cta atg tgg    1162
Leu Leu Val Ala Thr Trp Val Leu Val Ala Gly Ile Tyr Leu Met Trp
                360                 365                 370

agg cac gaa agg atc aag aag act tcc ttt tct acc acc aca cta ctg    1210
Arg His Glu Arg Ile Lys Lys Thr Ser Phe Ser Thr Thr Thr Leu Leu
            375                 380                 385
```

```
ccc ccc att aag gtt ctt gtg gtt tac cca tct gaa ata tgt ttc cat    1258
Pro Pro Ile Lys Val Leu Val Val Tyr Pro Ser Glu Ile Cys Phe His
        390                 395                 400

cac aca att tgt tac ttc act gaa ttt ctt caa aac cat tgc aga agt    1306
His Thr Ile Cys Tyr Phe Thr Glu Phe Leu Gln Asn His Cys Arg Ser
    405                 410                 415

gag gtc atc ctc gaa aag tgg cag aaa aag aaa ata gca gag atg ggt    1354
Glu Val Ile Leu Glu Lys Trp Gln Lys Lys Lys Ile Ala Glu Met Gly
420                 425                 430                 435

cca gtg cag tgg ctt gcc act caa aag aag gca gca gac aaa gtc gtc    1402
Pro Val Gln Trp Leu Ala Thr Gln Lys Lys Ala Ala Asp Lys Val Val
                440                 445                 450

ttc ctt ctt tcc aat gac gtc aac agt gtg tgc gat ggt acc tgt ggc    1450
Phe Leu Leu Ser Asn Asp Val Asn Ser Val Cys Asp Gly Thr Cys Gly
            455                 460                 465

aag agc gag ggc agt ccc agt gag aac tct caa gac ctc ttc ccc ctt    1498
Lys Ser Glu Gly Ser Pro Ser Glu Asn Ser Gln Asp Leu Phe Pro Leu
        470                 475                 480

gcc ttt aac ctt ttc tgc agt gat cta aga agc cag att cat ctg cac    1546
Ala Phe Asn Leu Phe Cys Ser Asp Leu Arg Ser Gln Ile His Leu His
    485                 490                 495

aaa tac gtg gtg gtc tac ttt aga gag att gat aca aaa gac gat tac    1594
Lys Tyr Val Val Val Tyr Phe Arg Glu Ile Asp Thr Lys Asp Asp Tyr
500                 505                 510                 515

aat gct ctc agt gtc tgc ccc aag tac cac ctc atg aag gat gcc act    1642
Asn Ala Leu Ser Val Cys Pro Lys Tyr His Leu Met Lys Asp Ala Thr
                520                 525                 530

gct ttc tgt gca gaa ctt ctc cat gtc aag cag cag gtg tca gca gga    1690
Ala Phe Cys Ala Glu Leu Leu His Val Lys Gln Gln Val Ser Ala Gly
            535                 540                 545

aaa aga tca caa gcc tgc cac gat ggc tgc tgc tcc ttg tagcccaccc     1739
Lys Arg Ser Gln Ala Cys His Asp Gly Cys Cys Ser Leu
        550                 555                 560

atgagaagca agagacctta aaggcttcct atcccaccaa ttacagggaa aaaacgtgtg 1799

atgatcctga agcttactat gcagcctaca aacagcctta gtaattaaaa cattttatac 1859

caataaaatt ttcaaatatt gctaactaat gtagcattaa ctaacgattg gaaactacat 1919

ttacaacttc aaagctgttt tatacataga aatcaattac agctttaatt gaaaactgta 1979

accattttga taatgcaaca ataaagcatc ttcagc                           2015
```

<210> 20
<211> 560
<212> PRT
<213> Homo sapiens

<400> 20

```
Met Ser Leu Val Leu Leu Ser Leu Ala Ala Leu Cys Arg Ser Ala Val
 1               5                  10                  15

Pro Arg Glu Pro Thr Val Gln Cys Gly Ser Glu Thr Gly Pro Ser Pro
            20                  25                  30

Glu Trp Met Leu Gln His Asp Leu Ile Pro Gly Asp Leu Arg Asp Leu
        35                  40                  45

Arg Val Glu Pro Val Thr Thr Ser Val Ala Thr Gly Asp Tyr Ser Ile
    50                  55                  60

Leu Met Asn Val Ser Trp Val Leu Arg Ala Asp Ala Ser Ile Arg Leu
65                  70                  75                  80

Leu Lys Ala Thr Lys Ile Cys Val Thr Gly Lys Ser Asn Phe Gln Ser
                85                  90                  95

Tyr Ser Cys Val Arg Leu Glu Cys Ser Gly Ala Ile Met Ala Arg Cys
            100                 105                 110

Asp Leu Asn Leu Leu Gly Ser Ser Asp Arg Ser Ala Ser Ala Ser Arg
        115                 120                 125

Ala Ala Gly Thr Ala Gly Val Gly His Gln Thr Trp Leu Ile Phe Val
    130                 135                 140

Val Phe Val Glu Gly Gly Phe Thr Val Leu Leu Val Leu Asn Ser Ser
145                 150                 155                 160

Ala Gln Ala Ile Cys Leu Pro Arg Leu Pro Lys Val Leu Gly Leu Gln
                165                 170                 175

Trp Thr Phe Ser Tyr Ile Gly Phe Pro Val Glu Leu Asn Thr Val Tyr
            180                 185                 190

Phe Ile Gly Ala His Asn Ile Pro Asn Ala Asn Met Asn Glu Asp Gly
        195                 200                 205

Pro Ser Met Ser Val Asn Phe Thr Ser Pro Gly Cys Leu Asp His Ile
    210                 215                 220

Met Lys Tyr Lys Lys Lys Cys Val Lys Ala Gly Ser Leu Trp Asp Pro
225                 230                 235                 240

Asn Ile Thr Ala Cys Lys Lys Asn Glu Glu Thr Val Glu Val Asn Phe
                245                 250                 255

Thr Thr Thr Pro Leu Gly Asn Arg Tyr Met Ala Leu Ile Gln His Ser
            260                 265                 270

Thr Ile Ile Gly Phe Ser Gln Val Phe Glu Pro His Gln Lys Lys Gln
        275                 280                 285

Thr Arg Ala Ser Val Val Ile Pro Val Thr Gly Asp Ser Glu Gly Ala
    290                 295                 300

Thr Val Gln Leu Thr Pro Tyr Phe Pro Thr Cys Gly Ser Asp Cys Ile
305                 310                 315                 320

Arg His Lys Gly Thr Val Val Leu Cys Pro Gln Thr Gly Val Pro Phe
                325                 330                 335
```

Pro Leu Asp Asn Asn Lys Ser Lys Pro Gly Gly Trp Leu Pro Leu Leu
340 345 350

Leu Leu Ser Leu Leu Val Ala Thr Trp Val Leu Val Ala Gly Ile Tyr
355 360 365

Leu Met Trp Arg His Glu Arg Ile Lys Lys Thr Ser Phe Ser Thr Thr
370 375 380

Thr Leu Leu Pro Pro Ile Lys Val Leu Val Val Tyr Pro Ser Glu Ile
385 390 395 400

Cys Phe His His Thr Ile Cys Tyr Phe Thr Glu Phe Leu Gln Asn His
405 410 415

Cys Arg Ser Glu Val Ile Leu Glu Lys Trp Gln Lys Lys Lys Ile Ala
420 425 430

Glu Met Gly Pro Val Gln Trp Leu Ala Thr Gln Lys Lys Ala Ala Asp
435 440 445

Lys Val Val Phe Leu Leu Ser Asn Asp Val Asn Ser Val Cys Asp Gly
450 455 460

Thr Cys Gly Lys Ser Glu Gly Ser Pro Ser Glu Asn Ser Gln Asp Leu
465 470 475 480

Phe Pro Leu Ala Phe Asn Leu Phe Cys Ser Asp Leu Arg Ser Gln Ile
485 490 495

His Leu His Lys Tyr Val Val Val Tyr Phe Arg Glu Ile Asp Thr Lys
500 505 510

Asp Asp Tyr Asn Ala Leu Ser Val Cys Pro Lys Tyr His Leu Met Lys
515 520 525

Asp Ala Thr Ala Phe Cys Ala Glu Leu Leu His Val Lys Gln Gln Val
530 535 540

Ser Ala Gly Lys Arg Ser Gln Ala Cys His Asp Gly Cys Cys Ser Leu
545 550 555 560

<210> 21
<211> 521
<212> PRT
<213> Homo sapiens

<400> 21

Met Ser Leu Val Leu Leu Ser Leu Ala Ala Leu Cys Arg Ser Ala Val
1 5 10 15

Pro Arg Glu Pro Thr Val Gln Cys Gly Ser Glu Thr Gly Pro Ser Pro
20 25 30

Glu Trp Met Leu Gln His Asp Leu Ile Pro Gly Asp Leu Arg Asp Leu
35 40 45

Arg Val Glu Pro Val Thr Thr Ser Val Ala Thr Gly Asp Tyr Ser Ile
50 55 60

Leu Met Asn Val Ser Trp Val Leu Arg Ala Asp Ala Ser Ile Arg Leu
65 70 75 80

```
Leu Lys Ala Thr Lys Ile Cys Val Thr Gly Lys Ser Asn Phe Gln Ser
                85                  90                  95

Tyr Ser Cys Val Arg Cys Asn Tyr Thr Glu Ala Phe Gln Thr Gln Ser
            100                 105                 110

Gly Gly Lys Trp Thr Phe Ser Tyr Ile Gly Phe Pro Val Glu Leu Asn
        115                 120                 125

Thr Val Tyr Phe Ile Gly Ala His Asn Ile Pro Asn Ala Asn Met Asn
    130                 135                 140

Glu Asp Gly Pro Ser Met Ser Val Asn Phe Thr Ser Pro Gly Cys Leu
145                 150                 155                 160

Asp His Ile Met Lys Tyr Lys Lys Lys Cys Val Lys Ala Gly Ser Leu
                165                 170                 175

Trp Asp Pro Asn Ile Thr Ala Cys Lys Lys Asn Glu Glu Thr Val Glu
            180                 185                 190

Val Asn Phe Thr Thr Thr Pro Leu Gly Asn Arg Tyr Met Ala Leu Ile
        195                 200                 205

Gln His Ser Thr Ile Ile Gly Phe Ser Gln Val Phe Glu Pro His Gln
    210                 215                 220

Lys Lys Gln Thr Arg Ala Ser Val Val Ile Pro Val Thr Gly Asp Ser
225                 230                 235                 240

Glu Gly Ala Thr Val Gln Leu Thr Pro Tyr Phe Pro Thr Cys Gly Ser
                245                 250                 255

Asp Cys Ile Arg His Lys Gly Thr Val Val Leu Cys Pro Gln Thr Gly
            260                 265                 270

Val Pro Phe Pro Leu Asp Asn Asn Lys Ser Lys Pro Gly Gly Trp Leu
        275                 280                 285

Pro Ala Ala Ala Glu Pro Lys Ser Cys Asp Lys Thr His Thr Cys Pro
    290                 295                 300

Pro Cys Pro Ala Pro Glu Leu Leu Gly Gly Pro Ser Val Phe Leu Phe
305                 310                 315                 320

Pro Pro Lys Pro Lys Asp Thr Leu Met Ile Ser Arg Thr Pro Glu Val
                325                 330                 335

Thr Cys Val Val Val Asp Val Ser His Glu Asp Pro Glu Val Lys Phe
            340                 345                 350

Asn Trp Tyr Val Asp Gly Val Glu Val His Asn Ala Lys Thr Lys Pro
        355                 360                 365

Arg Glu Glu Gln Tyr Asn Ser Thr Tyr Arg Val Val Ser Val Leu Thr
    370                 375                 380

Val Leu His Gln Asp Trp Leu Asn Gly Lys Glu Tyr Lys Cys Lys Val
385                 390                 395                 400

Ser Asn Lys Ala Leu Pro Ala Pro Ile Glu Lys Thr Ile Ser Lys Ala
                405                 410                 415
```

```
Lys Gly Gln Pro Arg Glu Pro Gln Val Tyr Thr Leu Pro Pro Ser Arg
            420                 425                 430

Asp Glu Leu Thr Lys Asn Gln Val Ser Leu Thr Cys Leu Val Lys Gly
        435                 440                 445

Phe Tyr Pro Ser Asp Ile Ala Val Glu Trp Glu Ser Asn Gly Gln Pro
    450                 455                 460

Glu Asn Asn Tyr Lys Thr Thr Pro Pro Val Leu Asp Ser Asp Gly Ser
465                 470                 475                 480

Phe Phe Lys Leu Thr Val Asp Lys Ser Arg Trp Gln Gln Gly Asn Val
                485                 490                 495

Phe Ser Cys Ser Val Met His Glu Ala Leu His Asn His Tyr Thr Gln
            500                 505                 510

Lys Ser Leu Ser Leu Ser Pro Gly Lys
        515                 520
```

<210> 22
<211> 585
<212> PRT
<213> Homo sapiens

<400> 22

```
Met Ser Leu Val Leu Leu Ser Leu Ala Ala Leu Cys Arg Ser Ala Val
  1               5                  10                  15

Pro Arg Glu Pro Thr Val Gln Cys Gly Ser Glu Thr Gly Pro Ser Pro
             20                  25                  30

Glu Trp Met Leu Gln His Asp Leu Ile Pro Gly Asp Leu Arg Asp Leu
         35                  40                  45

Arg Val Glu Pro Val Thr Thr Ser Val Ala Thr Gly Asp Tyr Ser Ile
     50                  55                  60

Leu Met Asn Val Ser Trp Val Leu Arg Ala Asp Ala Ser Ile Arg Leu
 65                  70                  75                  80

Leu Lys Ala Thr Lys Ile Cys Val Thr Gly Lys Ser Asn Phe Gln Ser
                 85                  90                  95

Tyr Ser Cys Val Arg Leu Glu Cys Ser Gly Ala Ile Met Ala Arg Cys
            100                 105                 110

Asp Leu Asn Leu Leu Gly Ser Ser Asp Arg Ser Ala Ser Ala Ser Arg
        115                 120                 125

Ala Ala Gly Thr Ala Gly Val Gly His Gln Thr Trp Leu Ile Phe Val
    130                 135                 140

Val Phe Val Glu Gly Gly Phe Thr Val Leu Leu Val Leu Asn Ser Ser
145                 150                 155                 160

Ala Gln Ala Ile Cys Leu Pro Arg Leu Pro Lys Val Leu Gly Leu Gln
                165                 170                 175

Trp Thr Phe Ser Tyr Ile Gly Phe Pro Val Glu Leu Asn Thr Val Tyr
            180                 185                 190
```

Phe Ile Gly Ala His Asn Ile Pro Asn Ala Asn Met Asn Glu Asp Gly
195 200 205

Pro Ser Met Ser Val Asn Phe Thr Ser Pro Gly Cys Leu Asp His Ile
210 215 220

Met Lys Tyr Lys Lys Lys Cys Val Lys Ala Gly Ser Leu Trp Asp Pro
225 230 235 240

Asn Ile Thr Ala Cys Lys Lys Asn Glu Glu Thr Val Glu Val Asn Phe
245 250 255

Thr Thr Thr Pro Leu Gly Asn Arg Tyr Met Ala Leu Ile Gln His Ser
260 265 270

Thr Ile Ile Gly Phe Ser Gln Val Phe Glu Pro His Gln Lys Lys Gln
275 280 285

Thr Arg Ala Ser Val Val Ile Pro Val Thr Gly Asp Ser Glu Gly Ala
290 295 300

Thr Val Gln Leu Thr Pro Tyr Phe Pro Thr Cys Gly Ser Asp Cys Ile
305 310 315 320

Arg His Lys Gly Thr Val Val Leu Cys Pro Gln Thr Gly Val Pro Phe
325 330 335

Pro Leu Asp Asn Asn Lys Ser Lys Pro Gly Gly Trp Leu Pro Ala Ala
340 345 350

Ala Glu Pro Lys Ser Cys Asp Lys Thr His Thr Cys Pro Pro Cys Pro
355 360 365

Ala Pro Glu Leu Leu Gly Gly Pro Ser Val Phe Leu Phe Pro Pro Lys
370 375 380

Pro Lys Asp Thr Leu Met Ile Ser Arg Thr Pro Glu Val Thr Cys Val
385 390 395 400

Val Val Asp Val Ser His Glu Asp Pro Glu Val Lys Phe Asn Trp Tyr
405 410 415

Val Asp Gly Val Glu Val His Asn Ala Lys Thr Lys Pro Arg Glu Glu
420 425 430

Gln Tyr Asn Ser Thr Tyr Arg Val Val Ser Val Leu Thr Val Leu His
435 440 445

Gln Asp Trp Leu Asn Gly Lys Glu Tyr Lys Cys Lys Val Ser Asn Lys
450 455 460

Ala Leu Pro Ala Pro Ile Glu Lys Thr Ile Ser Lys Ala Lys Gly Gln
465 470 475 480

Pro Arg Glu Pro Gln Val Tyr Thr Leu Pro Pro Ser Arg Asp Glu Leu
485 490 495

Thr Lys Asn Gln Val Ser Leu Thr Cys Leu Val Lys Gly Phe Tyr Pro
500 505 510

Ser Asp Ile Ala Val Glu Trp Glu Ser Asn Gly Gln Pro Glu Asn Asn
515 520 525

```
Tyr Lys Thr Thr Pro Pro Val Leu Asp Ser Asp Gly Ser Phe Phe Leu
    530                 535                 540

Tyr Ser Lys Leu Thr Val Asp Lys Ser Arg Trp Gln Gln Gly Asn Val
545                 550                 555                 560

Phe Ser Cys Ser Val Met His Glu Ala Leu His Asn His Tyr Thr Gln
                565                 570                 575

Lys Ser Leu Ser Leu Ser Pro Gly Lys
            580                 585
```